# EUROPEAN PATENT SPECIFICATION

(11) **EP 4 584 262 B1**
(45) Date of publication and mention of the grant of the patent: **18.11.2026**
(21) Application number: 23798501.5
(22) Date of filing: 08.09.2023
(51) Int. Cl.: C07C 6/04, C07C 67/333, C07D 407/04, B01J 31/22, C07C 6/02, C07D 409/04, C07F 15/00, C07F 17/02

(54) **NOVEL RUTHENIUM COMPLEXES, METHOD OF THEIR SYNTHESIS, INTERMEDIATE COMPOUNDS USED IN THIS METHOD, METHOD OF THEIR SYNTHESIS AND USE OF NOVEL RUTHENIUM COMPLEXES IN OLEFIN METATHESIS REACTIONS**
NEUE RUTHENIUMKOMPLEXE, VERFAHREN ZU IHRER HERSTELLUNG, IN DIESEM VERFAHREN VERWENDETE ZWISCHENPRODUKTE, VERFAHREN ZU DEREN HERSTELLUNG SOWIE VERWENDUNG DER NEUEN RUTHENIUMKOMPLEXE IN OLEFINMETATHESEREAKTIONEN
NOUVEAUX COMPLEXES DE RUTHÉNIUM, LEUR PROCÉDÉ DE SYNTHÈSE, COMPOSÉS INTERMÉDIAIRES UTILISÉS DANS CE PROCÉDÉ, LEUR PROCÉDÉ DE SYNTHÈSE ET L'UTILISATION DE NOUVEAUX COMPLEXES DE RUTHÉNIUM DANS DES RÉACTIONS DE MÉTATHÈSE D'OLÉFINES

(30) Priority: 08.09.2022 PL 44222622
(43) Date of publication of application: 16.07.2025
(73) Proprietor: Uniwersytet Warszawski, 00-927 Warszawa (PL)
(72) Inventor: KAJETANOWICZ, Anna, 04-030 Warszawa (PL); SYTNICZUK, Adrian, 05-300 Minsk Mazowiecki (PL); STRUZIK, Filip, 02-116 Warszawa (PL)
(74) Representative: JD&P Patent Attorneys Joanna Dargiewicz & Partners
(86) International application number: PCT/IB2023/058921
(87) International publication number: WO 2024/052871

(56) References cited:
- WO-A1-2017/055945
- VANESSA M MARX ET AL: "Cyclic Alkyl Amino Carbene (CAAC) Ruthenium Complexes as Remarkably Active Catalysts for Ethenolysis", ANGEWANDTE CHEMIE INTERNATIONAL EDITION, VERLAG CHEMIE, HOBOKEN, USA, vol. 54, no. 6, 17 December 2014 (2014-12-17), pages 1919 - 1923, XP072070151, ISSN: 1433-7851, DOI: 10.1002/ANIE.201410797
- ANDERSON D R ET AL: "Synthesis and Reactivity of Olefin Metathesis Catalysts Bearing Cyclic (Alkyl)(Amino)Carbenes", ANGEWANDTE CHEMIE INTERNATIONAL EDITION, VERLAG CHEMIE, HOBOKEN, USA, vol. 46, 10 July 2007 (2007-07-10), pages 7262 - 7265, XP002468754, ISSN: 1433-7851, DOI: 10.1002/ANIE.200702085

## Description

The subject of the invention are novel ruthenium complexes with CAAC type ligands (*Cyclic Alkyl Amino Carbene*) which have found a widespread use as catalysts and/or (pre)catalysts of olefin metathesis reactions and their use in olefin metathesis reactions. The subject of the invention also includes intermediate compounds used to synthesize novel ruthenium complexes with CAAC ligands, as well as the method for synthesis of novel ruthenium complexes with CAAC ligands. This invention is used as desired tool in the widely understood organic synthesis, in the selective synthesis of olefins with a C=C bond, in particular in cross metathesis reaction with ethylene; ethenolysis.

Significant progress has been made in recent years in the use of olefin metathesis in organic synthesis [R. H. Grubbs (Editor), A. G. Wenzel (Editor), D. J. O'Leary (Editor), E. Khosravi (Editor), Handbook of Olefin Metathesis, 2. Edition, 3 volumes, 2015, Wiley-VCH Verlag GmbH & Co. KGaA, 1608 pages*].* Many ruthenium-based homogeneous olefin metathesis catalysts are known in the state-of-the-art which show high activity in various types of metathesis reactions, as well as high tolerance to functional groups present in the substrate/product. Due to the combination of these features, metathesis catalysts are of key importance in modern organic synthesis and in the industry. The most widely present ruthenium complexes in the literature and the ones finding the most widespread use in olefin metathesis reactions include Grubbs-type ruthenium complexes (**Gru-I, Gru-II** and **Gru-III),** Hoveyda-Grubbs complexes (**Hov-I** and **Hov-II**), as well as indenylidene complexes (**Ind-I, Ind-II** and **Ind-III**), 1^{st}, 2^{nd} and 3^{rd} generation [Grubbs et al. Chem. Rev. 2010, 110, 1746-1787*;* Nolan et al. Chem. Commun. 2014, 50, 10355-10375]*.* In other cases, most structures of olefin metathesis catalysts are derived from the aforementioned ruthenium complexes.

Recently, the NHC ligand in the ruthenium catalyst structure has been replaced by a cyclic (alkyl) (amino) carbene ligand (CAAC), and resulted complexes occupy an important place in modern organic synthesis due to their use i.e. in cross metathesis reactions and in ring-closing metathesis [Grubbs et al. Chem. Rev., 2010, 110, 1746-1787*,* WO2017055945A1]*.*

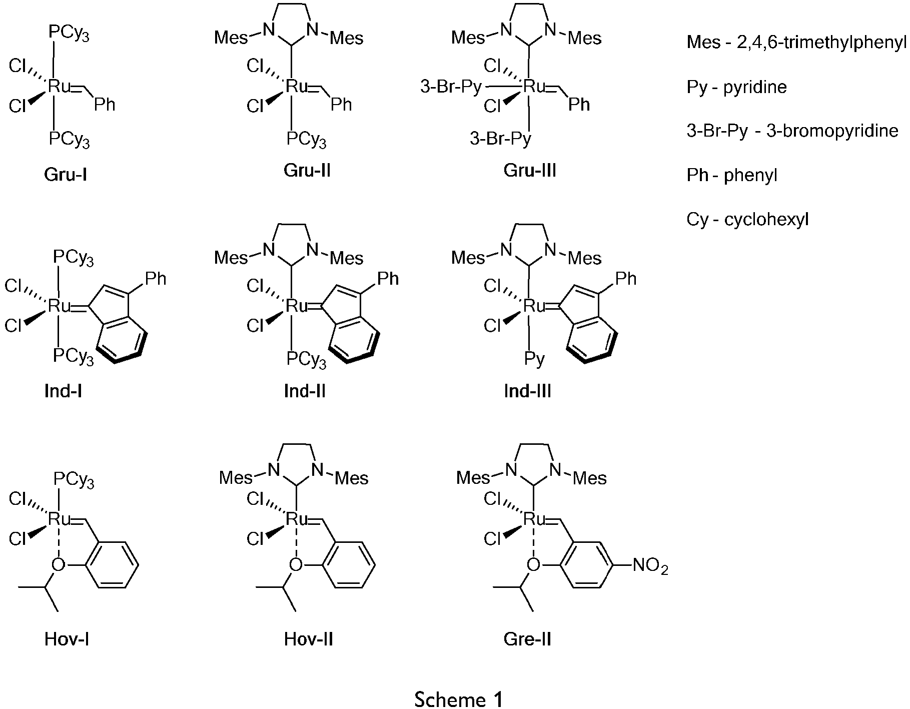

In modern organic synthesis, in an era of increasingly scarce resources, including fossil fuels, and the consequent risk of a shortage of raw materials for the synthesis for example, polymers based on products derived, for example, from crude oil, it is very important to develop new technologies and reactions that enable the synthesis of target compounds. Such processes include the ethenolysis reaction, particularly the ethenolysis of methyl/ethyl derivatives of long-chain fatty acids. In particular, ruthenium complexes with CAAC ligands in the ruthenium coordination sphere are used for this purpose. The first literature report on ruthenium catalysts for olefin metathesis with CAAC ligands dates back to 2005 [Bertrandt et al. Angew. Chem. Int. Ed., 2005, 44, 5705-5709*].* In this scientific publication, Bertrandt described for the first time the CAAC ligands and their use in organic synthesis. In a subsequent publication from 2007 [Bertrandt et al. Angew. Chem. Int. Ed., 2007, 46,7262-7265*],* Bertrandt described first method of the synthesis of ruthenium complexes with CAAC ligands. In both cases, the ligands contained 2,6-diisopropylbenzene at the nitrogen atom, and either two methyl (Ru5) or cyclohexyl substituent (Ru10) at the carbon atom C2. Next, the team of Prof. Bertrand, in cooperation with the team of Prof. Grubbs, synthesized and tested 17 catalysts in terms of their activity in the ethenolysis reaction of methyl oleate, in which the CAAC ligand contained symmetric substituents at the nitrogen atom: mesityl, 2,6-diisopropylbenzene, 2,6-diethylbenzene, as well as asymmetric ones 2-ethyl-6-methylbenzene, 2-isopropyl-6-methylbenzene, 2-methyl-6-tertbutylbenzene, while at the C2 carbon atom, two of the following substituents: methyl, ethyl, n-propyl, cyclohexyl, adamantyl or phenyl [Bertrandt et al. Angew. Chem. Int. Ed., 2015, 54, 1919-1923]*.* These catalysts showed high activity towards the C-C double bond of methyl oleate in the presence of ethylene overpressure, leading to the formation of valuable industrial products in the olefin metathesis reaction: 1-decene and 9-decenoic acid methyl ester.

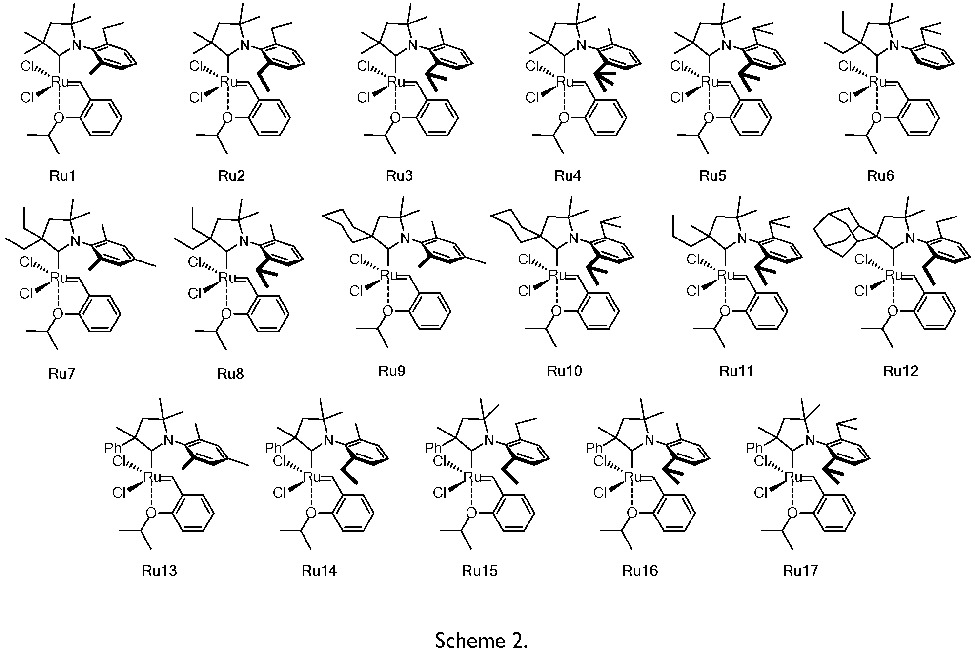

In 2017, Gawin et al. first published a method for a synthesis of indenylidene-type complexes with two CAAC ligands [Gawin et al., Angew. Chem. Int. Ed. 2017, 56, 981-986*;* EP3356379B1]*.* The publication presents a method for the preparation of indenylidene complexes with two CAAC ligands, and tests the activity of the complexes in selected reactions including macrocyclisation, ethenolysis, and cross metathesis reaction of α-olefins. This document also discloses a novel approach to the synthesis of Hoveyda-Grubbs catalysts with CAAC ligands, involving thermal dissociation of one CAAC ligand in an indenylidene complex, followed by reaction of the intermediate with the relevant styrene.

Vanessa M. Marx et. al. discloses CAAC ruthenium complexes which are remarkably active catalysts for ethenolysis [Vanessa M. Marx et. al.Angew. Chem. Int. Ed., 2014, 54(6), 1919-1923].

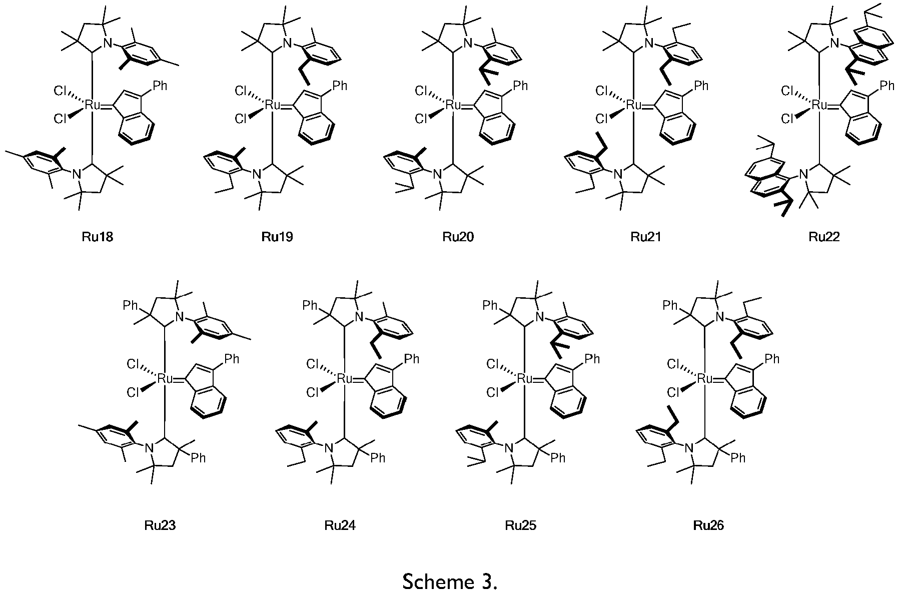

Also in 2017, Gawin et al. published a method to synthesize Hoveyda-Grubbs analogues with a nitro group in the *para* position as substrates using bis-CAAC complexes [Gawin et al. ACS Catal. 2017, 7, 5443-5449*].* The complexes proved to be effective in macrocyclization and cross-metathesis reactions with acrylonitrile.

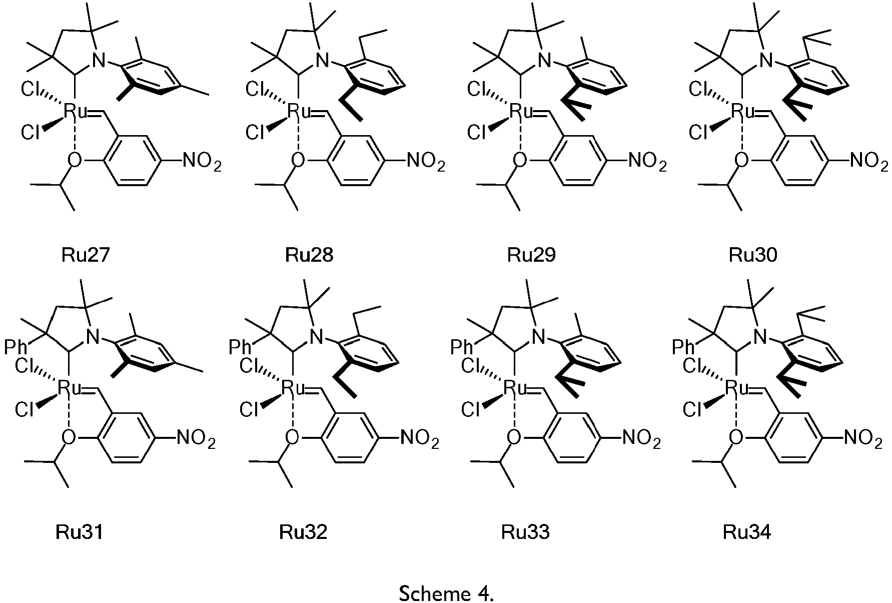

The European patent [EP3356379B1] discloses the structures Ru35-Ru37 with a modified benzylidene fragment.

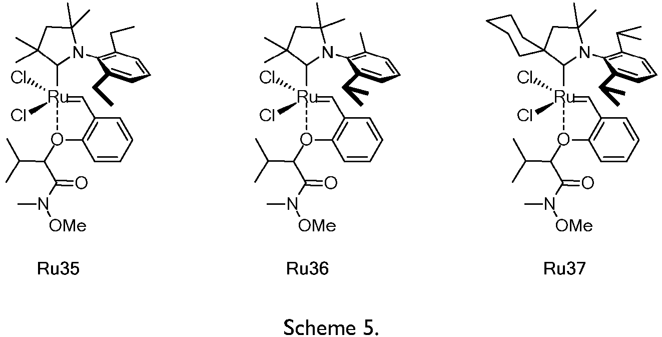

Subsequent modifications of ruthenium catalysts involved Hoveyda-Grubbs type complexes, wherein the hydrogen atom in the styrene part was replaced with an EWG or an EDG group. By modulating the nature of the styrenyl ether ligand, Mignagni et al. studied the reactivity of Ru38 and Ru39 catalysts [FR2947189A1*;* FR2934178A1]. It was shown that the presence of amino group donating electrons has a negative impact on the catalytic activity. On the other hand, the modification of Ru40 with an electron acceptor SO₂NMe₂ group (Zhan type catalyst) enabled the synthesis of an extremely active catalyst in the ethenolysis reaction of fatty acids [EP1905777B1*;* US2011/0306815A1]*.*

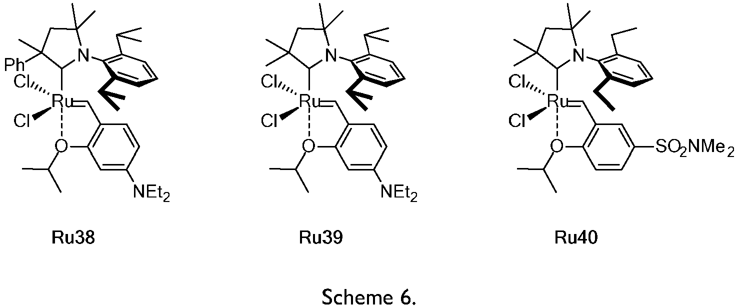

Verpoort et al. studied the impact of labile chelating groups: benzyl ether, benzyl thioether, and benzylamine [WO2017/185324A1]. All catalysis transformed methyl oleate with high selectivity and high TON values [*Turnover number* - the number of catalytic cycles, the number of moles of substrate undergoing reactions, calculated per one mole of the catalyst] (from 180,000 to 210,000). A reaction performed with ethylene (99.995%), in the presence of Ru43 chelated with benzylamine and an activator (HSiCl₃), yielded the highest recorded thus far TON value (390,000).

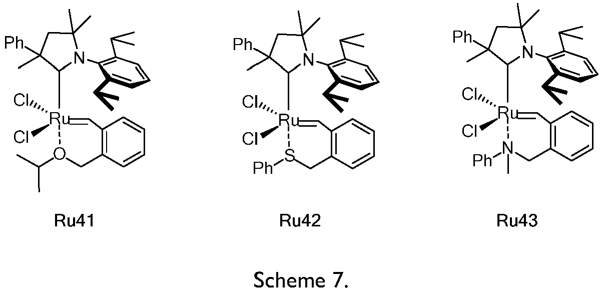

Lemcoff et al. showed that analogues of Hoveyda-Grubbs type catalysts chelated with sulfur in the benzylidene part are present in the cis/trans pairs Ru44-Ru47. The activity of complexes was studied i.e. in polymerization reactions of norbornene derivatives [Rozenberg, I. et al. ACS Catal. 2018, 8, 8182-8191].

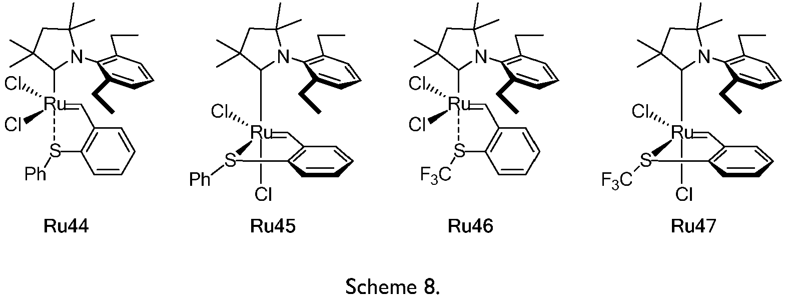

The limited availability of a wide range of structurally various aldehydes used as substrates in the synthesis of CAAC ligands is a significant problem known in state-of-the-art. Their use is mostly limited to simple aldehydes comprising isobutanal or 2-phenylpropanal. The high prices and low availability of other aldehyde derivatives, especially those with heterocyclic rings, as well as long and complicated synthesis pathways for these and other derivatives, in which the expected products are obtained with low yields, significantly limit the possibility of designing novel ruthenium catalysts containing modified CAAC ligands. These characteristics pose a significant limitation to the further development of organometallic catalysis based on ruthenium with novel CAAC ligands. Obtaining novel catalysts for olefin metathesis with ligands based, in particular, on various types of aldehydes with planned properties is becoming difficult to implement in chemical synthesis and economically unjustifiable in industrial scale-up.

In the search for novel ruthenium complexes with high catalytic activity and improved stability and selectivity enabling high TON values to be obtained, it is important that convenient synthetic paths based on easily available and inexpensive substrates lead to these compounds. From the point of view of industrial scaling, it is also important for the planned synthesis to be efficient at every stage and that the reaction products can be purified in a simple way, using techniques such as crystallization or distillation. It is also important to expand the library of ligands, the use of which will comprise an alternative and/or improved source of structures of ruthenium complexes used as catalysts in i.e. ethenolysis reactions of ester derivatives of fatty acids.

From an industrial point of view, it is extremely important that the activity of the new ruthenium complexes allows ethenolysis processes to be carried out with unpurified vegetable oils through a multi-step, laborious procedure of purifying raw materials from compounds that deactivate active catalyst molecules, such as oxygen, water, amines, sulfur compounds, halogenated compounds, peroxides, and others. It is equally important from an industrial point of view that the metathesis process can be carried out with ethylene available in an industrial installation, and not just commercially available high-purity ethylene (ethylene 3.0 = 99.9% purity or ethylene 4.5 = 99.995% purity). It is worth emphasizing that it is not possible to reproduce and apply under industrial conditions the procedures carried out in a glovebox under argon atmosphere, which are described in reputable journals by numerous respected scientists. Therefore, it is essential to search for novel ruthenium catalysts that, on the one hand, will be robust under reaction conditions, and at the same time active and efficient for performing the metathesis process.

Equally important is that transport and storage of catalysts should be easy and possible under normal atmospheric conditions, without the use of inert gases and low temperatures. Such properties of catalysts expected by the industry will enable their easy use on a large industrial scale, which will not require complex apparatus.

It was surprisingly discovered that complexes containing CAAC ligands with a heteroaromatic substituent were found to exhibit the properties desired and sought after by industry. Unexpectedly, it turned out that the ethenolysis processes catalyzed by the novel ruthenium complexes tolerate the presence of oxygen, so that the apparatus can be assembled in air, moreover, ethylene of lower purity than in the processes known in the literature can be used, as well as derivatives of fatty acids esters (oils of plant origin) without the need for a long purification process to high purity of raw materials.

### Summary of the invention

The subject of this invention is a precursor of cyclic alkyl amine carbenes (CAAC) with the formula **CAAC-1** 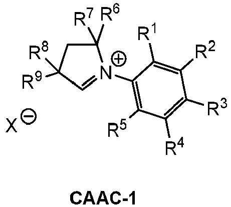 in which
X denotes an anion selected from a group comprising a halogen anion, BF₄⁻, PF₆⁻, ClO₄⁻, CF₃SO₂O⁻; R¹, R², R³, R⁴ and R⁵ denote independently a hydrogen atom, a C₁-C₁₂ alkyl group, a C₃-C₁₂ cycloalkyl group, a C₅-C₂₀ aryl group or a C₅-C₂₀ heteroaryl group, a C₅-C₂₅ aralkyl group, which may be independently substituted by one and/or more substituents selected from a group comprising a hydrogen atom, a halogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxy group, a C₅-C₂₄ aryloxy group, a C₅-C₂₀ heteroaryloxy group, a sulfide group (-SR"), an amino group (-NR"₂), in which the R" group independently denotes a hydrogen atom, C₁-C₅ alkyl, C₆-C₂₄ aryl, C₇-C₂₄ aralkyl,
alternatively R¹, R², R³, R⁴ and R⁵ are connected and form a C₅-C₂₅ ring;
wherein each substituent R⁶, R⁷, and R⁸ denotes a hydrogen atom, a halogen atom, a C₁-C₁₂ alkyl group or a C₅-C₂₀ aryl group, which may be independently substituted by one and/or more substituents selected from a group comprising a hydrogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxy group, a C₅-C₂₄ aryloxy group, a C₅-C₂₀ heteroaryloxy group or a halogen atom, a sulfide group (-SR"), an amino group (-NR"₂), in which the R" group independently denotes a hydrogen atom, C₁-C₅ alkyl, C₆-C₂₄ aryl, C₇-C₂₄ aralkyl;
substituent R⁹ denotes a substituted or unsubstituted heterocyclic group or an organometallic complex group such as ferrocene, which may be independently substituted by one and/or more substituents selected from a group comprising a hydrogen atom, a hydrogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxy group, a C₅-C₂₄ aryloxy group, a C₅-C₂₀ heteroaryloxy group, a sulfide group (-SR"), an amino group (-NR"₂), in which the R" group independently denotes a hydrogen atom, C₁-C₅ alkyl, C₆-C₂₄ aryl, C₇-C₂₄ aralkyl,
alternatively, R⁶ and R⁷ and/or R⁸ and R⁹ are connected and form a C₅-C₂₅ ring.

Preferably the substituent R⁹ denotes a substituted or unsubstituted heterocyclic group selected from a heterocyclic group selected from thiophene, benzothiophene, furan, benzofuran, pyrrole, benzopyrrole, aziridine, oxirane, thiirane, azetidine, oxetane, thietane, pyrrolidine, tetrahydrofuran, tetrahydrothiophene, piperidine, tetrahydropyran, thiane, pyridine, azepane, oxepane, thiepane, azepine, oxepin, thiepine, oxazole, imidazole, thiazole, isoxazole, pyrazole, isothiazole, triaryne, pyrrolidine, pyridine, pyrimidine, hydantoin, quinoline, isoquinoline, chromonyl, coumarin, indole, indolysin, indazole, purine, quinolysine, isoquinol, quinol, phthalazine, naphthyridine, carbazole, β-carboline, or substitute R⁹ denotes a substituted or unsubstituted organometallic complex group comprising cyclopentyl rings and metal atom selected from iron, cobalt, nickel, chromium, titanium, zirconium (metallocenes).

Preferably the substituent R⁹ denotes a substituted or unsubstituted heterocyclic group selected from thiophene, benzothiophene, furan, benzofuran or ferrocene.

Preferably, the precursor defined above has a structure represented by the formula **L1, L2, L3, L4, L5, L6, L7, L8, L9, L10, L11, L12, L13, L14, L15, L16, L17, L18, L19, L20, L21 or L22:**

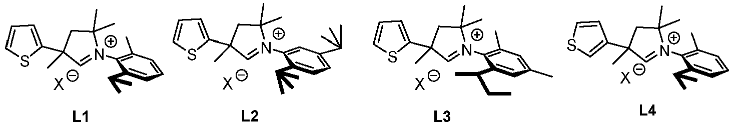

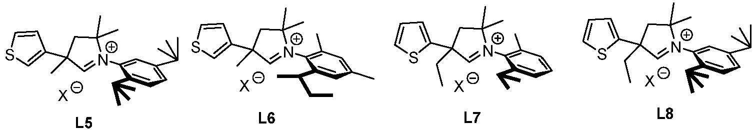

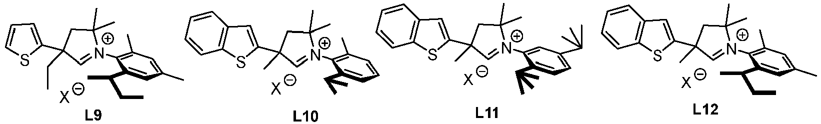

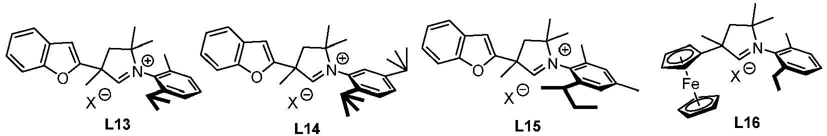

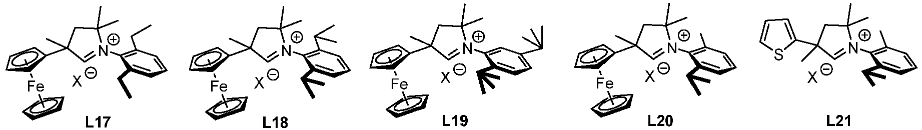

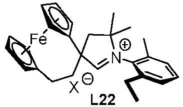

The subject of the invention is also a ruthenium complex of formula **1-Ru** 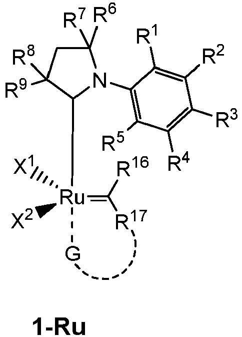 in which:
X¹ and X² independently from each other denote an anionic ligand selected from a group comprising a halogen anion, a group -CN, -SCN, -OR^{a}, -SR^{a}, -O(C=O)R^{a}, -O(SO₂)R^{a} and -OSi(R^{a})₃, in which R^{a} denotes C₁-C₁₂ alkyl, C₃-C₁₂ cycloalkyl, C₂-C₁₂ alkenyl or C₅-C₂₀ aryl, which is optionally substituted by at least one C₁-C₁₂ alkyl, C₁-C₁₂ perfluoroalkyl, C₁-C₁₂ alkoxyl, C₅-C₂₄ aryloxyl, C₅-C₂₀ heteroaryloxyl or a halogen atom;
R¹, R², R³, R⁴ and R⁵ denote independently a hydrogen atom, a C₁-C₁₂ alkyl group, a C₃-C₁₂ cycloalkyl group, a C₅-C₂₀ aryl group or a C₅-C₂₀ heteroaryl group, a C₅-C₂₅ aralkyl group, which may be independently substituted by one and/or more substituents selected from a group comprising a hydrogen atom, a halogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxy group, a C₅-C₂₄ aryloxy group, a C₅-C₂₀ heteroaryloxy group, a sulfide group (-SR"), an amino group (-NR"₂), in which the R" group independently denotes a hydrogen atom, C₁-C₅ alkyl, C₆-C₂₄ aryl, C₇-C₂₄ aralkyl, alternatively R¹, R², R³, R⁴ and R⁵ are connected and form a C₅-C₂₅ ring;
each substituent R⁶, R⁷, and R⁸ denotes a hydrogen atom, a halogen atom, an alkyl C₁-C₁₂ group or a C₅-C₂₀ aryl group, which may be substituted independently by one and/or more substituents selected from a group comprising a hydrogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxy group, a C₅-C₂₄ aryloxy group, a C₅-C₂₀ heteroaryloxy group or a halogen atom, a sulfide group (-SR"), an amino group (-NR"₂), in which the R" group independently means a hydrogen atom, C₁-C₅ alkyl, C₆-C₂₄ aryl, C₇-C₂₄ aralkyl;
substituent R⁹ denotes a substituted or unsubstituted heterocyclic group and/or organometallic complex group which may be independently substituted by one and/or more substituents selected from a group comprising a hydrogen atom, a halogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxy group, a C₅-C₂₄ aryloxy group, a C₅-C₂₀ heteroaryloxy group, a sulfide group (-SR"), an amino group (-NR"₂), in which the R" group independently denotes a hydrogen atom, C₁-C₅ alkyl, C₆-C₂₄ aryl, C₇-C₂₄ aralkyl,
alternatively R⁶ and R⁷ and/or R⁸ and R⁹ are connected and form a C₅-C₂₅ ring;
R¹⁶ and R¹⁷ denote independently a hydrogen atom, a halogen atom, C₁-C₂₅ alkyl, C₃-C₁₂ cycloalkyl, C₁-C₁₂ perfluoroalkyl, C₂-C₂₅ alkene, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₅ aryl, C₅-C₂₅ aryloxyl, C₆-C₂₅ arylalkyl, C₅-C₂₅ heteroaryl, C₅-C₂₅ heteroaryloxyl, C₅-C₂₅ perfluoroaryl, a 3-12-membered heterocycle comprising a sulfur, oxygen, nitrogen, selenium or a phosphorus atom;
wherein the substituents R¹⁶ and R¹⁷ may be connected forming a ring selected from a group comprising C₃-C₂₅ cycloalkyl, C₃-C₂₅ cycloalkenyl, C₃-C₂₅ cycloalkynyl, C₅-C₂₅ aryl, C₅-C₂₅ heteroaryl, C₅-C₂₅ perfluoroaryl, a 3-12-membered heterocycle containing a sulfur, oxygen, nitrogen, selenium or phosphorus atom, which may be independently substituted by one or more substituents selected from a group comprising a hydrogen atom, a halogen atom, C₁-C₂₅ alkyl, C₃-C₂₅ cycloalkyl, C₁-C₁₂ perfluoroalkyl, C₂-C₂₅ alkene, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₅ aryl, C₅-C₂₅ aryloxyl, C₆-C₂₅ arylalkyl, C₅-C₂₅ heteroaryl, C₅-C₂₅ heteroaryloxyl, C₅-C₂₅ perfluoroaryl, a 3-12-membered heterocycle;
G is selected from such as
   - a ligand with the formula **CAAC-1** 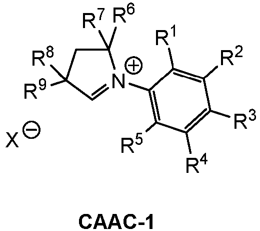 in which X and the substituents R¹ to R⁹ have the meanings defined above
      or
   - heteroatom 1
selected from a group comprising an oxygen, sulfur or selenium atom, substituted by a group selected from such as a hydrogen atom, a halogen atom, an oxygen atom, C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or heteroaryloxyl C₅-C₂₄, a 3-12-membered heterocycle, optionally substituted by an acyl group (-COR'), a cyano group (-CN), a carboxyl group (-COOH), an ester group (-COOR'), an ester group (-CH₂COOR'), an ester group (-CHR'COOR'), an ester group (-C(R')₂COOR'), an amide group (-CONR'₂), a Weinreb-type amide (-CON(R')(OR')), a sulfone group (-SO₂R'), a formyl group (-CHO), a sulfonamide group (-SO₂NR'₂), a ketone group (-COR'), a thioamide group (-CSNR'₂), a thioketone (-CSR'), a thionoester group (-CSOR'), a thioester group (-COSR'), a dithioester group (-CS₂R'), in which the group R' denotes independently C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl, C₅-C₂₄ heteroaryloxyl and then the dashed line denotes a direct bond between a heteroatom and the substituent R¹⁷ or it denotes a bond between the substituent R¹⁷ and a heteroatom via a methylene bridge -CH₂-, -CHR'-, or -CR'₂- wherein the substituent R¹⁴ is C₅-C₁₅ aryl, optionally substituted by 1-4 substituents independently selected from a group comprising a hydrogen atom, a halogen atom, C₁-C₂₅ alkyl, C₃-C₂₅ cycloalkyl, C₂-C₂₅ alkenyl, C₃-C₂₅ cycloalkenyl, C₂-C₂₅ alkynyl, C₃-C₂₅ cycloalkynyl, C₁-C₂₅ perfluoroalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₆-C₂₀ heteroaryl or C₅-C₂₄ heteroaryloxyl, a 3-12-membered heterocycle, an alkoxyl group (-OR"), a sulfide group (-SR"), a sulfoxide group (-S(O)R"), a sulfonium group (-S⁺R"₂), a sulfone group (-SO₂R"), a sulfonamide group (-SO₂NR"₂), an amino group (-NR"₂), an ammonium group (-N⁺R"₃), a nitro group (-NO₂), a cyano group (-CN), a phosphinous group (-P(O)(OR")₂), a phosphinic group (-P(O)R"(OR")), a phosphonine group (-P(OR")₂), a phosphine group (-PR"₂), a phosphine oxide group (-P(O)R"₂), a phosphonium group (-P⁺R"₃), a carboxyl group (-COOH), an ester group (-COOR"), an amide group (-CONR"₂), an amide group (-NR"C(O)R"), a formyl group (-CHO), a ketone group (-COR"), a thioamide group (-CSNR"₂), a thioketone group (-CSR"), a thionoester group (-CSOR"), a thioester group (-COSR"), a dithioester group (-CS₂R"), in which the R" group denotes C₁-C₅ alkyl, C₁-C₅ perfluoroalkyl, C₆-C₂₄ aryl, C₇-C₂₄ aralkyl, C₅-C₂₄ perfluoroaryl, or
   -heteroatom 2
selected from a group comprising a nitrogen or a phosphorus atom, substituted by the group selected from such as a hydrogen atom, methylidene, optionally substituted by the substitute R', C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, a 3-12-membered heterocycle, an acyl group (-COR'), an ester group (-COOR'), a tert-butyloxycarbonyl group (t-Boc) or a 9-fluorenylmethoxycarbonyla group (Fmoc), a carbamine group (-CONR'₂), a sulfone group (-SO₂R'), a formyl group (-CHO), in which the R' group denotes C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, optionally substituted with an acyl group (-COR'), a cyano group (-CN), a carboxyl group (-COOH), an ester group (-COOR'), an ester group (-CH₂COOR'), an ester group (-CHR'COOR'), an ester group (-C(R')₂COOR'), an amide group (-CONR'₂), a sulfone group
   (-SO₂R'), a formyl group (-CHO), a sulfonamide group (-SO₂NR'₂), a ketone group (-COR'), a thioamide group (-CSNR'₂), a thioketone group (-CSR'), a thionoester group (-CSOR'), a thioester group (-COSR') or a dithioester group (-CS₂R'), in which the R' group denotes C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, and then the dashed line denotes a direct bond between the heteroatom and the substituent R¹⁴ or it denotes a bond between the substituent R¹⁷ with a heteroatom via methylene bridge (CH₂)-, -(CHR')-, or -(CR'₂)-; wherein the substituent R¹⁷ is C₅-C₁₅ aryl, optionally substituted with 1-4 substituents independently selected from the group comprising a hydrogen atom, a halogen atom, C₁-C₂₅ alkyl, C₃-C₂₅ cycloalkyl, C₂-C₂₅ alkenyl, C₃-C₂₅ cycloalkenyl, C₂-C₂₅ alkynyl, C₃-C₂₅ cycloalkynyl, C₁-C₂₅ perfluoroalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₆-C₂₀ heteroaryl or C₅-C₂₄ heteroaryloxyl, a 3-12-membered heterocycle, an alkoxyl group (-OR"), a sulfide group (-SR"), a sulfoxide group (-S(O)R"), a sulfonium group (-S⁺R"₂), a sulfone group (-SO₂R"), a sulfonamide group (-SO₂NR"₂), an amino group (-NR"₂), an ammonium group (-N⁺R"₃), a nitro group (-NO₂), a cyano group (-CN), a phosphinous group (-P(O)(OR")₂), a phosphinic group (-P(O)R"(OR")), a phosphonine group (-P(OR")₂), a phosphine group (-PR"₂), a phosphine oxide group (-P(O)R"₂), a phosphonium group (-P⁺R"₃), a carboxyl group (-COOH), an ester group (-COOR"), an amide group (-CONR"₂), an amide group (-NR"C(O)R"), a formyl group (-CHO), a ketone group (-COR"), a thioamide group (-CSNR"₂), a thioketone group (-CSR"), a thionoester group (-CSOR"), a thioester group (-COSR") or a dithioester group (-CS₂R"), in which the group R" denotes C₁-C₅ alkyl, C₁-C₅ perfluoroalkyl, C₆-C₂₄ aryl, C₇-C₂₄ aralkyl, C₅-C₂₄ perfluoroaryl, or
- heteroatom 3
selected from a group comprising a halogen atom and then the dashed line denotes a direct bond between the heteroatom and the R¹⁷ substituent, wherein the R¹⁷ substituent is C₅-C₁₅ aryl, or C₅-C₂₅ polyaryl, optionally substituted with 1-4 substituents selected independently from a group comprising a hydrogen atom, a halogen atom, C₁-C₂₅ alkyl, C₃-C₂₅ cycloalkyl, C₂-C₂₅ alkenyl, C₃-C₂₅ cycloalkenyl, C₂-C₂₅ alkynyl, C₃-C₂₅ cycloalkynyl, C₁-C₂₅ perfluoroalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₆-C₂₀ heteroaryl or C₅-C₂₄ heteroaryloxyl, a 3-12-membered heterocycle, an alkoxy group (-OR"), a sulfide group (-SR"), a sulfoxide group (-S(O)R"), a sulfonium group (-S⁺R"₂), a sulfone group (-SO₂R"), a sulfonamide group (-SO₂NR"₂), an amino group (-NR"₂), an ammonium group (-N⁺R"₃), a nitro group (-NO₂), a cyano group (-CN), a phosphonous group (-P(O)(OR")₂), a phosphinous group (-P(O)R"(OR")), a phosphonine group (-P(OR")₂), a phosphine group (-PR"₂), a phosphine oxide group (-P(O)R"₂), a phosphonium group (-P⁺R"₃), a carboxyl group (-COOH), an ester group (-COOR"), an amide group (-CONR"₂), an amide group (-NR"C(O)R"), a formyl group (-CHO), a ketone group
(-COR"), a thioamide group (-CSNR"₂), a thioketone group (-CSR"), a thionoester group (-CSOR"), a thioester group (-COSR"), a dithioester group (-CS₂R"), in which the R" group denotes C₁-C₅ alkyl, C₁-C₅ perfluoroalkyl, C₆-C₂₄ aryl, C₇-C₂₄ aralkyl, C₅-C₂₄ perfluoroaryl.

The ruthenium complex is preferably represented by the formula**1a-Ru** 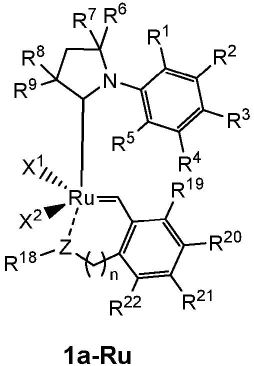 in which X¹ and X² and the R¹, R², R³, R⁴, R⁵, R⁶, R⁷, R⁸, R⁹ substituents have the meanings defined above
'n' means 1 or 0
Z is selected from a group comprising halogen atoms, O atom, S atom, Se atom, or a NR‴ group, in which R‴ denotes methylidene, C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, a 3-12-membered heterocycle, an acyl group (-COR'), an ester group (-COOR'), a tert-butylcarboxycarbon group (t-Boc) or a 9-fluorenylmethoxycarbonyl group (Fmoc), a carbamine group (-CONR'₂), a sulfone group (-SO₂R'), a formyl group (-CHO), in which the R' group denotes C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl,
or a halogen atom, wherein if Z denotes a halogen atom, R¹⁸ is absent;
R¹⁸ means independently a hydrogen atom, C₁-C₂₅ alkyl, C₁-C₂₅ cycloalkyl, C₅-C₂₀ alkoxyl, C₅-C₂₀ aryl, C₅-C₂₄ aryloxyl, a -COOR‴ group, a -CH₂COOR‴ group, a -CONR‴₂ group, a -CH₂CONR‴₂ group, a -COR‴ group, a -CH₂COR‴ group, a -CON(OR‴)(R‴) group, a -CH₂CON(OR‴)(R‴) group or a halogen atom, wherein R‴ denotes C₁-C₁₂ alkyl, C₃-C₁₂ cycloalkyl, C₂-C₁₂ alkenyl, C₆-C₂₀ aryl, which are optionally substituted with at least one C₁-C₁₂ alkyl, C₁-C₁₂ perfluoroalkyl, C₁-C₁₂ alkoxyl, C₆-C₂₄ aryloxyl, or a halogen atom;
R¹⁹, R²⁰, R²¹, and R²² denote independently a hydrogen atom, a halogen atom, a C₁-C₂₅ alkyl group, a C₂-C₂₅ alkenyl group, a C₅-C₂₅ aryl group, an alkoxy group (-OR"), a sulfide group (-SR"), a sulfoxide (-S(O)R"), a sulfonium group (-S⁺R"₂), a sulfone group (-SO₂R"), a sulfonamide group (-SO₂NR"₂), an amino group (-NR"₂), an ammonium group (-N⁺R"₃), a nitro group (-NO₂), a cyano group (-CN), a phosphonous group (-P(O)(OR")₂), a phosphinous group (-P(O)R"(OR")), a phosphonine group (-P(OR")₂), a phosphine group (-PR"₂), a phosphine oxide group (-P(O)R"₂), a phosphonium group (-P⁺R"₃), a carboxyl group (-COOH), an ester group (-COOR"), an amide group (-CONR"₂), an amide group (-NR"C(O)R'), a formyl group (-CHO), a ketone group (-COR"), a thioamide group (-CSNR"₂), a thioketone group (-CSR"), a thionoester group (-CSOR"), a thioester group (-COSR"), a dithioester group (-CS₂R"), in which the R" group denotes C₁-C₅ alkyl, C₁-C₅ perfluoroalkyl, C₆-C₂₄ aryl, C₇-C₂₄ aralkyl, C₅-C₂₄ perfluoroaryl, wherein the R¹⁶, R¹⁷, R¹⁸ and R¹⁹ substituents may be connected, thus forming a substituted or unsubstituted C₄-C₁₀ cyclic or C₄-C₁₂ polycyclic system.

The ruthenium complex is preferably represented by the formula**1b-Ru** 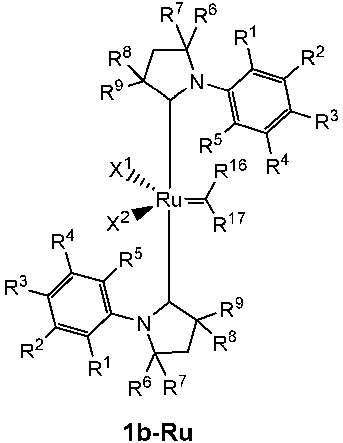 in which X¹ and X² and the R¹, R², R³, R⁴, R¹, R⁶, R⁷, R⁸, R⁹ substituents have the meanings defined above
R¹⁶ and R¹⁷ independently denote a hydrogen atom, a halogen atom, C₁-C₂₅ alkyl, optionally substituted; C₃-C₂₅ cycloalkyl, optionally substituted; C₁-C₁₂ perfluoroalkyl, optionally substituted; C₂-C₂₅ alkene, optionally substituted; C₂-C₂₅ alkenyl, optionally substituted; C₃-C₂₅ cycloalkenyl, optionally substituted; C₂-C₂₅ alkynyl, optionally substituted; C₃-C₂₅ cycloalkynyl, optionally substituted; C₁-C₂₅ alkoxyl, optionally substituted; C₅-C₂₅ aryl, optionally substituted; C₅-C₂₅ aryloxyl, optionally substituted; C₆-C₂₅ arylalkyl, optionally substituted; C₅-C₂₅ heteroaryl, optionally substituted; C₅-C₂₅ heteroaryloxyl, optionally substituted; C₅-C₂₅ perfluoroaryl, optionally substituted; a 3-12-membered heterocycle containing a sulfur, oxygen, nitrogen, selenium or a phosphorus atom, optionally substituted;
wherein the R¹⁶ and R¹⁷ substituents may be connected, forming a ring selected from a group comprising C₃-C₂₅ cycloalkyl, cycloalkenyl C₃-C₂₅, C₃-C₂₅ cycloalkynyl, C₅-C₂₅ aryl, C₅-C₂₅ heteroaryl, C₅-C₂₅ perfluoroaryl, a 3-12-membered heterocycle comprising a sulfur, oxygen, nitrogen, selenium or a phosphorus atom, which may be independently substituted with one or more substituents selected from a group comprising a hydrogen atom, a halogen atom, C₁-C₂₅ alkyl, C₃-C₂₅ cycloalkyl, C₁-C₁₂ perfluoroalkyl, C₂-C₂₅ alkene, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₅ aryl, C₅-C₂₅ aryloxyl, C₆-C₂₅ arylalkyl, C₅-C₂₅ heteroaryl, C₅-C₂₅ heteroaryloxyl, C₅-C₂₅ perfluoroaryl, a 3-12-membered heterocycle.

The ruthenium complex is preferably represented with the formulas **Ru1a, Ru2a, Ru3a, Ru4a, Ru5a, Ru6a, Ru7a, Ru8a, Ru9a, Ru10a, Ru11a, Ru11a, Ru12a, Ru13a, Ru14a, Ru15a, Ru16a, Ru17a, Ru18a, Ru19a, Ru20a, Ru21a, Ru22a:**

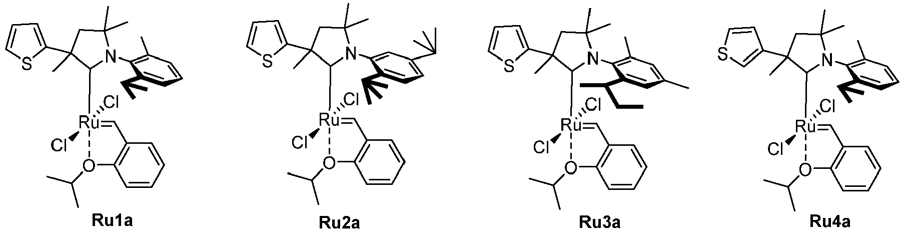

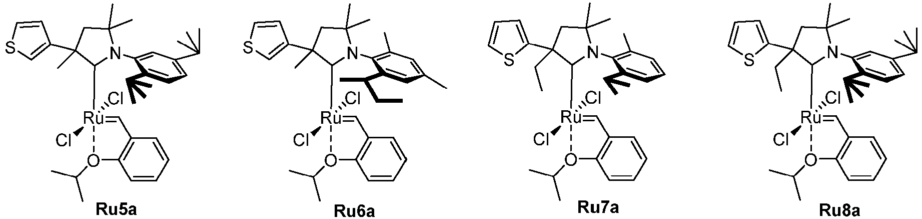

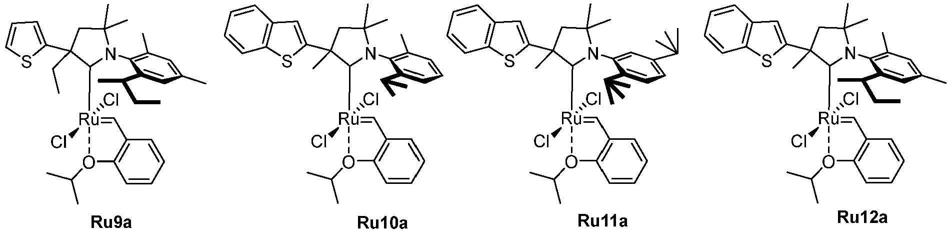

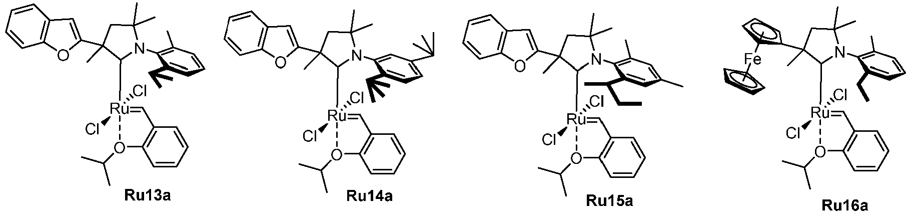

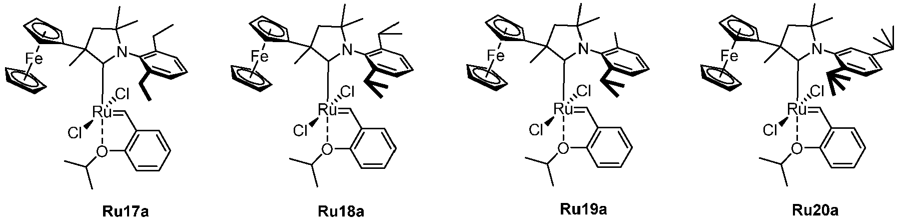

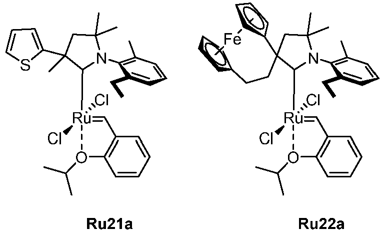

The subject of the invention is also a method of obtaining a ruthenium complex of formula **1a-Ru** 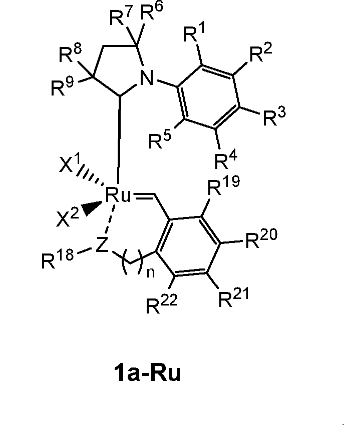 defined above, in which the alkylidene ruthenium complex with the formula 10 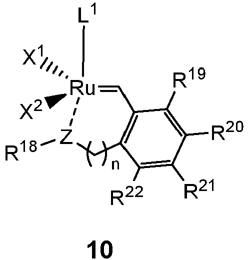 in which:
L¹ denotes a neutral ligand selected from a group comprising pyridine or substituted pyridine, P(R')₃, P(OR')₃, O(R')₂, N(R')₃, wherein each R' independently denotes C₁-C₁₂ alkyl, C₃-C₁₂ cycloalkyl, C₅-C₂₀ aryl, C₇-C₂₄ aralkyl, C₅-C₂₄ perfluoroaryl, a 5-12-membered heteroaryl;
N, Z, X¹, X² and the substituents R¹⁸, R¹⁹, R²⁰, R²¹ and R²² have the meanings defined above
undergoes reaction with carbene of formula 8 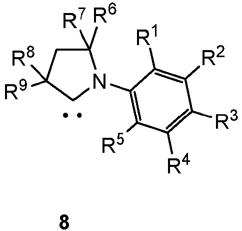 in which the substituents R¹ to R⁹ have the meanings defined above.

The subject of the invention also relates to the use of the compound of formula **1-Ru,** defined above, as a precatalyst and/or a catalyst in olefin metathesis reactions, in particular in ring-closing metathesis (RCM), cross-metathesis (CM), homometathesis (cross-metathesis between two molecules of the same olefin), ethenolysis, isomerization, in the reaction of diastereoselective ring-rearrangement metathesis (DRRM) reactions, 'alkene-alkyne' (en-yn) metathesis or ROMP or ADMET polymerization reactions.

The reaction is preferably carried out in an organic solvent, such as toluene, mesitylene, hexane, cyclohexane, ethyl acetate, methyl acetate, methyl carbonate, ethyl carbonate, tert-butyl-methyl ether, cyclopentyl-methyl ether, diethyl ether, THF, 2-Me-THF, 4-Me-THP, dioxane, DME, PAO. PEG, paraffin, esters of saturated fatty acids.

The reaction is preferably carried out in a solvent-free system.

The reaction is preferably carried out at a temperature between 20 °C and 200 °C.

The reaction is preferably carried out over a period between 5 minutes and 48 hours.

The **1-Ru** compound is preferably used in a quantity not greater than 10 mol%.

The **1-Ru** compound is preferably used in a quantity not greater than 0.1 mol%.

The **1-Ru** compound is preferably added to the reaction mixture in aliquots, as a solid and/or continuously, using a pump, as a solution in an organic solvent.

The gaseous by-product of the reaction, selected from ethylene, propylene, butylene, is preferably actively removed from the reaction mixture using inert gas barbotage or under reduced pressure.

The subject of the invention is explained in embodiments in the Figures, in which:

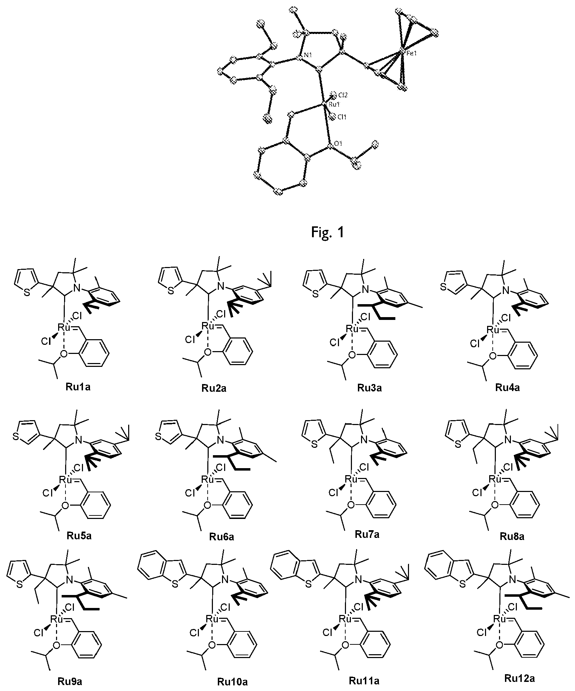

Fig. 1 presents the structure of the **Ru17a** compound obtained on the basis of X-ray structural analysis;

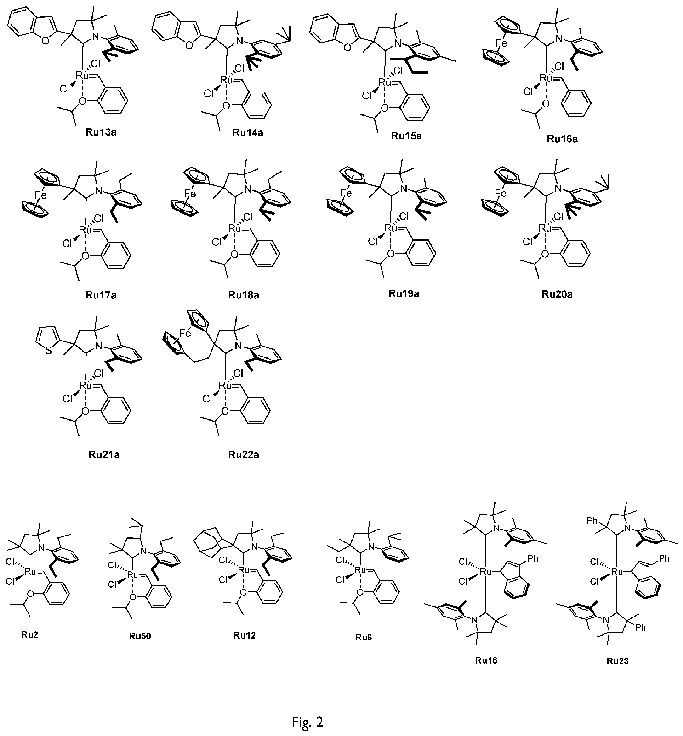

Fig. 2 presents a summary of precatalysts and catalysts of olefin metathesis available on the market, and of novel precatalysts and catalysts according to the invention;
Fig. 3 presents a photography showing: (A) the set up and closed reaction system - autoclave, and (B) the reaction system (autoclave), which is open prior to the ethenolysis reaction and does not require a glovebox at any stage of the reaction process.

In this description, terms used have the following meanings.

Terms undefined in this document have meanings which are provided and understood by the specialist in the field in light of the best knowledge available, this disclosure and the context of the patent application description.

Unless otherwise stated, the following conventions of chemical terms are used in this description and have the meanings indicated as in the definitions below:
As used in this description, the term *"halogen atom"* means an element selected from F, Cl, Br, I.

The term "carbene" means an electrically inert molecule in which the carbon atom has two non-bonding electrons occurring in the singlet or triplet state and is linked by a single covalent bond to two groups or linked by a double covalent bond to one group. The term 'carbene' also includes carbene analogues in which the carbene carbon atom is replaced by another chemical element such as boron, silicon, germanium, tin, lead, nitrogen, phosphorus, sulfur, selenium or tellurium.

The term *"alkyl"* refers to a saturated, linear or branched hydrocarbon substituent with the indicated number of carbon atoms. Examples of alkyl substituents include -methyl, -ethyl, -*n*-propyl, -*n*-butyl, -*n*-pentyl, -*n*-hexyl, -*n*-heptyl, -*n*-octyl, -*n*-nonyl and -*n*-decyl. Representative branched -(C1-C10) alkyls include -isopropyl, -*sec*-butyl, -isobutyl, -*tert*butyl, -isopentyl, -neopentyl, -1-methylbutyl, -2-methylbutyl, -3-methylbutyl, -1,1-dimethylpropyl, -1,2-dimethylpropyl, -1-methylpentyl, -2-methylpentyl, -3-methylpentyl, -4-methylpentyl, -1-ethylbutyl, -2-ethylbutyl, -1,1-dimethylbutyl, -1,2-dimethylbutyl, -1,3-dimethylbutyl, -2,2-dimethylbutyl, -2,3-dimethylbutyl, -3,3-dimethylbutyl, -1-methylhexyl, -2-methylhexyl, -3-methylhexyl, -4-methylhexyl, -1,2-dimethylpentyl, -1,3-dimethylpentyl, -5-methylhexyl, -1,2-dimethylhexyl, -1,3-dimethylhexyl, -3,3-dimethylhexyl, -1,2-dimethylheptyl, -1,3-dimethylheptyl, -3,3-dimethylheptyl and the like.

The term *"alkoxyl"* refers to an alkyl substituent as defined above, connected via an oxygen atom.

The term *"perfluoroalkyl"* denotes an alkyl group as defined above, in which all the hydrogen atoms have been replaced with the same or different halogen atoms.

The term *"cycloalkyl"* refers to a saturated, mono- or polycyclic hydrocarbon substituent with the indicated number of carbon atoms. Examples of cycloalkyl substituent include -cyclopropyl, -cyclobutyl, -cyclopentyl, -cyclohexyl, -cycloheptyl, -cyclooctyl, -cyclononyl, -cyclodecyl and the like.

The term "alkenyl" refers to an unsaturated, linear or branched, acyclic hydrocarbon substituent with the indicated number of hydrogen atoms and containing at least one double carbon-carbon bond. Examples of alkenyl substituents include -vinyl, -allyl, -1-butenyl, -2-butenyl, -isobutenyl, -1-pentenyl, -2-pentenyl, -3-methyl-1-butenyl, -2-methyl-2-butenyl, -2,3-dimethyl-2-butenyl, -1-hexenyl, -2-hexenyl, -3-hexenyl, -1-heptenyl, -2-heptenyl, -3-heptenyl, -1-octenyl, -2-octenyl, -3-octenyl, -1-nonenyl, -2-nonenyl, -3-nonenyl, -1-decenyl, -2-decenyl, -3-decenyl and the like. The term "cycloalkenyl" refers to an unsaturated, cyclic or branched, cyclic hydrocarbon substituent with the indicated number of hydrogen atoms and containing at least one double carbon-carbon bond. Examples of cycloalkenyl substituents include -cyclopropene, -cyclobutene, -cyclopentene, -cyclohexene, -cycloheptene, -cyclooctene, -cyclononene, -cyclodecene, -metylcyclopropene, -ethylcyclobutene, -isopropylcyclo pentene, -methylcyclohexene and the like.

The term *"aryl"* refers to an aromatic, mono- or polycyclic hydrocarbon substituent with the indicated number of carbon atoms. Examples of aryl substituents include -phenyl, -tolyl, -xylyl, -naphthtyl, -2,4,6-trimethylphenyl, -2-fluorophenyl, -4-fluorophenyl, -2,4,6-trifluorophenyl, -2,6-difluorophenyl, -4-nitrophenyl and the like.

The term *"aralkyl"* refers to an alkyl substituent as defined above, substituted with at least one aryl as defined above. Examples of aralkyl substituents include -benzyl, -diphenylmethyl, -triphenylmethyl and the like.

The term "heteroaryl" refers to an aromatic mono- or polycyclic hydrocarbon substituent with the indicated number of carbon atoms, in which at least one carbon atom has been replaced with a heteroatom selected from O, N and S atoms. Examples of heteroaryl substituents include -furyl, -thienyl, -imidazolyl, -oxazolyl, -thiazolyl, -isoxazolyl, -triazolyl, -oxadiazolyl, -thiadiazolyl, - tetrazolyl, -pyridyl, -pyrimidyl, -triazinyl, -indolyl, -benzo[b]furyl, -benzo[b]thienyl, -indazolyl, - benzoimidazolyl, -azaindolyl, -quinolyl, -isoquinolyl, -carbazolyl and the like.

The term "heterocycle" refers to a saturated, unsaturated or partially unsaturated hydrocarbon substituent, with the indicated number of carbon atoms, in which at least one carbon atom has been replaced with a heteroatom selected from O, N and S atoms. Example heterocycle substituents include -furyl, -thiophenyl, -pyrrolyl, -oxazolyl, -imidazolyl, -thiazolyl, -isoxazolyl, -pyrazolyl, -isothiazolyl, -triazinyl, -pyrrolidinonyl, -pyrrolidinyl, -hydantoinyl, -oxiranyl, -oxetanyl, -tetrahydrofuranyl, -tetrahydrothiophenyl, -quinolinyl, -isoquinolinyl, -chromonyl, -coumarinyl, -indolyl,-indolysinyl, -benzo[b]furanyl, -benzo[b]thiophenyl, -indazolyl, -purinyl, -4H-quinolysinyl, - isoquinolyl, -quinolyl, -phtalazinyl, -naphtyridinyl, -carbazolyl, -β-carbolinyl and the like.

The term "neutral ligand" refers to an non-charged substituent capable of coordinating with a metallic center (transition metal atom). Examples of such ligands may include: N-heterocyclic carbenes (NHC), cyclic (alkyl) (amino) carbenes (CAAC), amines, phosphines and their oxides, alkyl and aryl phosphites and phosphates, arsines and their oxides, ethers, alkyl and aryl sulfides, coordinated unsaturated or aromatic hydrocarbons, alkyl and aryl halides, nitriles, isonitriles, sulfides, sulfoxides, sulfones, thioketones, thioamides, thioester, thionoesters and dithioesters.

The term "anionic ligand" refers to a substituent capable of coordinating with a charged metallic center (transition metal atom), which can partially or fully compensate the charge of the metallic center. Examples of such ligands may include fluoride, chloride, bromide, iodide, cyanide, cyanate and thiocyanate anions, carboxylic acid anions, alcohol anions, phenol anions, thiol and thiophenol anions, anions of hydrocarbons with delocalized charge (e.g. cyclopentadiene anion), anions of (organo)sulfuric acids and (organo)phosphoric acids and their esters (such as e.g. anions of alkylsulfonic and arylsulfonic acids, anions of alkylphosphoric and arylphosphoric acids, anions of alkyl and aryl esters of sulfuric acid, anions of alkyl and aryl esters of phosphoric acids, anions of alkyl and aryl esters of alkylphosphoric and arylphosphoric acids).

The term "heteroatom" means an atom selected from the group comprising oxygen sulfur, nitrogen, phosphorous, boron, silicon, arsenic, selenium, tellurium.

The term "PAO" stands for polyolefins, an abbreviation for *Poly-Alpha-Olefins,* in the case of the present invention, an abbreviation used for low molecular weight polyolefins used as high boiling point solvents. It also denotes a class of solvents and/or lubricants that are the product of the polymerization of ethylene derivatives, leading to the formation of branched, saturated hydrocarbons, used here as heat-resistant, non-polar, high boiling point solvents.

### Embodiments of the invention

The following examples are included only to illustrate the invention and to clarify particular aspects of the invention, not to limit it, and should not be equated with the entire scope of the invention as defined in the appended claims. In the following examples, unless otherwise indicated, standard materials and methods used in the field were used or followed manufacturers' recommendations for specific reactants and methods.

When necessary, model compounds for the metathesis reaction were purified by fractional distillation and then stored under inert gas atmosphere over activated neutral aluminum oxide. Tetrahydrofuran was purified by sodium-potassium alloy distillation in the presence of benzophenone, and then stored over 4Å molecular sieves. When expedient, selected reactions were carried out under an argon atmosphere using reaction vessels heated at 130 °C. Aluminum oxide (Al₂O₃, neutral, Brockman Grade I) was activated by heating at 150 °C under reduced pressure for 16 hours.

Starting compounds for the synthesis of aldehyde derivatives were commercially available.

### Example I

### Synthesis of new aldehydes, precursors of CAAC ligands

The following Scheme 9 illustrates the first three steps in the synthesis of CAAC ligand precursors to synthesize ruthenium catalysts for olefin metathesis (with the general formulae **Ru1a** to **Ru21a,** Scheme 9), which are the subject of the present invention.

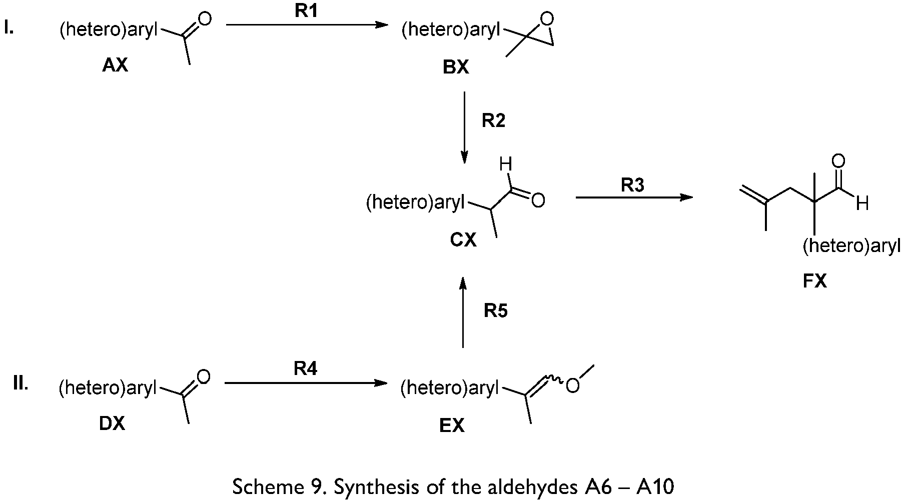

Reactions **R1** through **R5,** shown in Scheme 9, were carried out using commercially available substrates based on procedures described in the literature with modifications developed by the authors of the invention. Unless otherwise written, the reactions described used commercially available solvents and paid no attention to the presence of oxygen and/or moisture.

### Reaction R1

In the **R1** step (Scheme 9), the synthesis of an epoxide with the general formula **BX** is carried out. For this purpose, a ketone of the general formula **AX** and a trimethyl sulfonyl salt, preferably either trimethyl sulfonyl bromide (Me₃SBr) or iodide (Me₃Sl), are used. The transformation is carried out in an organic solvent, preferably acetonitrile (MeCN) or another organic solvent, using a stoichiometric amount of the sulfonium salt and using a metal hydroxide, preferably potassium hydroxide (KOH). The reaction mixture is carried out at an elevated temperature, preferably 60 °C for 2 to 16 hours. The product is separated by filtration from the reaction mixture and distillation of the solvent.

### Embodiment of R1

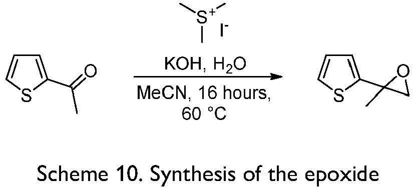

Into a reaction vessel equipped with a stirring element, 2-acetylthiophene (7.01 g, 6.00 mL, 55.0 mmol, 1.0 equiv.), Me₃Sl (14.9 g, 71.5 mmol, 1.3 equiv.), KOH (9.44 g, 0.14 mmol, 2.6 equiv.), distilled water (0.25 mL) and MeCN (55 mL) were placed under an argon atmosphere. The contents of the vessel were stirred for 16 hours at 60 °C. After the reaction was completed, 15 mL of diethyl ether (Et₂O) was added, the precipitate was filtered off and washed again with diethyl ether (40 mL), the solvent was evaporated under reduced pressure. The remaining dark pink oil was washed with diethyl ether (40 mL), the solvent was evaporated and washed with n-hexane (40 mL), the solvent was evaporated under reduced pressure to give the expected product as a yellow oil in 91% yield (6.98 g, 49.8 mmol).

**¹H NMR (400 MHz, CDCl₃) δ ppm:** 7.21 (dd, *J* = 5.1, 1.2 Hz, 1H), 7.04 (dd, *J* = 3.7, 1.3 Hz, 1H), 6.96 (dd, *J* = 5.1, 3.6 Hz, 1H), 3.07 - 3.03 (m, 2H), 1.78 - 1.78 (m, 3H);

**¹³C NMR (101 MHz, CDCl₃) δ ppm:** 145.9, 127.2, 125.0, 124.8, 58.7, 55.2, 22.2;

### Reaction R2

In step **R2** (Scheme 9), the synthesis of an aldehyde of the general formula **CX** is carried out. For this purpose, an epoxide of the general formula **BX** and Lewis acid, preferably SiO₂, chloroform, ZnCl₂ or ZnBr₂ are used. The transformation is carried out in an organic solvent, preferably ethyl acetate (EtOAc), chloroform or toluene (PhMe). The reaction mixture is carried out at room temperature until full conversion of the substrate (2.5 - 48 hours). The product is purified by distillation of the solvent and then used in the next step without further purification.

### Embodiment of performing reaction R2

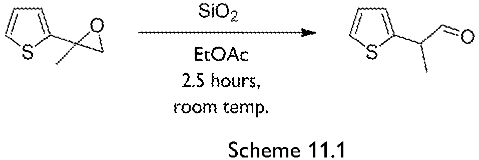

2-Methyl-2-(thiophen-2-yl)epoxide (6.6 g, 47.0 mmol, 1.0 equiv.), silicon oxide (3.76 g) and EtOAc (90 mL) were placed in a reaction vessel equipped with a stirring element. The reaction was carried out for 2.5 hours at room temperature. Silicon oxide was filtered off and solvent was distilled off under reduced pressure, to give the expected product as a yellow oil in 69% yield (2.60 g, 18.5 mmol).

### Alternative embodiment of performing reaction R2

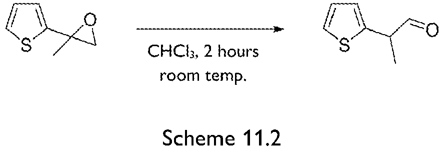

2-Methyl-2-(thiophen-2-yl)epoxide (6.6 g, 47.0 mmol, 1.0 equiv.) and chloroform (60 mL) were placed in a reaction vessel equipped with a stirring element. The reaction was carried out for 2 hours at room temperature. The solvent was distilled off under reduced pressure to give the expected product as a yellow oil in >99% yield (6.59 g, 47.0 mmol).

### Alternative embodiment of performing reaction R2

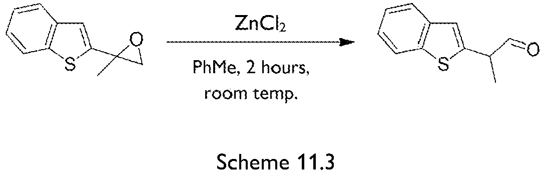

2-Methyl-2-(benzothiophen-2-yl)epoxide (5.32 g, 28.0 mmol, 1 equiv.), zinc chloride (3.81 g, 28.0 mmol, 1 equiv.) and toluene (119 mL) were placed in a reaction vessel equipped with a stirring element. The reaction was carried out for 2 hours at room temperature. The reaction mixture was filtered off on a Schott funnel, the solvent was distilled off under reduced pressure, and the crude product was sublimed under reduced pressure using a Kugelrohr glass oven, to give the expected product as a colorless solid in 97% yield (5.17 g, 27.2 mmol).

### Reaction R3

In step **R3** (Scheme 9), the synthesis of an aldehyde with the general formula **FX** is carried out. For this purpose, an aldehyde of the general formula **CX** and an alkenyl halide, preferably chloride, a metal hydroxide, preferably NaOH, a quaternary ammonium salt, preferably tetra-*N-*butylammonium bromide, are used. The transformation is carried out in an organic solvent, preferably toluene. The reaction mixture is carried out at an elevated temperature, preferably 40 - 60 °C for 30 minutes to 5 hours. The product is separated by extraction from the reaction mixture. It is then dried over a drying agent, preferably sodium sulfate or magnesium sulfate, solid is filtered off, and the solvent is distilled off under reduced pressure. The product is used in the next step without additional purification.

### Embodiment of performing reaction R3

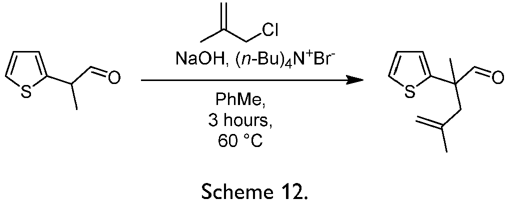

Into a reaction vessel equipped with a stirring element were placed thiophene-2-ylpropanal (6.4 g, 46.0 mmol, 1.00 equiv.), 3-chloro-2-methylpropene (5.1 g, 55.0 mmol, 1.20 equiv.), potassium hydroxide (2.7 g, 69.0 mmol, 1.50 equiv.), tetrabutylammonium bromide (0.6 g, 1.8 mmol, 0.04 equiv.), toluene (60 mL) and water (3 mL). The reaction was carried out for 3 hours at 60 °C. The mixture was cooled to room temperature, 15 mL of water was added and extracted with toluene (4 x 25 mL), dried over sodium sulfate. The drying agent was filtered off and the solvent was distilled off under reduced pressure, to give the expected product as a yellow oil in 68% yield (7.3 g, 31.0 mmol,).

**Table 1. The obtained aldehydes**

| Structure/Number | Molecular formula | Molar mass [g/mol] | ¹H NMR δ (ppm) C***H***O (solvent) |
|---|---|---|---|
| 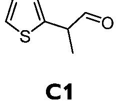 | C₇H₈OS | 140.21 | 9.64 (d, *J* = 1.7 Hz) (CHCl₃) |
| 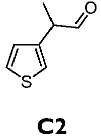 | C₇H₈OS | 140.21 | 9.55 (d, *J* = 1.8 Hz) (CHCl₃) |
| 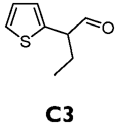 | C₈H₁₀OS | 154.23 | 9,64 (d, *J* = 3.5 Hz), 9.61 (d, J = 2.4 Hz, 3H) (CHCl₃) |
| 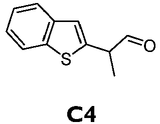 | C₁₁H₁₀OS | 190.27 | 9.73 (d, *J* = 1.6 Hz) (CHCl₃) |
| 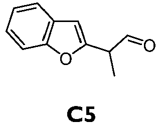 | C₁₁H₁₀O₂ | 174.20 | 9.76 (d, *J* = 1.4 Hz) (CD₂Cl₂) |
| 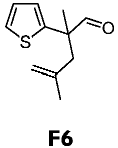 | C₁₁H₁₄OS | 194.30 | 9.52 (s) (CDCl₃) |
| 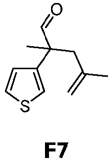 | C₁₁H₁₄OS | 194.30 | 9.52 (s) (CDCl₃) |
| 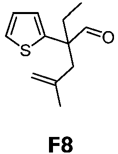 | C₁₂H₁₆OS | 208.32 | 9.50 (s) (CDCl₃) |
| 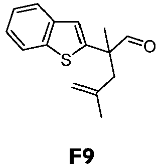 | C₁₅H₁₆OS | 244.36 | 9.61 (s) (CDCl₃) |
| 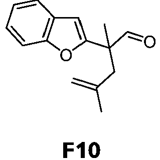 | C₁₅H₁₆O₂ | 228.29 | 9.67 (s) (CD₂Cl₂) |

### Reaction R4

In step **R4** (Scheme 9), the synthesis of an enol ether of the general formula **EX** is carried out. For this purpose, **DX** ketone and a suitable Wittig reagent, preferably chloride, a strong base, preferably potassium tert-butoxide, are used. The transformation is carried out in an anhydrous organic solvent, preferably tetrahydrofuran. The reaction mixture is carried out in the temperature range -78 °C - RT for 16 hours. Then n-heptane is added to precipitate phosphine oxide. After filtering off solids and distilling off the solvent, the product is separated by column chromatography.

### Alternative embodiment of performing reaction R4

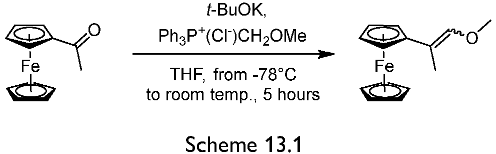

Wittig's reagent (13.2 g, 38.5 mmol, 1.35 equiv.), potassium tert-butoxide (20.4 g, 38.5 mmol, 1.35 equiv.) and anhydrous tetrahydrofuran (57 mL) were placed in a reaction vessel equipped with a stirring element under an argon atmosphere. The reaction was carried out for one hour at -78 °C. The mixture was then warmed to room temperature and allowed to stir vigorously for 30 minutes. The mixture was cooled again to -78 °C, after which a solution of acetylferrocene (6.50 g, 28.5 mmol, 1 equiv.) in anhydrous tetrahydrofuran (10 mL) was added dropwise. After 30 minutes, the mixture was warmed to room temperature and the reaction was carried out for 16 hours at this temperature. The solvent was then evaporated under reduced pressure, to the residue n-heptane (250 mL) was added, the phosphine oxide was precipitated and filtered off. The solvent was distilled off under reduced pressure, the crude product was purified by column chromatography (Al₂O₃×5%H₂O) collecting fractions using eluent from 0 to 20% ethyl acetate in n-hexane. The expected product was obtained as a red-orange oil with a yield of 96% (6.98 g, 27.2 mmol).

**¹H NMR (400 MHz, CDCl₃) 6 ppm:** 6.22 - 6.20 (m, 0.53×1H), 5.96 - 5.94 (m, 0.47×1H), 4.60 - 4.56 (m, 0.47×2H), 4.25 - 4.22 (m, 0.53×2H), 4.19 - 4.16 (m, 0.47×2H), 4.16 - 4.14 (m, 0.53×2H), 4.12 (s, 0.53×5H), 4.08 (s, 0.47×5H), 3.65 (s, 0.47×3H), 3.64 (s, 0.53×3H), 1.93 (d, *J* = 1.4 Hz, 0.53×3H), 1.84 (d, *J* = 1.4 Hz, 0.47×3H).

**¹³C NMR (101 MHz, CDCl₃) 6 ppm:** 142.8, 141.5, 11.0, 109.3, 87.8, 83.6, 69.1, 68.9, 67.9, 67.7, 67.6, 64.2, 59.9, 59.8, 17.5, 12.8.

### Reaction R5

In step **R5** (Scheme 9), the synthesis of an aldehyde of the general formula **CX** is carried out. For this purpose, an enol ether **EX** and a suitable inorganic acid, preferably hydrobromic acid, are used. The transformation is carried out in a mixture of organic solvent - water, preferably acetone - water (4 - 1 v/v). The acid is added dropwise at a temperature below -50 °C, using a dry ice-acetone cooling bath, and then the mixture is heated to 45 °C and stirred vigorously for another 2 hours until full conversion of the substrate is achieved. Next, a weak base, preferably sodium bicarbonate, is added until a pH of 8 is obtained, the product is extracted with an organic solvent, preferably methylene chloride, then dried over a drying agent, preferably sodium or magnesium sulfate. After filtering off solids and evaporating the solvent under reduced pressure, the product is obtained.

### Embodiment of performing reaction R5

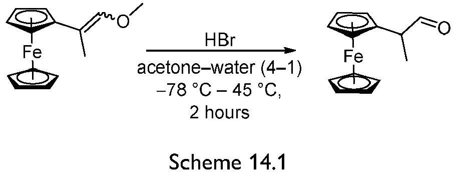

Into a reaction vessel equipped with a stirring element, ether (2.60 g, 10.2 mmol, 1 equiv.), acetone (12 mL) and water (3 mL) were placed. The mixture was cooled below -50 °C and HBr (48% aqueous solution, 2.30 mL, 2 equiv.) was added dropwise under an argon atmosphere. The reaction was then carried out for 2 hours at 45 °C. An aqueous solution of NaHCO₃ was added to the reaction mixture until a pH of 8. The product was extracted with methylene chloride, dried over magnesium sulfate, which was then filtered off. The solvent was distilled off under reduced pressure to give the expected product as a maroon oil in 93% yield (2.30 g, 9.50 mmol).

**¹H NMR (400 MHz, CDCl₃) δ ppm:** 9.72 (d, *J* = 2.2 Hz, 1H), 4.21 - 4.19 (m, 2H), 4.16 (s, 4H), 4.12 - 4.09 (m, 2H), 3.26 (qd, *J* = 7.0, 2.3 Hz, 1H), 1.36 (d, *J* = 7.0 Hz, 3H).

**¹³C NMR (101 MHz, CDCl₃) 6 ppm:** 201.0, 85.0, 68.8, 68.3, 68.3, 67.1, 67.0, 45.7, 14.7.

### Example II

### Synthesis of CAAC ligands

The following Scheme 15 illustrates the synthesis of CAAC ligands allowing the preparation of ruthenium catalysts for olefin metathesis (general formula **Ru1a** to **Ru21a,** Scheme 18), which are the subject of the present invention.

The **R6** and **R8** reactions shown in Scheme 15 were carried out using commercially available compounds based on procedures described in the literature with modifications developed by the authors. Unless otherwise written, the reactions described used commercially available solvents and paid no attention to the presence of oxygen and/or moisture.

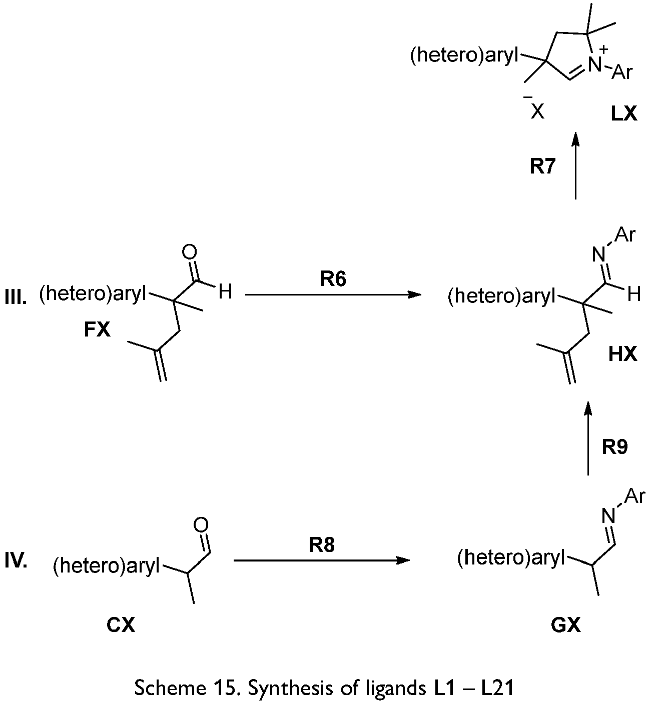

In step **R6** (Scheme 15), the synthesis of imine **HX** is carried out, for this purpose a suitable aniline and an aldehyde of the general formula **FX** are used, in the presence of an acid, preferably p-toluenesulfonic acid (PTSA). The reaction is carried out preferably in toluene or other organic solvent. The reaction mixture is carried out at the boiling point of the solvent. The product is separated by filtration through neutral Al₂O₃ and distillation of the solvent. The product is used in the next step without additional purification.

In step **R7** (Scheme 15), the synthesis of a CAAC ligand of the general formula **LX** is carried out, for this purpose imine of the general formula **HX** from step **R6** or **R9** is used in the presence of an acid, preferably 4 normal hydrochloric acid in dioxane. The reactions are carried out under an argon atmosphere in anhydrous toluene at 85 °C. Chloride ions are then exchanged, preferably to tetrafluoroborate ions, and the crude product is precipitated from a mixture of organic solvents, preferably methanol:diethyl ether.

In step **R8** (Scheme 15), the synthesis of imine **GX** is carried out. For this purpose, a suitable aniline and an aldehyde of the general formula **CX** are used, in the presence of Lewis acid, preferably titanium(IV) isopropanolate. The reaction is carried out in an anhydrous organic solvent, preferably in methanol or another organic solvent at a temperature in the range of 25 - 45 °C. The product is separated by precipitation and filtration through neutral Celite and distillation of the solvent. The product is used in the next step without additional purification.

In step **R9** (Scheme 15), the synthesis of imine **HX** is carried out, and for this purpose imine **GX,** an alkyl halide, preferably a chloride, a base, preferably n-BuLi, and an organic solvent, preferably tetrahydrofuran (THF) or another organic solvent are used. The reaction is carried out at -78 °C - RT for 10 minutes, cooled to -20 °C, the alkyl halide is added, then warmed to room temperature and the reaction is carried out for 16 hours. The product is separated by filtration through neutral Celite and distillation of the solvent. The product is used in the next step without additional purification.

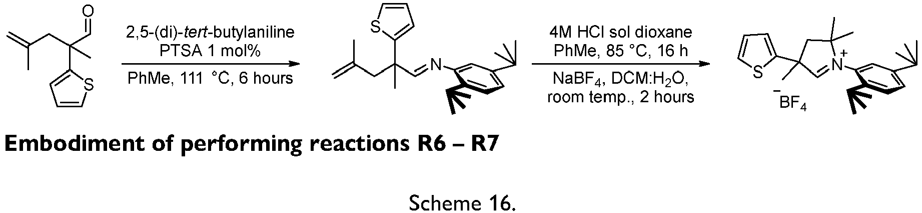

In a round-bottom flask equipped with a stirring element, 2,4-dimethyl-2-thiophenylpent-4-enal (2.11 g, 9.00 mmol, 1.00 equiv.), aniline (1.87 g, 9.00 mmol, 1.00 equiv.) and PTSA (17 mg, 0.09 mmol, 1 mol%) dissolved in PhMe (C=0.30 M) were placed. The reaction was carried out at boiling temperature of toluene until the full conversion of the substrates (collecting water in a Dean-Stark apparatus). The solvent was evaporated under reduced pressure, the crude reaction mixture was dissolved in *n*-hexane, filtered through neutral alumina (Al₂O₃, neutral, Brockman Grade I), washed with a mixture of *n*-hexane:ethyl acetate (98:2, v/v), and dried under reduced pressure to give imine in 44% yield (1.50 g, 3.94 mmol), which was used in the next step without further purification.

The imine from the previous step, 4 M HCl (solution in dioxane, 2.53 g, 2.46 mL, 9.83 mmol, 2.5 equiv.) and anhydrous PhMe (C=0.50 M) were placed in a round-bottom flask under an argon atmosphere. The reaction was carried out for 16 hours at 85 °C. The solvent was evaporated under reduced pressure. The crude product was dissolved in a methylene chloride water mixture, NaBF₄ (0.86 g, 7.86 mmol, 2.0 equiv.) was added and ion exchange was carried out for 2 h. The organic fraction was collected, washed with water and dried over sodium sulfate. The product was precipitated from the MeOH:Et₂O mixture, to give final product as a colorless crystals in 39% yield (0.72 g, 1.53 mmol).

**¹H NMR (400 MHz, CDCl₃):** 9.62 - 9.55 (m, 1H), 8.94 (s, 1H), 7.68 - 7.62 (m, 1H), 7.56 - 7.47 (m, 3H), 7.39 - 7.31 (m, 5H), 7.29 - 7.27 (m, 1H), 7.16 - 7.07 (m, 2H), 6.71 - 6.61 (m, 1H), 3.08 (dd, *J* = 23.3, 13.9 Hz, 2H), 2.72 (dd, *J* = 54.9, 14.0 Hz, 2H), 2.16 (s, 3H), 1.95 (s, 3H), 1.85 (s, 3H), 1.57 - 1.52 (m, 5H), 1.45 (s, 4H), 1.34 (s, 5H), 1.27 (s, 4H), 1.17 (s, 5H).

**¹³C NMR (101 MHz, CDCl₃): δ** 184.9, 184.5, 151.0, 150.5, 142.2, 142.0, 142.0, 141.2, 132.1, 131.0, 130.7, 130.6, 129.2, 128.9, 128.6, 127.0, 126.6, 126.3, 125.5, 125.5, 123.1, 82.5, 82.5, 81.9, 81.9, 52.6, 52.4, 50.5, 49.2, 37.7, 37.1, 34.7, 34.5, 33.8, 33.7, 31.0, 30.9, 30.7, 29.4, 29.0, 28.9, 27.2, 26.6.

### Embodiment of carrying out reactions R8 - R9 - R7

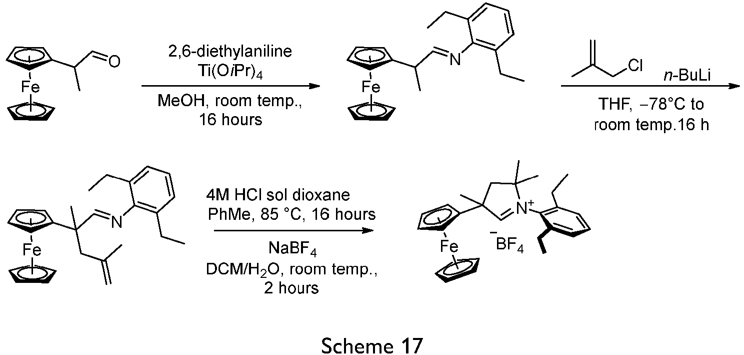

In a round-bottomed flask under an argon atmosphere, 2-ferrocenylpropanal (5.45 g, 22.5 mmol, 1.00 equiv.) and anhydrous methanol (150 mL) were placed, followed by the addition of titanium(IV) isopropanolate (13.3 mL, 45 mmol, 2.00 equiv.). To the reaction mixture thus prepared, 2,6-diethylaniline (4.27 mL, 25.8 mmol, 1.15 equiv.) was added dropwise. The reaction was carried out for 16 h at 45 °C. The solvent was evaporated under reduced pressure. The crude product was dissolved in n-pentane and then filtered through neutral Celite. The solvent was evaporated to give the crude product as a maroon oily liquid in 73% yield (6.15 g, 16.5 mmol).

Imine (6.36 g, 17.1 mmol, 1.00 equiv.) and anhydrous tetrahydrofuran (8.55 mL) were placed in a round-bottom flask-previously heated under reduced pressure-under an argon atmosphere. The mixture was then cooled to -78 °C in a dry ice - acetone cooling bath. A solution of n-BuLi (2.35 M solution in n-hexane, 8.72 mL, 20.5 mmol, 1.20 equiv.) was then added dropwise with intense stirring, followed by warming to room temperature. After one hour, the reaction mixture was cooled again to -78 °C in a dry ice-acetone cooling bath and 3-chloro-2-methylpropene (2.50 mL, 25.6 mmol, 1.50 eqn.) was added dropwise. The reaction was carried out for 16 h at room temperature. The solvent was evaporated under reduced pressure. The crude product was purified by distillation to give a maroon oily liquid in 100% yield (7.30 g, 17.1 mmol).

The imine from the previous step (7.30 g, 17.1 mmol, 1 equiv.) dissolved in anhydrous PhMe (C=500 mM) was placed in a round-bottom flask under an argon atmosphere. The mixture was cooled to -78 °C in a dry ice-acetone cooling bath. HCl solution (4 M solution in dioxane, 12.8 mL, 51.2 mmol, 3 equiv.) was then added dropwise and the reaction was carried out for 16 h at 85 °C with vigorous stirring. The solvent was evaporated under reduced pressure. The crude product was dissolved in methylene chloride (about 10 mL), a saturated aqueous solution of NaBF₄ (3.75 g, 34.1 mmol, 2 equiv.) was added and the ion exchange was carried out for 2 h with vigorous stirring. The mixture was extracted three times with methylene chloride, dried with anhydrous magnesium sulfate and filtered through neutral Celite, after which the solvent was evaporated under reduced pressure. The product was precipitated from the DCM:Et₂O mixture to give red crystals in 47% yield (4.14 g, 8.05 mmol).

**Table 2. Summary of CAAC ligands obtained according to the general procedure from Example III.**

| Structure/Number | Selected analytical data |
|---|---|
| 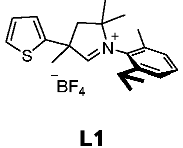 | **¹H NMR (400 MHz, CDCl₃): 6 ppm** 9.53 - 9.44 (m, 0.53×1H), 9.39 - 9.27 (m, 0.4×1H), 9.25 - 9.17 (m, 0.07×1H) |
| | **¹³C NMR (101 MHz, CDCl₃): δ ppm:** 186.5, 145.1, 144.8, 134.2, 133.5, 131.7, 131.7, 130.4, 130.3, 130.2, 129.3, 129.1, 129.1, 127.3, 127.0, 126.9, 126.5, 126.4, 126.0, 125.5, 125.1, 84.3, 83.9, 52.8, 52.8, 50.4, 30.1, 29.5, 29.1, 29.0, 29.0, 28.2, 27.9, 27.7, 27.2, 26.1, 26.0, 22.2, 21.9, 19.7, 19.7, 19.2, 18.9, 15.4 |
| 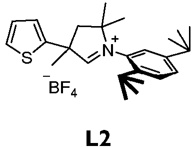 | **¹H NMR (400 MHz, CDCl₃): δ ppm:** 9.62 - 9.55 (m, 1H), 8.94 (s, 1H), 7.68 - 7.62 (m, 1H), 7.56 - 7.47 (m, 3H), 7.39 - 7.31 (m, 5H), 7.29 - 7.27 (m, 1H), 7.16 - 7.07 (m, 2H), 6.71 - 6.61 (m, 1H), 3.08 (dd, J = 23.3, 13.9 Hz, 2H), 2.72 (dd, J = 54.9, 14.0 Hz, 2H), 2.16 (s, 3H), 1.95 (s, 3H), 1.85 (s, 3H), 1.57 - 1.52 (m, 5H), 1.45 (s, 4H), 1.34 (s, 5H), 1.27 (s, 4H), 1.17 (s, 5H); |
| | **¹³C NMR (101 MHz, CDCl₃): 6 ppm:** δ 184.9, 184.5, 151.0, 150.5, 142.2, 142.0, 142.0, 141.2, 132.1, 131.0, 130.7, 130.6, 129.2, 128.9, 128.6, 127.0, 126.6, 126.3, 125.5, 125.5, 123.1, 82.5, 82.5, 81.9, 81.9, 52.6, 52.4, 50.5, 49.2, 37.7, 37.1, 34.7, 34.5, 33.8, 33.7, 31.0, 30.9, 30.7, 29.4, 29.0, 28.9, 27.2, 26.6 |
| 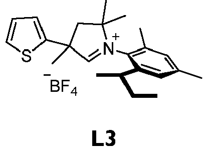 | **¹H NMR (400 MHz, CDCl₃):** δ 9.22 (s, 0.20×1H), 9.13 (s, 0.28×1H), 9.11 (s, 0.22×1H), 9.05 (s, 0.30×1H) |
| | **¹³C NMR (101 MHz, CDCl₃):** δ 186.5, 186.3, 186.1, 144.6, 144.5, 143.7, 143.6, 142.4, 142.3, 142.0, 141.9, 141.8, 141.8, 141.6, 133.7, 133.6, 133.2, 133.1, 131.0, 130.9, 130.9, 130.9, 129.2, 129.1, 129.0, 128.5, 128.1, 128.0, 127.4, 127.2, 127.1, 127.1, 126.4, 126.3, 126.3, 126.0, 126.0, 126.0, 125.9, 84.6, 84.2, 83,7, 83.6, 52.6, 52.6, 52.6, 52.5, 50.4, 50,2, 50.1, 50.0, 36.8, 36.8, 36,1, 36.0, 33.4, 33.3, 29.1, 29.0, 29.0, 28,9, 28.8, 28.3, 28.1, 27.9, 27,9, 27.5, 27.5, 27.4, 26.6, 22.6, 22.5, 21.3, 21.3, 21.3, 20.0, 20.0, 19,6, 19.5, 19.2, 19.1, 12.7, 12.5, 12.3, 11.8 |
| 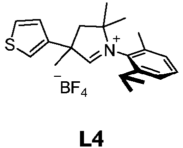 | **¹H NMR (400 MHz, CD₂Cl₂):** δ 9.29 (s, 0.5×1H), 9.24 (s, 0.5×1H), 7.56 - 7.44 (m, 3H), 7.41 - 7.37 (m, 0.5×1H), 7.35 - 7.32 (m, 0.5×1H), 7.31 - 7.23 (m, 2H), 3.16 (d, *J* = 14.0 Hz, 0.5×1H), 3.12 (d, *J* = 14.1 Hz, 0.5×1H), 2.75 - 2.62 (m, 0.5×1H), 2.69 (d, *J* = 14.1 Hz, 0.5×1H), 2.66 (d, *J* = 13.9 Hz, 0.5×1H), 2.34 (s, 0.5×3H), 2.41 - 2.26 (m, 0.5×1H), 2.07 (s, 0.5×3H), 1.95 (s, 0.5×3H), 1.89 (s, 0.5×3H), 1.62 (s, 0.5×3H), 1.57 (s, 0.5×3H), 1.45 (s, 0.5×3H), 1.42 (s, 0.5×3H), 1.40 (d, *J* = 6.7 Hz, 0.5×3H),1.23 (d, *J* = 6.7 Hz, 0.5×3H),1.15 (d, *J* = 6.7 Hz, 0.5×3H),0.95 (d, *J* = 6.8 Hz, 0.5×3H) |
| | **¹³C NMR (101 MHz,** CD₂Cl₂): **δ** 187.9, 145.2, 145.1, 140.2, 139.9, 134.3, 133.9, 132.0, 132.0, 131.0, 130.6, 130.5, 129.1, 129.0, 125.8, 125.7, 125.7, 125.7, 122.9, 122.8, 85.0, 84.6, 53.4, 53.4, 49.2, 49.0, 30.4, 29.8, 28.7, 28.6, 28.1, 27.9, 27.6, 27.1, 26.1, 26.0, 22.1, 22.1, 19.8, 19.4 |
| 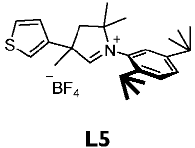 | **¹H NMR (400 MHz, CDCl₃):** δ 9.58 (s, 0.57×1H), 9.10 (s, 0.43×1H), 7.63 (d, *J* = 8.7 Hz, 0.57×1H), 7.55 - 7.47 (m, 2H), 7.46 - 7.40 (m, 1H), 7.38 (dd, *J* = 3.0, 1.5 Hz, 0.43×1H), 7.30 (dd, *J* = 5.1, 1.5 Hz, 0.57×1H), 7.27 (dd, *J* = 5.0, 1.6 Hz, 0.43×1H), 7.22 (d,*J* = 2.0 Hz, 0.43×1H), 6,63 (d, *J* = 2.1 Hz, 0.57×1H), 3.24 (d, *J* = 14.0 Hz, 0.57×1H), 3.07 (d, *J* = 13.8 Hz, 0.43×1H), 2.75 (d, *J* = 13.7 Hz, 0.43×1H), 2.53 (d, *J* = 14.0 Hz, 0.57×1H), 2.01 (s, 0.43×3H), 1.83 (s, 0.43×3H), 1.82 (s, 0.57×3H), 1.52 (s, 0.43×3H), 1.51 (s, 0.57×3H), 1.44 (s, 0.57×3H), 1.42 (s, 0.57×9H), 1.32 (s, 0.43×9H), 1.25 (s, 0.57×9H), 1.12 (s, 0.43×9H) |
| | **¹³C NMR (101 MHz, CDCl₃):** δ 186.1, 185.6, 150.8, 150.4, 141.9, 141.3, 139.5, 139.2, 132.0, 131.1, 130.9, 130.8, 128.8, 128.5, 128.4, 128.4, 125.8, 125.5, 125.0, 123.1, 122.5, 122.3, 82.5, 81.9, 52.9, 52.7, 48.7, 47.6, 37.6, 37.1, 34.6, 34.5, 33.7, 33.6, 30.9, 30.9, 30.7, 28.7, 28.4, 27.8, 27.0, 26.2 |
| 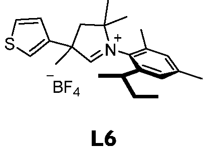 | **¹H NMR (400 MHz, CDCl₃):** δ 9.38 (s, 0.23×1H), 9.31 (s, 0.27×1H), 9.28 (s, 0.23×1H), 9.22 (s, 0.23×1H), 7.60 (m, 0.23×1H + 0.27×1H), 7.49 (m, 0.23×1H+0.27×1H), 7.45 - 7.40 (m, 1H), 7.34 - 7.26 (m, 1H), 7.04 - 6.98 (m, 1H), 6.98 - 6.89 (m, 1H), 3.23 (d, *J* = 13.9 Hz, 0.27×1H), 3.23 (d, *J* = 14.3 Hz, 0.27×1H), 3.22 (d, *J* = 14.2 Hz, 0.23×1H), 3.18 (d, *J* = 14.2 Hz, 0.23×1H), 2.67 (d, *J* = 14.2 Hz, 0.27×1H), 2.63 (d, *J* = 14.1 Hz, 0.23×1H), 2.60 (d, *J* = 14.2 Hz, 0.27×1H), 2.60 (d, *J* = 14.1 Hz, 0.23×1H), 2.39 - 1.19 (m, 18H), 1.32 (d, *J* = 6.7 Hz, 0.27×3H), 1.16 (d, *J* = 6.7 Hz, 0.27×3H), 1.11 (d, *J* = 6.7 Hz, 0.23×3H), 1.01 (t, *J* = 7.4 Hz, 0.23×3H), 0.85 (d, *J* = 6.7 Hz, 0.23×3H), 0.78 (t, *J* = 7.4 Hz, 0.27×3H), 0.70 (t, *J* = 7.4 Hz, 0.23×3H), 0.45 (t, *J* = 7.4 Hz, 0.27×3H) |
| | **¹³C NMR (101 MHz, CDCl₃):** δ 188.0, 188.0, 187.9, 187.5, 144.5, 144.3, 143.6, 143.5, 141.8, 141.7, 141.6, 141.5, 139.5, 139.5, 139.3, 139.3, 133.6, 133.5, 133.1, 132.9, 130.9, 130.9, 130.8, 130.7, 128.8, 128.7, 128.5, 128.4, 128.4, 128.3, 128.2, 126.1, 126.1, 126.0, 126.0, 125.8, 125.6, 125.6, 125.6, 122.8, 122.7, 122.6, 84.4, 84.1, 83.7, 83.6, 53.0, 52.9, 52.9, 48.7, 48.5, 48.1, 47.9, 36.8, 36.8, 36.1, 35.8, 33.3, 33.3, 28.9, 28.9, 28.7, 28.7, 28.5, 28.4, 28.4, 28.1, 27.4, 27.2, 27.1, 27.1, 27.0, 25.8, 22.5, 22.4, 21.3, 21.3, 21.3, 19.9, 19.8, 19.6, 19.4, 19.0, 18.9, 12.6, 12.5, 12.2, 11.9 |
| 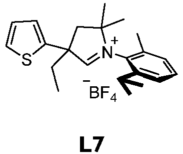 | **¹H NMR (400 MHz, CDCl₃):** 9.44 (s, 0.5×1H), 9.33 (s, 0.5×1H), 7.48 (dd, *J* = 3.7, 1.1 Hz, 0.5×1H), 7.46 - 7.31 (m, 3H), 7.25 - 7.20 (m, 1H), 7.19 - 7.15 (m, 0.5×1H), 7.14 - 7.10 (m, 1H), 3.05 (d, *J* = 14.3 Hz, 1H), 2.79 - 2.63 (m, 1h+0.5×1H), 2.54 - 2.42 (m, 0.5×1H), 2.42 - 2.13 (m, 0.5×3H+2H), 2.00 (s, 0.5×3H), 1.60 (d, *J* = 18.8 Hz, 3H), 1.47 (d, *J* = 8.3 Hz, 3H), 1.37 (d, *J* = 6.7 Hz, 0.5×3H), 1.16 (dd, *J* = 8.5, 6.7 Hz, 3H), 1.02 (td, *J* = 7.4, 2.7 Hz, 3H), 0.87 (d, *J* = 6.7 Hz, 0.5×3H); |
| | **¹³C NMR (101 MHz, CDCl₃):** δ 186.4, 186.0, 144.9, 144.9, 140.4, 140.2, 134.1, 133.6, 131.7, 131.6, 130.5, 130.4, 130.2, 130.1, 129.4, 129.3, 127.8, 127.7, 126.3, 125.8, 125.2, 125.1, 84.0, 83.5, 57.6, 57.5, 46.9, 46.3, 35.3, 34.4, 30.2, 29.1, 28.7, 27.7, 27.5, 26.8, 26.0, 25.9, 22.0, 22.0, 19.8, 18.9, 8.7 |
| 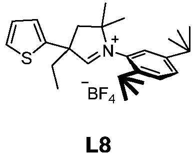 | **¹H NMR (400 MHz, CDCl₃):** 9.71 (s, 0H), 9.59 (s, 0H), 9.35 (s, 0H), 9.01 (s, 0H), 7.66 - 7.47 (m, 1H), 7.43 - 7.26 (m, 1H), 7.16 - 7.10 (m, 0H), 6.63 - 6.55 (m, 0H), 3.08 (d, *J* = 14.0 Hz, 0H), 2.96 - 2.79 (m, 0H), 2.69 - 2.52 (m, 0H), 2.50 - 2.25 (m, 0H), 2.07 - 1.94 (m, 0H), 1.81 (s, 1H), 1.55 (d, *J* = 4.1 Hz, 1H), 1.49 (s, 1H), 1.44 (s, 2H), 1.33 (s, 1H), 1.25 (s, 2H), 1.11 (s, 2H), 0.92 (td, *J* = 7.3, 2.4 Hz, 1H); |
| | **¹³C NMR (101 MHz, CDCl₃):** δ 184.1, 150.8, 150.4, 141.9, 141.4, 139.9, 139.1, 132.0, 131.1, 130.7, 129.3, 129.1, 128.8, 128.6, 127.7, 127.5, 126.3, 125.7, 125.5, 123.3, 82.2, 81.7, 57.2, 57.0, 48.2, 45.1, 37.6, 37.0, 34.7, 34.5, 34.2, 33.7, 33.7, 33.5, 33.1, 33.0, 31.2, 31.0, 30.9, 29.3, 27.2, 26.5, 8.3, 8.2 |
| 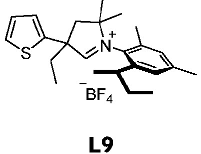 | **¹H NMR (400 MHz, CDCl₃):** 9.51 - 9.46 (m, 0.25×1H), 9.41 - 9.36 (m, 0.28×1H), 9.33 - 9.29 (m, 0.21×1H), 9.27 - 9.22 (m, 0.26×1H), |
| | **¹³C NMR (101 MHz, CDCl₃):** 186.7, 186.4, 186.2, 185.8, 144.9, 144.4, 143.8, 143.7, 141.9, 141.8, 141.8, 141.6, 140.4, 140.3, 140.1, 139.8, 133.7, 133.6, 133.4, 133.3, 131.0, 131.0, 130.9, 130.8, 129.7, 129.5, 129.4, 128.5, 128.4, 128.2, 128.1, 128.0, 128.0, 127.9, 126.2, 126.2, 126.2, 126.1, 125.7, 125.7, 83.9, 83.6, 83.1, 83.0, 57.5, 57.4, 57.3, 57.3, 47.1, 46.9, 46.3, 46.1, 37.1, 36.9, 35.8, 35.8, 35.4, 35.3, 34.3, 33.3, 33.3, 29.8, 29.0, 29.0, 28.9, 28.8, 27.8, 27.6, 27.4, 27.3, 27.3, 26.2, 22.6, 22.2, 21.4, 21.3, 19.8, 19.8, 19.7, 19.7, 18.9, 18.8, 12.6, 12.5, 12.2, 11.9, 8.8, 8.7, 8.7, 8.5 |
| 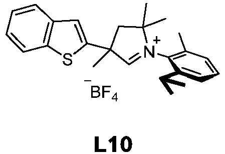 | **¹H NMR (400 MHz, CD₂Cl₂):** δ 9.23 (s, 0.5×1H), 9.19 (s, 0.5×1H), 7.93 - 7.80 (m, 2H), 7.62 (m, 0.5×1H), 7.53 - 7.47 (m, 0.5×1H+1H), 7.46 - 7.38 (m, 2H+0.5×1H), 7.38 - 7.34 (m, 0.5×1H), 7.31 - 7.28 (m, 0.5×1H), 7.27 - 7.24 (m, 0.5×1H), 3.18 (d, *J* = 14.3 Hz, 0.5×1H), 3.13 (d, *J* = 14.2 Hz, 0.5×1H), 2.83 (d, *J* = 14.2 Hz, 0.5×1H), 2.82 (d, *J* = 14.2 Hz, 0.5×1H), 2.75 - 2.57 (m, 1H), 2.35 (s, 0.5×3H), 2.19 (s, 0.5×3H), 2.13 (s, 0.5×3H), 2.07 (s, 0.5×3H), 1.66 (s, 0.5×3H), 1.61 (s, 0.5×3H), 1.56 (s, 0.5×3H), 1.55 (s, 0.5×3H), 1.41 (d, *J* = 6.7 Hz, 0.5×3H),1.29 (d, *J* = 6.7 Hz, 0.5×3H),1.17 (d, *J* = 6.8 Hz, 0.5×3H),1.00 (d, *J* = 6.7 Hz, 0.5×3H) |
| | **¹³C NMR (101 MHz, CD₂Cl₂): δ** 186.5, 186.2, 145.1, 144.9, 143.3, 142.8, 140.3, 140.1, 139.4, 139.2, 134.1, 133.8, 132.1, 132.0, 130.8, 130.7, 130.5, 130.4, 125.9, 125.8, 125.7, 125.6, 125.6, 125.6, 124.8, 124.7, 123.7, 123.3, 122.7, 122.6, 85.3, 84.8, 50.4, 50.2, 30.2, 29.8, 28.6, 28.6, 28.4, 27.7, 27.7, 27.6, 26.2, 26.0, 22.1, 21.9, 19.7, 19.6 |
| 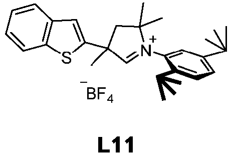 | **¹H NMR (400 MHz, CD₂Cl₂):** δ 9.48 (s, 0.6×1H), 9.03 (s, 0.4×1zH), 7.92 - 7.80 (m, 2H), 7.72 (d, *J* = 8.7 Hz, 0.6×1H), 7.66 - 7.55 (m, 1H+0.4×1H), 7.56 (d, *J* = 0.7 Hz, 0.6×1H), 7.53 (d, *J* = 0.7 Hz, 0.4×1H), 7.46 - 7.35 (m, 2H), 7.22 (d, *J* = 2.0 Hz, 0.4×1H), 6.78 (d, *J* = 2.1 Hz, 0.6×1H), 3.22 (d, *J* = 14.2 Hz, 0.6×1H), 3.17 (d, *J* = 13.9 Hz, 0.4×1H), 2.87 (d, *J* = 13.8 Hz, 0.4×1H), 2.69 (d, *J* = 14.2 Hz, 0.6×1H), 2.17 (s, 0.4×3H), 1.99 (s, 0.6×3H), 1.87 (s, 0.4×3H), 1.60 (s, 3H), 1.56 (s, 0.6×3H), 1.46 (s, 0.6×9H), 1.35 (s, 0.4×9H), 1.28 (s, 0.6×9H), 1.20 (s, 0.4×9H) |
| | **¹³C NMR (101 MHz, CD₂Cl₂): δ** 184.4, 184.3, 151.0, 151.0, 143.1, 142.9, 142.1, 141.8, 140.3, 140.1, 139.6, 139.0, 132.4, 131.7, 131.0, 130.9, 129.3, 129.1, 125.7, 125.7, 125.5, 125.5, 125.1, 124.7, 124.7, 124.6, 123.8, 123.4, 123.2, 122.6, 83.6, 82.8, 53.2, 53.2, 50.2, 48.8, 37.8, 37.4, 34.8, 34.7, 33.8, 30.9, 30.9, 30.8, 29.7, 28.7, 28.5, 27.3, 26.5 |
| 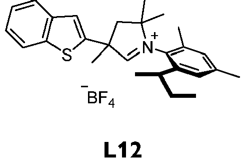 | **¹H NMR (400 MHz, CD₂Cl₂):** δ 9.19 (s, 0.22×1H), 9.13 (s, 0.25×1H), 9.09 (s, 0.53×1H) |
| | **¹³C NMR (101 MHz, CD₂Cl₂)**: δ 186.5, 186.3, 186.2, 186.2, 144.7, 144.4, 143.8, 143.7, 143.2, 143.1, 142.9, 142.8, 142.6, 142.5, 142.4, 142.3, 140.3, 140.2, 140.1, 139.3, 139.2, 139.2, 133.7, 133.6, 133.5, 133.3, 131.2, 131.1, 131.1, 131.0, 128.9, 128.5, 128.4, 126.6, 126.6, 126.6, 126.5, 125.8, 125.8, 125.8, 125.8, 125.6, 125.6, 125.5, 125.5, 124.8, 124.8, 124.7, 124.6, 123.8, 123.7, 123.7, 123.7, 122.6, 122.6, 122.6, 85.3, 85.0, 84.6, 84.5, 53.3, 53.2, 50.3, 50.1, 50.1, 50.1, 37.0, 37.0, 36.4, 36.4, 33.5, 33.5, 29.2, 29.0, 28.9, 28.7, 28.6, 28.5, 28.4, 28.3, 27.9, 27.9, 27.5, 27.5, 27.4, 27.1, 22.7, 22.6, 21.3, 21.3, 21.3, 20.2, 19.9, 19.7, 19.5, 19.5, 19.4, 12.7, 12.5, 12.2, 12.0 |
| 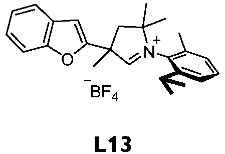 | **¹H NMR (400 MHz, CD₂Cl₂)**: δ 8.93 (s, 1H), 7.67 (dd, *J* = 0.8, 1.4 Hz, 0.45×1H), 7.66 (dd, *J* = 0.7, 1.4 Hz, 0.55×1H), 7.55 - 7.46 (m, 2H), 7.43 - 7.35 (m, 3H), 7.34 - 7.28 (m, 2H), 7.03 (d, *J* = 0.9 Hz, 0.45×1H), 6.97 (d, *J* = 0.9 Hz, 0.55×1H), 3.16 (d, *J* = 14.1 Hz, 0.45×1H), 3.10 (d, *J* = 14.0 Hz, 0.55×1H), 2.99 (hept, *J* = 6.7 Hz, 0.55×1H), 2.79 - 2.66 (m, 0.45×1H), 2.69 (d, *J* = 14.1 Hz, 0.45×1H), 2.68 (d, *J* = 14.0 Hz, 0.55×1H), 2.36 (s, 0.55×H), 2.33 (s, 0.45×H), 2.11 (s, 0.55×3H), 2.08 (s, 0.45×H), 1.71 (s, 0.55×3H), 1.69 (s, 0.45×3H), 1.67 (s, 0.55×3H), 1.67 (s, 0.45×3H), 1.41 (d, *J* = 6.9 Hz, 0.55×3H), 1.41 (d, *J* = 6.7 Hz, 0.45×3H), 1.16 (d, *J* = 6.7 Hz, 0.45×3H), 0.96 (d, *J* = 6.7 Hz, 0,55×3H) ppm |
| | **¹³C NMR (101 MHz, CD₂Cl₂):** δ 186.2, 185.9, 155.8, 155.8, 154.2, 153.6, 145.3, 145.2, 134.2, 134.0, 132.3, 132.2, 131.2, 131.0, 130.7, 128.3, 128.3, 126.2, 126.1, 126.0, 125.7, 124.3, 124.2, 122.5, 122.4, 111.6, 111.4, 106.3, 106.1, 85.8, 85.3, 52.2, 47.7, 47.4, 30.3, 29.8, 29.7, 28.5, 28.4, 27.6, 26.3, 26.2, 23.9, 23.0, 22.3, 22.2, 19.9, 19.6 |
| 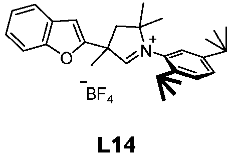 | **¹H NMR (400 MHz, CD₂Cl₂)** : δ 9.16 (s, 0.47×1H), 8.84 (s, 0.53×1H), 7.75 - 7.55 (m, 3H), 7.52 (dq, *J* = 18.5, 0.9 Hz, 0.47×1H), 7.50 (dq, *J* = 18.4, 0.9 Hz, 0.53×1H), 7.38 (m, 1H), 7.30 (m, 1H), 7.22 (d, *J* = 2.1 Hz, 0,53×1H), 7.03 (d, *J* = 0.9 Hz, 0.47×1H), 6.97 (d, *J* = 0.9 Hz, 0.53×1H), 6.89 (d, *J* = 2.1 Hz, 0.47×1H), 3.26 (d, *J* = 13.9 Hz, 0.47×1H), 3.22 (d, *J* = 13.7 Hz, 0.53×1H), 2.69 (d, *J* = 13.9 Hz, 0.53×1H), 2.60 (d, *J* = 14.1 Hz, 0.47×3H), 2.15 (s, 0.53×3H), 2.00 (s, 0.47×3H), 1.86 (s, 0.53×3H), 1.76 (s, 0.47×3H), 1.64 (s, 0.53×3H), 1.62 (s, 0.47×3H), 1.46 (s, 0.47×9H), 1.35 (s, 0.53×9H), 1.31 (s, 9H) |
| | **¹³C NMR (101 MHz, CD₂Cl₂):** δ 184.3, 183.9, 155.8, 155.6, 154,0, 153.3, 151.2, 151.1, 142.2, 142.1, 132.6, 131.9, 131.2, 131.1, 129.6, 129.3, 128.4, 128.3, 126.0, 126.0, 125.5, 124.2, 124.1, 124.0, 122.4, 122.3, 111.7, 111.7, 106.6, 106.2, 84.2, 83.5, 52.0, 51.9, 47.4, 46.6, 38.0, 37.5, 35.0, 34.9, 34.0, 33.9, 31.3, 31.1, 31.0, 30.1, 27.5, 27.3, 24.1, 23.7 |
| 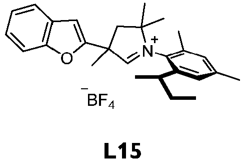 | **¹H NMR (400 MHz, CD₂Cl₂):** δ 8.87 (s, 0.4×H), 8.82 (s, 0.6×1H), 7.67 (d, *J* = 7.7 Hz, 1H), 7.48 (d, *J* = 8.2 Hz, 1H), 7.38 (td, *J* = 1.4, 7.8 Hz, 1H), 7.31 (td, *J* = 1.1, 7.5 Hz, 1H), 7.15 - 7.08 (m, 2H), 6.99 (d, *J* = 5.3 Hz, 0.6×1H), 6.99 (d, *J* = 5.3 Hz, 0.4×1H), 3.11 (d, *J* = 13.9 Hz, 0.6×1H), 3.11 (d, *J* = 14.2 Hz, 0.4×1H), 2.66 (d, *J* = 14.0 Hz, 0.6×1H), 2.66 (d, *J* = 14.0 Hz, 0.4×1H), 2.66 - 2.52 (m, 1H), 2.38 (s, 0.6×3H), 2.38 (s, 0.4×3H), 2.33 (s, 3H), 2.12 (s, 0.6×3H), 2.12 (s, 0.4×3H), 1.86 - 1.63 (m, 1H+3H+3H), 1.37 (d, *J* = 6.7 Hz, 0.6×3H), 1.46 - 1.26 (m, 1H), 1.07 (t, *J* = 7.4 Hz, 0.4×3H), 0.92 (d, *J* = 6.6 Hz, 0.4×3H), 0.45 (t, *J* = 7.4 Hz, 0.6×3H) |
| | **¹³C NMR (101 MHz, CD₂Cl₂)**: δ 186.2, 186.1, 155.8, 155.6, 154.1, 153.8, 144.9, 143.8, 142.6, 142.5, 133.6, 133.5, 131.2, 131.2, 128.2, 128.2, 126.6, 126.6, 126.0, 124.2, 124.2, 122.4, 111.4, 111.3, 106.4, 106.1, 85.1, 84.8, 51.9, 51.9, 47.5, 47.3, 36.4, 36.2, 33.8, 29.7, 29.3, 29.3, 27.7, 27.3, 23.2, 23.0, 22.7, 21.4, 21.4, 20.6, 19.8, 19.7, 12.7, 11.9 |
| 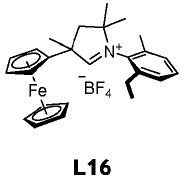 | **¹H NMR (400 MHz, CDCl₃):** δ 8.66 (bs, 0.57×H), 8.59 (bs, 0.43×1H), 7.42 - 7.33 (m, 1H), 7.29 (d, *J* = 7.6 Hz, 0.57×1H), 7.21 (d, *J* = 7.7 Hz, 1H), 7.16 (d, *J* = 7.4 Hz, 0.43×1H), 4.41 - 4.26 (m, 8H), 4.19 - 4.12 (m, 1H), 2.84 - 2.44 (m, 2H+0.57×2H), 2.37 - 2.23 (m, 2H×0.43+0.57×3H), 2.13 (s, 0.43×3H), 2.05 - 1.96 (m, 3H), 1.64 (s, 0.57×3H), 1.63 (s, 0.43×3H), 1.56 (s, 0.43×3H), 1.54 (s, 0.57×3H), 1.26 (t, *J* = 7.3 Hz, 0.43×3H), 1.04 (t, *J* = 7.3 Hz, 0.57×3H) ppm |
| | **¹³C NMR (101 MHz, CDCl₃): δ** 187.4, 187.4, 140.0, 139.9, 134.1, 133.5, 131.5, 131.5, 131.2, 131.1, 130.3, 129.8, 127.9, 127.8, 89.8, 89.5, 83.9, 83.8, 70.3, 70.3, 69.9, 69.8, 69.4, 69.3, 67.6, 67.2, 66,5, 66.2, 50.8, 50.8, 49.1, 48.9, 29.7, 29.1, 27.9, 27.5, 25.9, 25.6, 25.1, 24.6, 19.6, 19.6, 15.6, 14.8 |
| 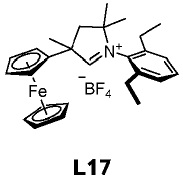 | **¹H NMR (400 MHz, CD₂Cl₂): δ** 8.48 (s, 1H), 7.52 (t, *J* = 7.8 Hz, 1H), 7.39 (d, *J* = 7.3 Hz, 1H), 7.31 (d, *J* = 7.7 Hz, 1H), 4.47 - 4.44 (m, 1H), 4.43 - 4.40 (m, 1H), 4.36 - 4.29 (m, 6H), 4.18 - 4.15 (m, 1H), 2.82 (d, *J* = 14.1 Hz, 1H), 2.69 (d, *J* = 14.1 Hz, 1H), 2.64 - 2.51 (m, 3H), 2.37 -2.26 (m, 1H), 2.03 (s, 3H), 1.65 (s, 3H), 1.55 (s, 3H), 1.30 (t, *J* = 7.5 Hz, 3H), 1.08 (t, *J* = 7.5 Hz, 3H) |
| | **¹³C NMR (101 MHz, CD₂Cl₂): δ** 187.0, 140.3, 140.0, 132.1, 128.5, 128.5, 89.8, 84.3, 70.8, 70.5, 69.7, 67.4, 66.7, 51.2, 49.7, 29.7, 28.0, 25.7, 25.6, 25.2, 15.8, 15.2 |
| 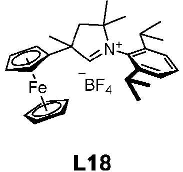 | ¹H **NMR (400 MHz, CD₂Cl₂):** δ 8.45 (s, 1H), 7.56 (t, J = 7.8 Hz, 1H), 7.41 - 7.37 (m, 1H), 7.36 - 7.31 (m, 1H), 4.47 - 4.43 (m, 1H), 4.43 - 4.40 (m, 1H), 4.39 - 4.36 (m, 1H), 4.34 (s, 5H), 4.20 - 4.14 (m, 1H), 2.86 (d, J = 14.2 Hz, 1H), 2.77 - 2.65 (m, 2H), 2.53 (hept, J = 7.4 Hz, 1H), 1.99 (s, 3H), 1.67 (s, 3H), 1.55 (s, 3H), 1.38 (d, J = 6.7 Hz, 3H), 1.32 (d, J = 6.8 Hz, 3H), 1.15 (d, J = 6.8 Hz, 3H), 0.87 (d, J = 6.8 Hz, 3H) |
| | **¹³C NMR (101 MHz, CD₂Cl₂):** δ 186.6, 145.1, 144.9, 132.6, 129.0, 126.2, 125.8, 89.9, 84.2, 70.9, 70.5, 69.8, 67.7, 66.7, 51.1, 49.0, 30.2, 30.1, 29.7, 27.8, 26.5, 26.3, 25.5, 22.6, 21.9 |
| 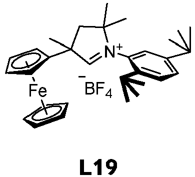 | **¹H NMR (400 MHz, CDCl₃):** δ 9.13 (s, 0.24×1H), 8.55 (s, 0.76×1H) ppm |
| 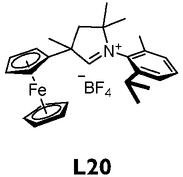 | **¹H NMR (400 MHz, CD₂Cl₂):** δ 8.57 (s, 0.56×1H), 8.41 (s, 0.44×1H), 7.51 - 7.45 (m, 1H), 7.40 - 7.36 (m, 0.44×1H), 7.35 - 7.32 (m, 0.56×1H), 7.30 - 7.26 (m, 0.56×1H), 7.26 - 7.22 (m, 0.44×1H), 4.46 - 4.42 (m, 1H), 4.41 - 4.36 (m, 1H + 0.44×1H), 4.32 (s, 0.44×5H), 4.32 (s, 0.56×5H), 4.26 - 4.24 (m, 0.56×1H), 4.22 - 4.19 (m, 0.44×1H), 4.17 - 4.15 (m, 0.56×1H), 2.86 (d, J = 14.2 Hz, 0.44×1H), 2.78 (d, J = 14.1 Hz, 0,56×1H), 2.75 - 2.65 (m, 1H + 0.44×1H), 2.59 (hept, J = 6.9 Hz, 0.56×1H), 2.33 (s, 0.56×3H), 2.16 (s, 0.44×3H), 2.01 (s, 0.56×3H), 1.99 (s, 0.44×3H), 1.67 (s, 0.56×3H), 1.65 (s, 0.44×3H), 1.59 (s, 0.44×3H), 1.55 (s, 0.56×3H), 1.39 (d, J = 6.7 Hz, 0.44×3H), 1.34 (d, J = 6.8 Hz, 0.56×3H), 1.13 (d, J = 6.8 Hz, 0.44×3H), 0.89 (d, J = 6.8 Hz, 0.56×3H) |
| | **¹³C NMR (101 MHz, CD₂Cl₂):** δ 187.4, 186.8, 145.2, 145.2, 134.2, 134.1, 132.1, 132.0, 131.0, 130.8, 130.7, 130.5, 126.0, 125.5, 90.2, 89.7, 84.6, 84.4, 70.6, 70.6, 70.5, 70.3, 69.7, 69.7, 67.6, 67.1, 67.0, 66.5, 51.2, 51.1, 49.6, 49.2, 30.1, 30.1, 30.0, 29.1, 28.4, 27.8, 26.4, 26.2, 25.8, 25.3, 22.5, 21.9, 20.0, 19.9 |
| 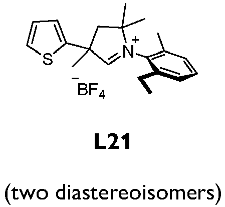 | **¹H NMR (400 MHz, CDCl₃):** δ 9.21 (s, 1H), 7.43 - 7.31 (m, 4H), 7.20 - 7.16 (m, 1H), 7.09 (dd, J = 5.2, 3.7 Hz, 1H), 3.08 (d, J = 14.2 Hz, 1H), 2.78 (d, J = 14.3 Hz, 1H), 2.63 - 2.52 (m, 2H), 2.10 (s, 3H), 2.06 (s, 3H), 1.61 (s, 3H), 1.53 (s, 3H), 1.29 (t, J = 7.5 Hz, 3H) |
| | **¹³C NMR (101 MHz, CDCl₃):** δ 186.6, 142.1, 140.2, 133.4, 131.4, 130.0, 129.2, 128.0, 127.1, 126.2, 84.2, 52.8, 50.4, 28.8, 28.0, 27.8, 24.9, 19.2, 14.8 |
| | **¹H NMR (400 MHz, CDCl₃):** δ 9.21 (s, 1H), 7.43 - 7.36 (m, 2H), 7.33 - 7.31 (m, 1H), 7.26 - 7.23 (m, 2H), 7.13 - 7.10 (m, 1H), 3.06 (d, J = 14.4 Hz, 1H), 2.78 (d, J = 14.2 Hz, 1H), 2.49 - 2.36 (m, 4H), 2.30 - 2.19 (m, 1H), 2.12 (s, 3H), 1.65 (s, 3H), 1.63 (s, 3H), 1.07 (t, J = 7.5 Hz, 3H) **¹³C NMR (101 MHz, CDCl₃):** δ 186.6, 142.4, 139.9, 134.3, 131.4, 130.4, 129.2, 128.0, 127.0, 126.4, 83.9, 52.8, 50.6, 28.8, 28.3, 27.4, 25.0, 19.6, 15.6 |

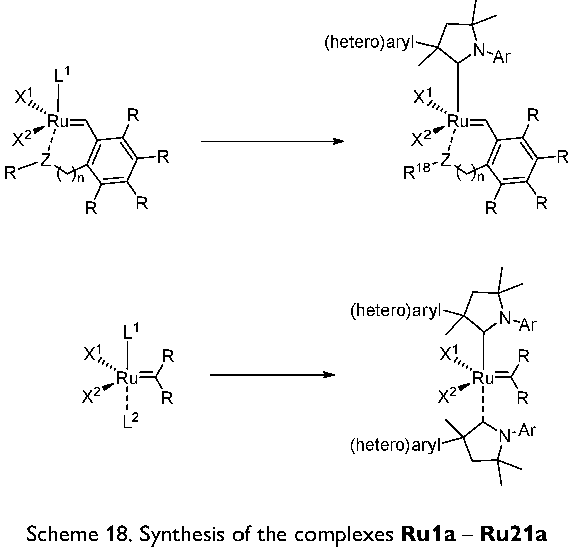

### Example III

Synthesis of the complexes **Ru1a** - **Ru20a** by using CAAC ligands

### Embodiment of the invention

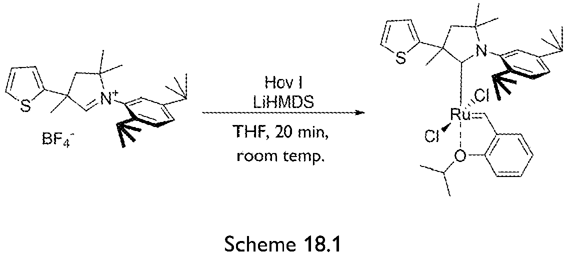

Under an argon atmosphere, the CAAC×BF₄ ligand (430 mg, 916 µmol, 2.20 equiv.), first-generation Hoveyda-Grubbs complex (250 mg, 416 µmol, 1.00 equiv.), and anhydrous THF (C_{CAAC}=0.1 M) were placed in a heated Schlenk vessel and stirred for 1 min. LiHMDS (153 mg, 916 µmol, 2.20 equiv.) was then added and stirred until full conversion was achieved (20 min). The crude mixture was filtered through neutral alumina (Al₂O₃, neutral, Brockman Grade I) with methylene chloride as eluent. The green fraction was collected and evaporated under reduced pressure. A small amount of n-pentane was then added and the mixture was placed in an ultrasonic bath. The product was filtered off and washed with cold n-pentane. The process was repeated twice and then three times using diethyl ether. After drying under vacuum, a green crystalline solid was obtained in 91% yield (267 mg, 380 µmol).

Using the method presented in Example **III,** a series of complexes from **Ru1a** to **Ru21a** were obtained, the structures of which are shown below.

All complexes in the table below were characterized by nuclear magnetic resonance spectroscopy. Table 3 summarizes the benzylidene proton shifts of each complex in the ¹H NMR spectrum in a given solvent.

**Table 3. Summary of the obtained structures of the ruthenium complexes according to Example IV general procedure and the shifts of their benzylidene proton in the ¹H NMR spectrum.**

| Structure/Symbol | Molecular formula | Molar mass [g/mol] | ¹H NMR δ (ppm) Ru=C***H*** (solvent) |
|---|---|---|---|
| 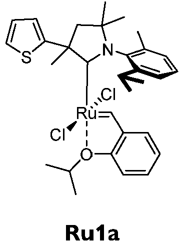 | C₃₁H₃₉Cl₂NORuS | 645.6890 | 18.03, 16.84, 16.54 |
| | | | CDCl₃ |
| 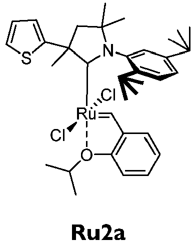 | C₃₅H₄₇Cl₂NORuS | 701.7970 | 17.89 |
| | | | CDCl₃ |
| 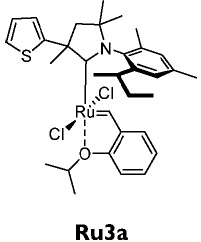 | C₃₃H₄₃Cl₂NORuS | 673.7430 | 16.38, 16.34 |
| | | | CDCl₃ |
| 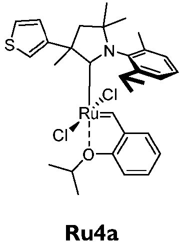 | C₃₁H₃₉Cl₂NORuS | 645.6890 | 16.31, 16.30 |
| | | | CD₂Cl₂ |
| 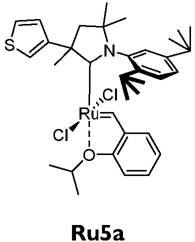 | C₃₅H₄₇Cl₂NORuS | 701.7970 | 17.98, 16.74 |
| | | | CD₂Cl₂ |
| 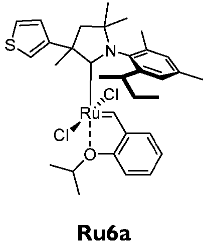 | C₃₃H₄₄Cl₂NORuS | 674.7510 | 16.39, 16.39, 16.35, 16.34 |
| | | | CDCl₃ |
| 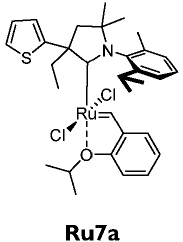 | C₃₂H₄₁Cl₂NORuS | 659.7160 | 16.43, 16.40 |
| | | | CDCl₃ |
| 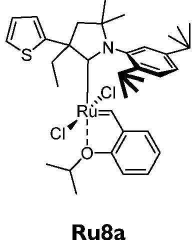 | C₃₆H₄₉Cl₂NORuS | 715.824 | 16.33, 16.32 |
| | | | CDCl₃ |
| 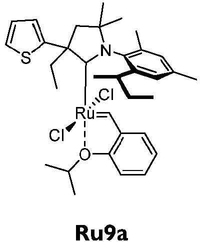 | C₃₄H₄₆Cl₂NORuS | 688.7780 | 16.47, 16.46, 16.42, 16.41 |
| | | | CDCl₃ |
| 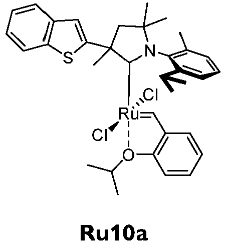 | C₃₅H₄₂Cl₂NORuS | 696.7570 | 16.39, 16.34 |
| | | | CD₂Cl₂ |
| 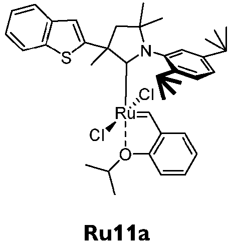 | C₃₉H₄₉Cl₂NORuS | 751.8570 | 18.16, 16.80 |
| | | | CD₂Cl₂ |
| 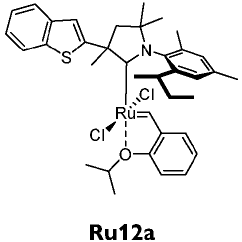 | C₃₇H₄₅Cl₂NORuS | 723.8030 | 16.45, 16.43, 16.40, 16.38 |
| | | | CD₂Cl₂ |
| 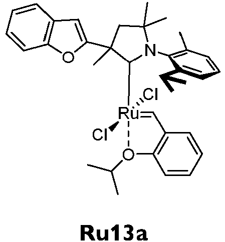 | C₃₅H₄₁Cl₂NO₂Ru | 679.6880 | 16.18, 16.16 |
| | | | CD₂Cl₂ |
| 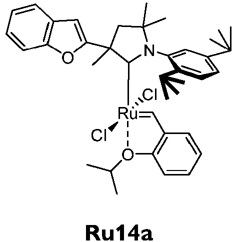 | C₃₉H₄₉Cl₂NO₂Ru | 735.7960 | 18.29, 16.52 |
| | | | CD₂Cl₂ |
| 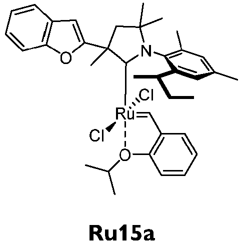 | C₃₇H₄₅Cl₂NO₂Ru | 707.7420 | 16.22, 16.18 |
| | | | CD₂Cl₂ |
| 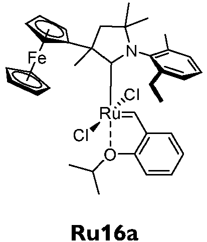 | C₃₆H₄₃Cl₂FeNORu | 733.5610 | 16.23, 16.21 |
| | | | CD₂Cl₂ |
| 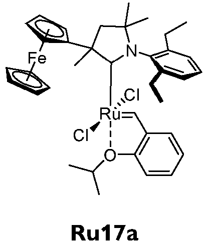 | C₃₇H₄₅Cl₂FeNORu | 747.1271 | 16.36 |
| | | | CDCl₃ |
| 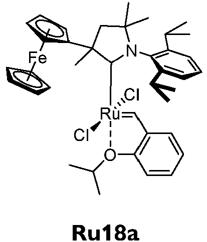 | C₃₉H₄₉Cl₂FeNORu | 775.6420 | 16.46 |
| | | | CDCl₃ |
| 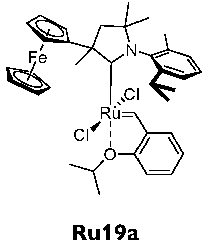 | C₃₇H₄₅Cl₂FeNORu | 747.5880 | 16.26, 16.20 |
| | | | CD₂Cl₂ |
| 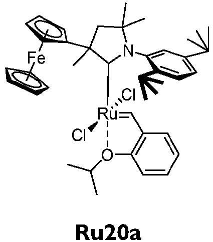 | C₄₁H₅₃Cl₂FeNORu | 803.6960 | 18.44, 16.31 |
| | | | CDCl₃ |
| 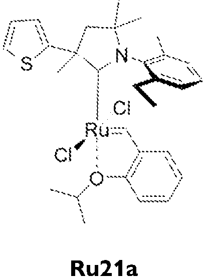 | C₃₀H₃₇Cl₂NORuS | 631.6620 | 16.29, 16.24 |
| | | | CD₂Cl₂ |

### Example V

### Study of the activity of complexes in the ethenolysis reaction of methyl oleate

Methyl oleate was degassed by stirring for a minimum of 30 minutes under reduced pressure (oil pump). During this time, a closed Schlenk vessel was prepared and weighed to obtain tare. The oil was then filtered through a syringe filter into the Schlenk vessel and the vessel was weighed together with the oil, thus obtaining the weight of the oil. The vessel with the substrate was subjected to reduced pressure. In a separate vessel, a catalyst solution (~3 mg in 2 mL of degassed toluene) was prepared. A preheated glass insert for the Amar reactor equipped with a stirring element was placed in the Amar reactor immersed in a preheated oil bath. The reactor was closed and the gas inside was evacuated using an oil pump. A portion of the catalyst (10 ppm or 3 ppm or 500 ppb) was added to the Schlenk vessel containing the oil and the mixture was immediately transferred into the reactor using a Teflon tube. The reactor was filled with ethylene to a dynamic pressure of 10 bar, then stirring was started and the reaction was run for 3 hours or 6 hours. The glovebox and inert gas atmosphere (argon) were not used during the reaction.

After that time, pressure was normalized and the autoclave was disassembled. SnatchCat metal scavenger (scavenger) solution was immediately added and the contents of the vessel were stirred for several minutes. A sample was then taken and, after dilution with toluene, subjected to GC analysis.

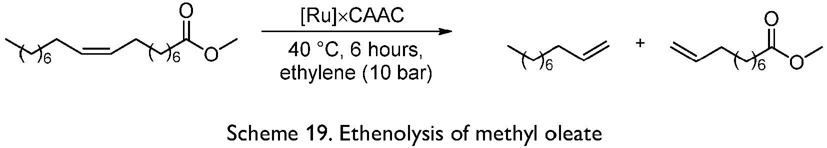

The results of the model reaction are shown in Table 2.

**Table 4. Comparison of the selectivity of the new ruthenium complexes and ruthenium complexes known from the literature in the process of ethenolysis**

| No. | Structure | Ethenolysis results | | |
|---|---|---|---|---|
| | | Loading [ppm] | TON | Selectivity |
| 1. | 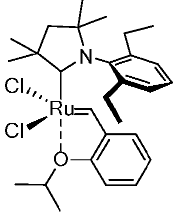 *Literature state-of-the-art* | 100 | 5,300¹ | 73% |
| | | 10 | 35,000¹ | 83% |
| 2. | 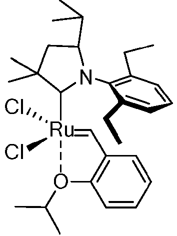 *Literature state-of-the-art* | 100 | 5,800² | 96% |
| | | 10 | 26,000² | 97% |
| 3. | 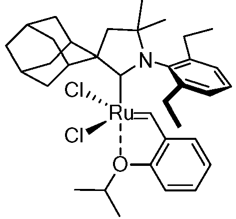 *Literature state-of-the-art* | 3 | -³ | - |
| 4. | 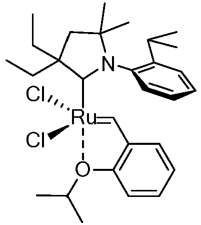 *Literature state-of-the-art* | 3 | 47,000³ | 63% |
| 5. | 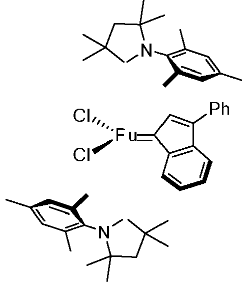 *Literature state-of-the-art* | 10 | -⁴ | - |
| 6. | 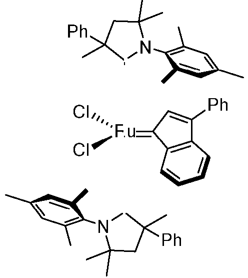 *Literature state-of-the-art* | 10 | 19,000⁴ | 92% |
| 7. | 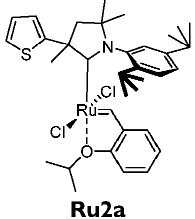 | 3 | 113,000 | 88% |
| | | 0.5 | 101,000 | 88% |
| 8. | 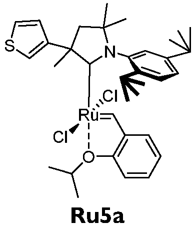 | 3 | 189,000 | 88% |
| | | 0.5 | 255,000 | 88% |
| 9. | 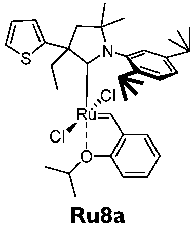 | 3 | 48,000 | 98% |
| | | 0.5 | 57,000 | 96% |
| 10. | 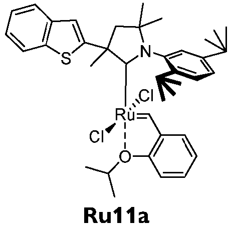 | 3 | 136,629 | 88% |
| | | 0.5 | 182,700 | 88% |
| 11. | 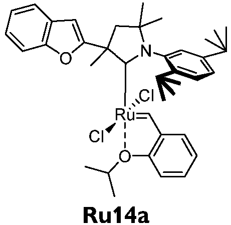 | 3 | 87,612 | 88% |
| | | 0.5 | 130,656 | 81% |
| 12. | 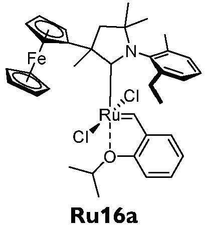 | 10 | 52,713 | 92% |
| | | 3 | 99,006 | 94% |
| 13. | 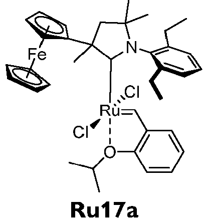 | 10 | 65,363 | 92% |
| | | 3 | 94,668 | 95% |
| 14. | 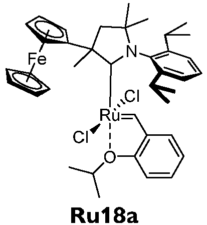 | 10 | 2,507 | 99% |
| | | 3 | 3,187 | 100% |
| 15. | 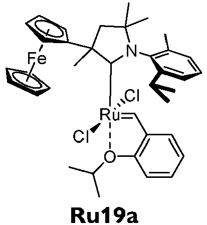 | 10 (isomer ratio =1:1) | 40,447 | 95% |
| | | 3 (isomer ratio =1:1) | 69,881 | 97% |
| 16. | 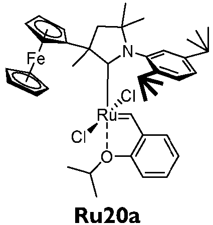 | 10 | 20,297 | 49% |
| | | 3 | 28,953 | 54% |
| | | 0.5 | 34,280 | 39% |
| 17. | 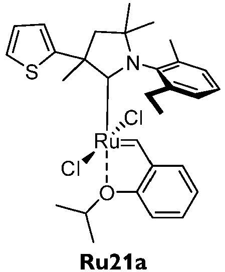 | 10 | 44,000 | 94% |
| | | 3 | 90,000 | 96% |

| | | | | |
|---|---|---|---|---|
| Example 1: ethylene 99.9%, <30 min, 40 °C, pressure of 10 bar Example 2: 4 hours, 40 °C, pressure of 10 bar Examples 3 and 4: ethylene 99.95%, 3 hours, 40 °C, pressure of 10 bar Examples 5 and 6: ethylene , 99.99%, 4 hours, 40 °C, pressure of 10 bar Examples 7 - 17: reactions for 10 ppm: ethylene 99.95%, 3 hours, 40 °C; reactions for 3 ppm: ethylene 99.95%, 3 - 6 hours, 40 °C; reactions for 0.5 ppm: ethylene 99.995%, 6 hours, 40 °C (ethylene pressure of 10 bar) ¹ Grubbs et al. Organometallics 2008, 27, 563-566. ² Zhang et al. Chem. Commun., 2013, 49, 9491-9493. ³ Bertrandt et al. Angew. Chem. Int. Ed., 2015, 54, 1919-1923*.* ⁴ Gawin et al. Angew. Chem. Int. Ed. 2017, 56, 981-986*.* | | | | |

### Example VI

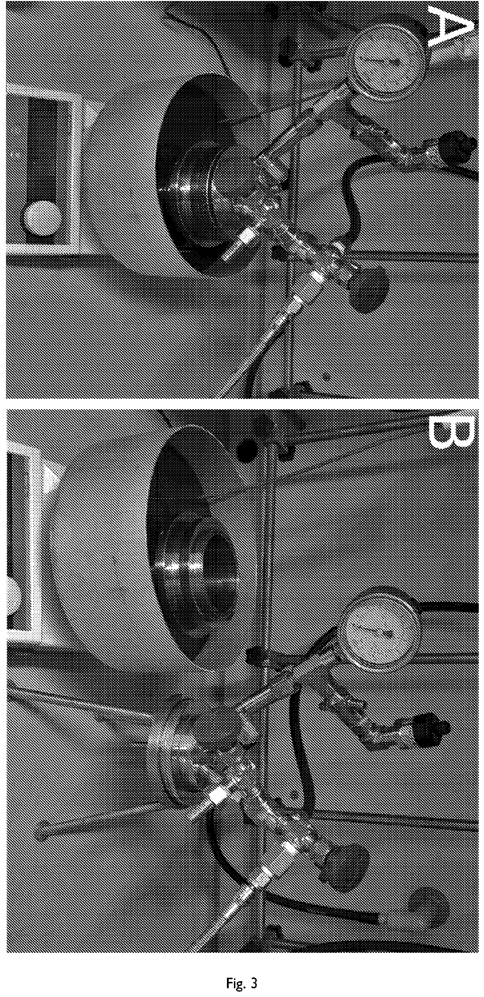

Testing the activity of the ruthenium complex in the ethenolysis reaction of methyl oleate outside the glovebox under conditions that do not require an inert gas protective atmosphere (Fig. 3). Fig. 3 shows A) the autoclave assembled in air, B) the open autoclave before the ethenolysis reaction.

The corresponding ethenolysis reactions described in Example V were performed outside the glovebox in an autoclave apparatus placed under the fume hood and located in the air - the reaction system shown in Fig. 3. Subsequent reaction steps were performed in the air without an inert gas protective atmosphere. Ethylene of different purities was used during the reaction, with the purity of ethylene being lower than that described in previous literature reports. Using the new ruthenium complexes **Ru1a** - **Ru22a** was observed to be resistant to the use of lower quality ethylene, which did not impair the reaction results as determined by TON and selectivity.

### Example VII

### Synthesis of a CAAC ligand and a spiro carbon catalyst

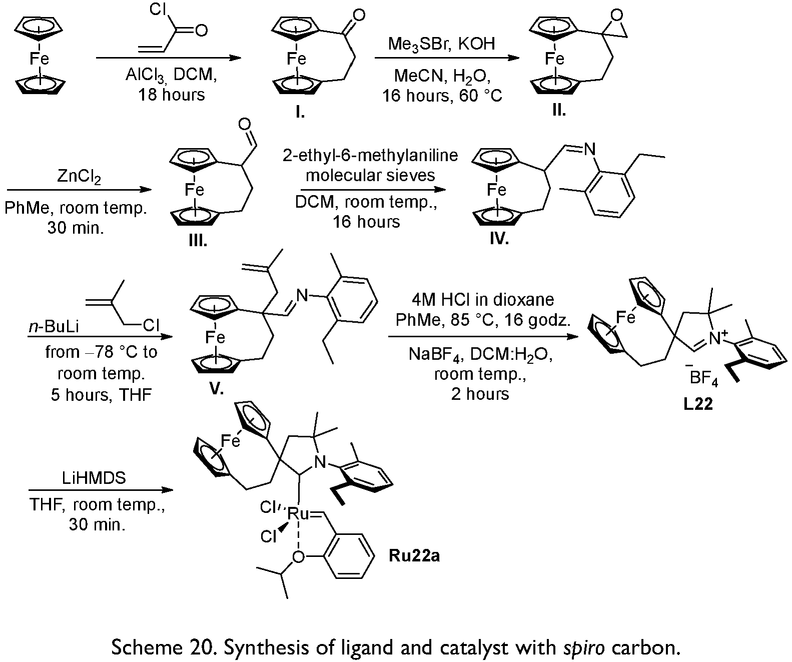

### Synthesis of compound I

Aluminium(III) chloride (14.3 g, 105 mmol, 1.00 equiv.) and anhydrous DCM (100 mL) were placed under an argon atmosphere in a three-neck flask equipped with a stirring element, and the suspension was cooled to -40 °C. In a two-neck flask under an argon atmosphere equipped with a stirring element, ferrocene (20.0 g, 105 mmol, 1.00 equiv.) was dissolved in anhydrous DCM (155 mL) and added dropwise into the aluminum(III) chloride suspension in DCM at -40 °C with stirring. The mixture was then cooled to -78 °C and acryloyl chloride (9.81 mL, 116 mmol, 1.10 equiv.) was added dropwise for 30 min at -78 °C. The reaction was carried out for 18 hours at -78 °C, then the contents of the flask were poured into water at 0 °C. The organic layer was separated, washed with brine, dried over MgSO₄, the drying agent was filtered through neutral Celite and the solvent was evaporated. The crude product was purified by column chromatography (Al₂O₃× 5% H₂O) to give the product as an orange solid (7.99 g, 33.3 mmol, 31%).

**¹H NMR (400 MHz, CDCl₃):** δ 4.87 - 4.79 (m, 2H), 4.66 - 4.56 (m, 2H), 4.43 - 4.30 (m, 2H), 4.10 - 3.94 (m, 2H), 3.06 - 2.86 (m, 4H).

**¹³C NMR (101 MHz, CDCl₃):** δ 212.0, 88.2, 74.1, 72.8, 71.2, 70.4, 69.4, 44.3, 31.9.

### Synthesis of compound II

Ketone 1 (1.16 g, 4.83 mmol, 1.0 equiv.), Me₃SBr (0.98 g, 6.28 mmol, 1.3 equiv.), KOH (0.70 g, 12.6 mmol, 2.6 equiv.), distilled water (0.25 mL), and MeCN (10 mL) were placed in a reaction vessel equipped with a stirring element under an argon atmosphere. The contents of the vessel were stirred for 16 h at 60 °C. After completion of the reaction, 5 mL of diethyl ether (Et₂O) was added, the precipitate was filtered off ,and the precipitate was washed again with diethyl ether (10 mL), the solvent was evaporated under reduced pressure. The residue was washed with diethyl ether (10 mL), the solvent evaporated and washed with n-hexane (10 mL), the solvent evaporated under reduced pressure to give the product as an orange solid (1.02 g, 4.01 mmol, 83%).

**¹H NMR (400 MHz, CDCl₃):** δ 4.44 - 4.35 (m, 1H), 4.25 - 4.22 (m, 1H), 4.19 - 4.16 (m, 2H), 4.10 - 4.04 (m, 4H), 2.95 (d, J = 5.5 Hz, 1H), 2.90 (dd, J = 0.6, 5.4 Hz, 1H), 2.33 - 2.16 (m, 3H), 2.09 - 1.99 (m, 1H).

**¹³C NMR (101 MHz, CDCl₃):** δ 86.6, 81.8, 70.9, 69.1, 68.9, 68.8, 68.7, 68.5, 68.4, 67.7, 56.4, 55.0, 42.5, 22.9.

### Synthesis of compound III

Epoxide **II** (0.94 g, 3.68 mmol, 1 equiv.), zinc(II) chloride (0.50 g, 3.68 mmol, 1 equiv.) and toluene (16 mL) were placed in a reaction vessel equipped with a stirring element. The reaction was carried out for 2 hours at room temperature. The reaction mixture was filtered off on a Schott funnel, the solvent was distilled off under reduced pressure to give the product as a bright orange solid (0.77 g, 3.04 mmol, 82%).

**¹H NMR (400 MHz, CDCl₃):** δ 9.82 (d, J = 1.5 Hz, 1H), 4.22 - 4.19 (m, 1H), 4.18 - 4.15 (m, 1H), 4.14 - 4.11 (m, 3H), 4.10 - 4.07 (m, 1H), 4.06 - 4.01 (m, 2H), 2.82 - 2.74 (m, 1H), 2.54 - 2.39 (m, 2H), 2.05 - 1.93 (m, 1H), 1.86 - 1.72 (m, 1H).

**¹³C NMR (101 MHz, CDCl₃):** δ 201.4, 86.4, 81.4, 71.5, 71.4, 70.0, 69.8, 68.9, 68.1, 68.0, 67.3, 50.4, 35.8, 23.9.

### Synthesis of compound IV

In a round-bottom flask-previously heated under reduced pressure-under an argon atmosphere, aldehyde **III** (0.7 g, 2.76 mmol, 1.0 equiv.), 2-ethyl-6-methylaniline (0.38 g, 2.76 mmol, 1.0 equiv.), 4Å molecular sieves (0.7 g), and methylene chloride (5.5 mL) were placed. The reaction was carried out for 16 h at room temperature. The molecular sieves were filtered off, the solvent was evaporated under reduced pressure to give the product as an orange solid. (0.56 g, 1.49 mmol, 54%).

**¹H NMR (400 MHz, CD₂Cl₂):** δ 7.75 (d, *J* = 4.7 Hz, 1H), 7.05 - 6.96 (m, 2H), 6.91 (t, *J* = 7.5 Hz, 1H), 4.22 - 4.18 (m, 1H), 4.16 - 4.12 (m, 2H), 4.13 - 4.05 (m, 4H), 4.06 - 4.00 (m, 1H), 3.08 - 2.94 (m, 1H), 2.57 - 2.47 (m, 2H), 2.43 (q, *J* = 7.5 Hz, 2H), 2.30 - 2.16 (m, 1H), 2.04 (s, 3H), 2.01 - 1.90 (m, 1H), 1.08 (t, *J* = 7.5 Hz, 3H).

**¹³C NMR (101 MHz, CD₂Cl₂):** δ 169.2, 151.0, 133.5, 128.2, 127.0, 126.6, 123.7, 86.9, 85.2, 71.6, 71.0, 69.6, 68.5, 68.2, 68.1, 67.2, 43.9, 39.2, 24.9, 24.4, 18.5, 14.9.

### Synthesis of compound V

Imine **IV** (0.55 g, 1.47 mmol, 1.00 equiv.) and anhydrous tetrahydrofuran (3.0 mL) were placed in a round-bottom flask-previously heated under reduced pressure-under an argon atmosphere. The mixture was then cooled to -78 °C in a dry ice - acetone cooling bath. A solution of n-BuLi (2.30 M solution in n-hexane, 0.77 mL, 1.76 mmol, 1.20 equiv.) was then added dropwise with intense stirring, followed by heating to room temperature. After one hour, the reaction mixture was cooled again to -78 °C in a dry ice - acetone cooling bath and 3-chloro-2-methylpropene (0.22 mL, 2.2 mmol, 1.50 equiv.) was added dropwise. The reaction was carried out for 16 h at room temperature. The solvent was evaporated under reduced pressure. The crude product was filtered through a syringe filter to give the product as a red oil.(0.51 g, 1.19 mmol, 80%).

**¹H NMR (400 MHz, CDCl₃):** δ 7.84 (s, 1H), 7.10 - 7.03 (m, 1H), 7.06 - 6.99 (m, 1H), 7.00 - 6.92 (m, 1H), 4.85 - 4.80 (m, 1H), 4.80 - 4.75 (m, 1H), 4.25 - 4.18 (m, 2H), 4.16 - 4.11 (m, 3H), 4.11 - 4.05 (m, 1H), 4.08 - 4.03 (m, 2H), 2.76 (d, J = 14.5 Hz, 1H), 2.70 - 2.64 (m, 1H), 2.58 (d, J = 14.5 Hz, 1H), 2.51 (qd, *J* = 1.6, 7.5 Hz, 2H), 2.43 - 2.32 (m, 2H), 2.24 - 2.19 (m, 1H), 2.15 (s, 3H), 1.69 (s, 3H), 1.16 (t, *J* = 7.5 Hz, 3H).

**¹³C NMR (101 MHz, CDCl₃):** δ 171.3, 150.4, 142.0, 133.3, 128.2, 127.0, 126.3, 123.7, 115.3, 89.6, 87.4, 68.8, 68.8, 68.5, 68.5, 68.4, 68.4, 67.7, 67.7, 46.2, 42.9, 29.8, 25.1, 24.6, 20.6, 19.1, 14.9.

### Synthesis of compound L22

The imine from the previous step (0.49 g, 1.15 mmol, 1 equiv.) dissolved in anhydrous PhMe (C=500 mM) was placed in a round-bottom flask under an argon atmosphere. The mixture was cooled to -78 °C in a dry ice - acetone cooling bath. HCl solution (0.7 mL, 4 M solution in dioxane, 2.88 mmol, 2.5 equiv.) was then added dropwise and the reaction was carried out for 16 h at 85 °C with vigorous stirring. The solvent was evaporated under reduced pressure. The crude product was dissolved in methylene chloride (about 5 mL), a saturated aqueous solution of NaBF₄ (0.25 g, 2.30 mmol, 2 equiv.) was added and the ion exchange was carried out for 2 h with vigorous stirring. The mixture was extracted three times with methylene chloride, dried with anhydrous magnesium sulfate and filtered through neutral Celite, after which the solvent was evaporated under reduced pressure. The product was precipitated from the DCM:Et₂O mixture to give the product as a light brown solid (0.27 g, 0.53 mmol, 45%).

### Synthesis of compound Ru22a

Under an argon atmosphere, the CAAC×BF₄ ligand **L22** (174 mg, 340 µmol, 1.20 equiv.), the first-generation Hoveyda-Grubbs complex (170 mg, 283 µmol, 1.00 equiv.) and anhydrous THF (C_{CAAC}=0.1 M) were placed in a preheated Schlenk flask and stirred for 1 min. LiHMDS (56 mg, 340 µmol, 1.20 equiv.) was then added and stirred until full conversion was achieved (5 min). After this time, copper(I) chloride (56 mg, 566 µmol, 2.00 equiv.) was added. The crude mixture was filtered through neutral alumina (Al₂O₃, neutral, Brockman Grade I) with methylene chloride as eluent. The green fraction was collected and the solvent was evaporated under reduced pressure. A small amount of n-pentane was then added and the mixture was placed in an ultrasonic bath. The product was drained and washed with cold n-pentane. The process was repeated twice and then using diethyl ether. After drying under vacuum, a green crystalline solid was obtained (164 mg, 220 µmol, 77%).

| Structure/Number | | | Selected analytical data |
|---|---|---|---|
| 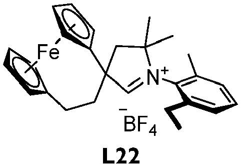 | | | ¹**H NMR (400 MHz, CD₂Cl₂):** δ 9.09 (s, 1H), 7.49 - 7.40 (m, 1H), 7.35 (d, *J* = 7.7 Hz, 0.62×1H), 7.27 (d, *J* = 7.2 Hz, 0.38×1H), 7.25 (d,*J* = 7.3 Hz, 0.38×1H), 7.20 (d, J = 7.5 Hz, 0.62×1H), 4.57 - 4.52 (m, 0.62×1H), 4.52 - 4.48 (m, 0.38×1H), 4.40 - 4.35 (m, 1H), 4.28 - 4.24 (m, 3H), 4.23 - 4.19 (m, 1H), 4.19 - 4.16 (m, 1H), 4.15 - 4.11 (m, 1H), 2.80 (d, J = 13.9 Hz, 0.62×1H), 2.79 (d, J = 13.9 Hz, 0.38×1H), 2.70 - 2.39 (m, 1H + 2×0.31×2H + 1H + 2H), 2.33 (s, 0.38×3H), 2.31 - 2.22 (m, 0.19×2H), 2.09 - 1.99 (m, 0.19×2H), 1.96 (s, 0.62×3H), 1.94 - 1.88 (m, 1H), 1.58 (s, 0.38×3H), 1.55 (s, 0.62×3H), 1.50 (s, |

| Structure/Number | Selected analytical data | | |
|---|---|---|---|
| | 0.62×3H), 1.48 (s, 0.38×3H), 1.26 (t, *J* = 7.5 Hz, 0.62×3H), 0.99 (t, *J* = 7.5 Hz, 0.38×3H); **¹³C NMR (101 MHz, Methylene chloride-*d*₂):** δ 189.7, 189.7, 140.5, 140.4, 134.3, 133.9, 131.9, 131.8, 131.4, 131.4, 130.2, 130.1, 128.3, 128.0, 86.3, 86.0, 84.6, 84.5, 84.4, 84.1, 72.0, 71.8, 71.8, 71.1, 69.9, 69,8, 69.3, 69.2, 69.1, 68.5, 68.4, 67.7, 67.5, 51.2, 51.1, 46.0, 45.7, 45.0, 44.8, 28.4, 27.6, 26.9, 25.5, 24.6, 19.7, 19.3, 19.2, 18.9, 15.6, 15.0 | | |

| Structure/Symbol | Molecular formula | Molar mass [g/mol] | ¹H NMR δ (ppm) Ru=C***H*** (solvent) |
|---|---|---|---|
| 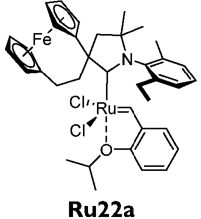 | C₃₇H₄₃Cl₂FeNORu | 745.1115 | 16.40, 16.36 CDCl₃ |

## Claims

1. A precursor of cyclic alkylamine carbenes (CAAC) of the formula **CAAC-1** 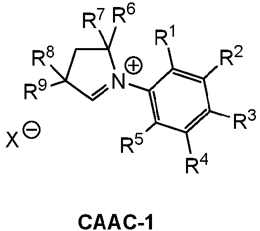
wherein X denotes an anion selected from the group comprising halogen atom, BF₄⁻, PF₆⁻, ClO₄⁻, CF₃SO₂O;
R¹, R², R³, R⁴ and R⁵ independently denote a hydrogen atom, a C₁-C₁₂ alkyl group, a C₃-C₁₂ cycloalkyl group, a C₅-C₂₀ aryl group or a C₅-C₂₀ heteroaryl group, a C₅-C₂₅ aralkyl group, which may be substituted independently by one and/or more substituents selected from the group comprising a hydrogen atom, a halogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxyl group, a C₅-C₂₄ aryloxyl group, a C₅-C₂₀ heteroaryloxyl group, a sulfide (-SR") group, an amino (-NR"₂) group, in which the R" group independently denotes a hydrogen atom, C₁-C₅ alkyl, C₆-C₂₄ aryl, C₇-C₂₄ aralkyl, alternatively R¹, R², R³, R⁴ and R⁵ are connected to form a C₅-C₂₅ ring;
each substituent R⁶, R⁷, and R⁸ denotes a hydrogen atom, a halogen atom, a C₁-C₁₂ alkyl group, or a C₅-C₂₀ aryl group, which may be substituted independently by one and/or more substituents selected from the hydrogen-containing group, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxyl group, a C₅-C₂₄ aryloxyl group, a C₅-C₂₀ heteroaryloxyl or a halogen atom, a sulfide group (-SR"), an amino group (-NR"₂), in which the R" group independently denotes a hydrogen atom, C₁-C₅ alkyl, C₆-C₂₄ aryl, C₇-C₂₄ aralkyl;
R⁹ substituent means a substituted or unsubstituted heterocyclic group or an organometallic complex group selected from thiophene, benzothiophene, furan, benzofuran or ferrocene, which can be substituted independently by one and/or more substituents selected from the group comprising a hydrogen atom, a halogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxyl group, a C₅-C₂₄ aryloxyl group, a C₅-C₂₀ heteroaryloxyl group, a sulfide group (-SR"), an amino group (-NR"₂), wherein the R" group independently denotes a hydrogen atom, C₁-C₅ alkyl, C₆-C₂₄ aryl, C₇-C₂₄ aralkyl.

2. The precursor of the formula **CAAC-1** according to claim 1, having the structure represented by the formula **L1, L2, L3, L4, L5, L6, L7, L8, L9, L10, L11, L12, L13, L14, L15, L16, L17, L18, L19, L20, L21** or **L22:**

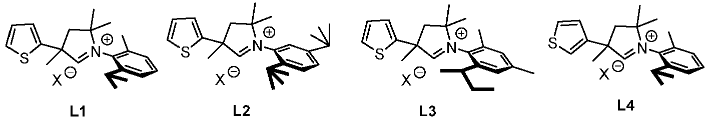

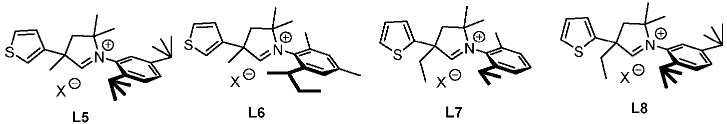

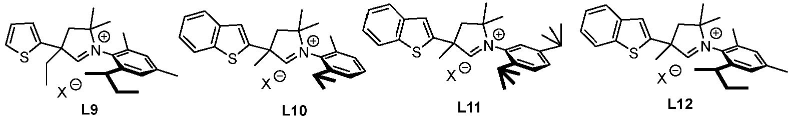

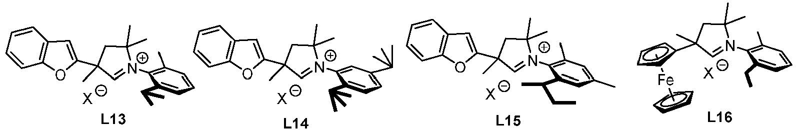

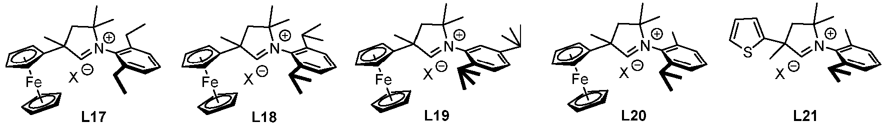

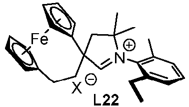

3. A ruthenium complex of formula **1-Ru** 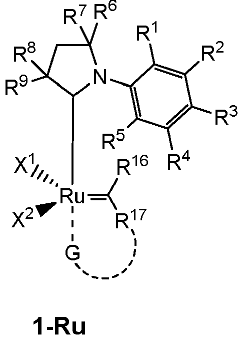 wherein:
X¹ and X² independently of each other denote an anionic ligand selected from the group comprising a halogen anion, a -CN, -SCN, -OR^{a}, -SR^{a}, -O(C=O)R^{a}, -O(SO₂)R^{a} and -OSi(R^{a})₃ group, wherein R^{a} denotes C₁-C₁₂ alkyl, C₃-C₁₂ cycloalkyl, C₂-C₁₂ alkenyl or C₅-C₂₀ aryl, which is optionally substituted with at least one C₁-C₁₂ alkyl, C₁-C₁₂ perfluoroalkyl, C₁-C₁₂ alkoxyl, C₅-C₂₄ aryloxyl, C₅-C₂₀ heteroaryloxyl or a halogen atom;
R¹, R², R³, R⁴ and R⁵ denote independently a hydrogen atom, a C₁-C₁₂ alkyl group, a C₃-C₁₂ cycloalkyl group, a C₅-C₂₀ aryl group or a C₅-C₂₀ heteroaryl group, a C₅-C₂₅ aralkyl group, which may be independently substituted with one and/or more substituents selected from the group comprising a hydrogen atom, a halogen atom, a C₁-C₁₂ alkyl group, a C₁-C₁₂ perfluoroalkyl group,
a C₅-C₂₀ aryl group, a C₅-C₂₀ perfluoroaryl group, a C₅-C₂₀ heteroaryl group, a C₁-C₁₂ alkoxyl group, a C₅-C₂₄ aryloxyl group, a C₅-C₂₀ heteroaryloxyl group, a sulfide group (-SR"), an amino group (-NR"₂), in which the R" group independently denotes a hydrogen atom, C₁-C₅ alkyl, C₆-C₂₄ aryl, C₇-C₂₄ aralkyl,
alternatively R¹, R², R³, R⁴ and R⁵ are bonded with each other to form a C₅-C₂₅ ring;
each substituent R⁶, R⁷, and R⁸ denotes a hydrogen atom, a halogen atom, a C₁-C₁₂ alkyl group, or a C₅-C₂₀ aryl group, which may be substituted independently with one and /or more substitutes selected from the group comprising a hydrogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, C₁-C₁₂ alkoxyl group, a C₅-C₂₄ aryloxyl group, a C₅-C₂₀ heteroaryloxyl group or halogen atom, a sulfide group (-SR"), an amino group (-NR"₂), in which the group R" independently denotes a hydrogen atom, C₁-C₅ alkyl, C₆-C₂₄ aryl, C₇-C₂₄ aralkyl;
R⁹ substituent denotes a substituted or unsubstituted heterocyclic group and/or organometallic complex group selected from thiophene, benzothiophene, furan, benzofuran or ferrocene, which may be substituted independently with one and/or more substituents selected from the group comprising a hydrogen atom, a halogen atom, a C₁-C₁₂ alkyl group, a C₁-C₁₂ perfluoroalkyl group, a C₅-C₂₀ aryl group, a C₅-C₂₀ perfluoroaryl group, a C₅-C₂₀ heteroaryl group, a C₁-C₁₂ alkoxyl group, a C₅-C₂₄ aryloxyl group, a C₅-C₂₀ heteroaryloxyl group, a sulfide group (-SR"), an amino group(-NR"₂), in which the R" group denotes independently a hydrogen atom, C₁-C₅ alkyl, C₆-C₂₄ aryl, C₇-C₂₄ aralkyl,
R¹⁶ and R¹⁷ denote independently a hydrogen atom, a halogen atom, C₁-C₂₅ alkyl, C₃-C₂₅ cycloalkyl, C₁-C₁₂ perfluoroalkyl, C₂-C₂₅ alkene, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₅ aryl, C₅-C₂₅ aryloxyl, C₆-C₂₅ arylalkyl, C₅-C₂₅ heteroaryl, C₅-C₂₅ heteroaryloxyl, C₅-C₂₅ perfluoroaryl, a 3-12-membered heterocycle comprising a sulfur, oxygen, nitrogen, selenium or a phosphorus atom;
whereby the substituents R¹⁶ and R¹⁷ may be linked together to form a ring selected from the group comprising C₃-C₂₅ cycloalkyl, C₃-C₂₅ cycloalkenyl, C₃-C₂₅ cycloalkynyl, C₅-C₂₅ aryl, C₅-C₂₅ heteroaryl, C₅-C₂₅ perfluoroaryl, a 3-12-membered heterocycle comprising a sulfur, oxygen, nitrogen, selenium or a phosphorus atom, which may be substituted independently by one or more substituents selected from the group comprising a hydrogen atom, a halogen atom, C₁-C₂₅ alkyl, C₃-C₂₅ cycloalkyl, C₁-Cₗ₂ perfluoroalkyl, C₂-C₂₅ alkene, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₅ aryl, C₅-C₂₅ aryloxyl, C₆-C₂₅ arylalkyl, C₅-C₂₅ heteroaryl, C₅-C₂₅ heteroaryloxyl, C₅-C₂₅ perfluoroaryl, a 3-12-membered heterocycle;
G is selected from such as
- ligand with the formula **CAAC-1** 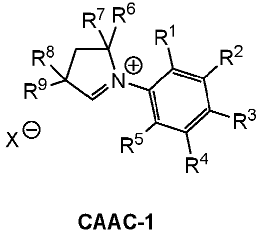 in which X and the substituents R¹ to R⁹ have the above defined meanings
either
- heteroatom **1**
selected from the group comprising an oxygen atom, a sulfur atom, a selenium atom, substituted by a group selected from such as a hydrogen atom, a fluorine atom, an oxygen atom, C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, a 3-12-membered heterocycle, optionally substituted with an acyl group (-COR'), a cyano group (-CN), a carboxyl group (-COOH), an ester group (-COOR'), an ester group (-CH₂COOR'), an ester group (-CHR'COOR'), an ester group (-C(R')₂COOR'), an amide group (-CONR'₂), a Weinreb-type amide (-CON(R')(OR')), a sulfone group (-SO₂R'), a formyl group (-CHO), a sulfonamide group
(-SO₂NR'₂), a ketone group (-COR'), a thioamide group (-CSNR'₂), a thioketone (-CSR'), a thionoester group (-CSOR'), a thioester group (-COSR'), a dithioester group (-CS₂R'), in which the group R' denotes independently C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl, C₅-C₂₄ heteroaryloxyl and then the dashed line denotes a direct bond between a heteroatom and the substituent R¹⁷ or denotes a bond between the substituent R¹⁷ and a heteroatom via a methylene bridge -CH₂-, -CHR'-, or -CR'₂- wherein the substituent R¹⁴ is C₅-C₁₅ aryl, optionally substituted with 1-4 substituents, independently selected from a group comprising a hydrogen atom, a halogen atom, C₁-C₂₅ alkyl, C₃-C₂₅ cycloalkyl, C₂-C₂₅ alkenyl, C₃-C₂₅ cycloalkenyl, C₂-C₂₅ alkynyl, C₃-C₂₅ cycloalkynyl, C₁-C₂₅ perfluoroalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₆-C₂₀ heteroaryl or C₅-C₂₄ heteroaryloxyl, a 3-12-membered heterocycle, an alkoxyl group (-OR"), a sulfide group (-SR"),
a sulfoxide group (-S(O)R"), a sulfonium group (-S⁺R"₂), a sulfone group (-SO₂R"), a sulfonamide group (-SO₂NR"₂), an amino group (-NR"₂), an ammonium group (-N⁺R"₃), a nitro group (-NO₂), a cyano group (-CN), a phosphinous group (-P(O)(OR")₂), a phosphinic group (-P(O)R"(OR")), a phosphonine group (-P(OR")₂), a phosphine group (-PR"₂), a phosphine oxide group (-P(O)R"₂), a phosphonium group (-P⁺R"₃), a carboxyl group (-COOH), an ester group (-COOR"), an amide group (-CONR"₂), an amide group (-NR"C(O)R"), a formyl group (-CHO), a ketone group (-COR"), a thioamide group (-CSNR"₂), a thioketone group (-CSR"), a thionoester group (-CSOR"), a thioester group (-COSR"), a dithioester group (-CS₂R"), in which the R" group denotes C₁-C₅ alkyl, C₁-C₅ perfluoroalkyl, C₆-C₂₄ aryl, C₇-C₂₄ aralkyl, C₅-C₂₄ perfluoroaryl, or
- heteroatom 2
selected from a group comprising a nitrogen atom or a phosphorus atom, substituted with the group selected from such as a hydrogen atom, methylidene, optionally substituted with the substituent R', C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, a 3-12-membered heterocycle, an acyl group (-COR'), an ester group (-COOR'), a tert-butyl butyloxycarbonyl group (t-Boc) or a 9-fluorenylmethoxycarbonyla group (Fmoc), a carbamine group (-CONR'₂), a sulfone group (-SO₂R'), a formyl group (-CHO), in which the R' group denotes C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, optionally substituted with an acyl group (-COR'), a cyano group (-CN), a carboxyl group (-COOH), an ester group (-COOR'), an ester group (-CH₂COOR'), an ester group (-CHR'COOR'), an ester group (-C(R')₂COOR'), an amide group (-CONR'₂), a sulfone group
(-SO₂R'), a formyl group (-CHO), a sulfonamide group (-SO₂NR'₂), a ketone group (-COR'), a thioamide group (-CSNR'₂), a thioketone group (-CSR'), a thionoester group (-CSOR'), a thioester group (-COSR'), a dithioester group (-CS₂R'), in which the R' group denotes C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, and then the dashed line denotes a direct bond between the heteroatom and the substituent R¹⁴ or it denotes a bond between the substituent R¹⁷ with a heteroatom via methylene bridge (CH₂)-, -(CHR')-, or -(CR'₂)-; wherein the substituent R¹⁷ is C₅-C₁₅ aryl, optionally substituted with 1-4 substituents independently selected from the group comprising a hydrogen atom, a halogen atom, C₁-C₂₅ alkyl, C₃-C₂₅ cycloalkyl, C₂-C₂₅ alkenyl, C₃-C₂₅ cycloalkenyl, C₂-C₂₅ alkynyl, C₃-C₂₅ cycloalkynyl, C₁-C₂₅ perfluoroalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₆-C₂₀ heteroaryl or C₅-C₂₄ heteroaryloxyl, a 3-12-membered heterocycle, an alkoxyl group (-OR"), a sulfide group (-SR"), a sulfoxide group (-S(O)R"), a sulfonium group (-S⁺R"₂), a sulfone group (-SO₂R"), a sulfonamide group (-SO₂NR"₂), an amino group (-NR"₂), an ammonium group (-N⁺R"₃), a nitro group (-NO₂), a cyano group (-CN), a phosphinous group (-P(O)(OR")₂), a phosphinic group (-P(O)R"(OR")), a phosphonine group (-P(OR")₂), a phosphine group (-PR"₂), a phosphine oxide group (-P(O)R"₂), a phosphonium group (-P⁺R"₃), a carboxyl group (-COOH), an ester group (-COOR"), an amide group (-CONR"₂), an amide group (-NR"C(O)R"), a formyl group (-CHO), a ketone group (-COR"), a thioamide group (-CSNR"₂), a thioketone group (-CSR"), a thionoester group (-CSOR"), a thioester group (-COSR"), a dithioester group (-CS₂R"), in which the group R" denotes C₁-C₅ alkyl, C₁-C₅ perfluoroalkyl, C₆-C₂₄ aryl, C₇-C₂₄ aralkyl, C₅-C₂₄ perfluoroaryl, or
- heteroatom 3
selected from a group comprising a halogen atom and then the dashed line denotes a direct bond between the heteroatom and the R¹⁷ substituent, wherein the R¹⁷ substituent is C₅-C₁₅ aryl, or C₅-C₂₅ polyaryl, optionally substituted with 1-4 substituents selected independently from a group comprising a hydrogen atom, a halogen atom, C₁-C₂₅ alkyl, C₃-C₂₅ cycloalkyl, C₂-C₂₅ alkenyl, C₃-C₂₅ cycloalkenyl, C₂-C₂₅ alkynyl, C₃-C₂₅ cycloalkynyl, C₁-C₂₅ perfluoroalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₆-C₂₀ heteroaryl or C₅-C₂₄ heteroaryloxyl, a 3-12-membered heterocycle, an alkoxy group (-OR"), a sulfide group (-SR"), a sulfoxide group (-S(O)R"), a sulfonium group (-S⁺R"₂), a sulfone group (-SO₂R"), a sulfonamide group (-SO₂NR"₂), an amino group (-NR"₂), an ammonium group (-N⁺R"₃), a nitro group (-NO₂), a cyano group (-CN), a phosphonous group (-P(O)(OR")₂), a phosphinous group (-P(O)R"(OR")), a phosphonine group (-P(OR")₂), a phosphine group (-PR"₂), a phosphine oxide group (-P(O)R"₂), a phosphonium group (-P⁺R"₃), a carboxyl group (-COOH), an ester group (-COOR"), an amide group (-CONR"₂), an amide group (-NR"C(O)R"), a formyl group (-CHO), a ketone group (-COR"), a thioamide group (-CSNR"₂), a thioketone group (-CSR"), a thionoester group (-CSOR"), a thioester group (-COSR"), a dithioester group (-CS₂R"), in which the R" group denotes C₁-C₅ alkyl, C₁-C₅ perfluoroalkyl, C₆-C₂₄ aryl, C₇-C₂₄ aralkyl, C₅-C₂₄ perfluoroaryl.

4. The ruthenium complex according to claim 3 represented by the formula **1a-Ru** 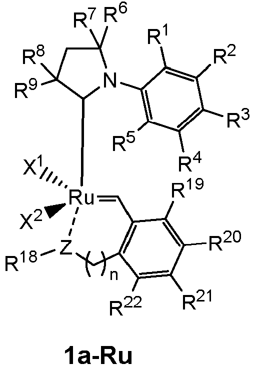 in which X¹ and X² and the R¹, R², R³, R⁴, R⁵, R⁶, R⁷, R⁸, R⁹ substituents have the meanings defined above
'n' means 1 or 0
Z is selected from a group comprising halogen atoms, O atom, S atom, Se atom, or a NR‴ group, in which R‴ denotes methylidene, C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl, a 3-12-membered heterocycle, an acyl group (-COR'), an ester group (-COOR'), a tert-butyloxycarbonyl group (t-Boc) or a 9-fluorenylmethoxycarbonyl group (Fmoc), a carbamine group (-CONR'₂), a sulfone group (-SO₂R'), a formyl group (-CHO), in which the R' group denotes C₁-C₂₅ alkyl, C₁-C₂₅ perfluoroalkyl, C₃-C₂₅ cycloalkyl, C₅-C₂₀ alkoxyl, C₅-C₂₀ aryl, C₅-C₂₀ perfluoroaryl, C₇-C₂₀ aralkyl, C₅-C₂₄ aryloxyl, C₂-C₁₂ alkenyl, C₆-C₂₀ heteroaryl or C₅-C₂₄ heteroaryloxyl,
or a halogen atom, wherein if Z denotes a halogen atom, R¹⁸ is absent;
R¹⁸ means independently a hydrogen atom, C₁-C₂₅ alkyl, C₁-C₂₅ cycloalkyl, C₅-C₂₀ alkoxyl, C₅-C₂₀ aryl, C₅-C₂₄ aryloxyl, a -COOR‴ group, a -CH₂COOR‴ group, a -CONR‴₂ group, a -CH₂CONR‴₂ group, a -COR‴ group, a -CH₂COR‴ group, a -CON(OR‴)(R‴) group, a -CH₂CON(OR‴)(R‴) group or a halogen atom, wherein R‴ denotes C₁-C₁₂ alkyl, C₃-C₁₂ cycloalkyl, C₂-C₁₂ alkenyl, C₆-C₂₀ aryl, which are optionally substituted with at least one C₁-C₁₂ alkyl, C₁-C₁₂ perfluoroalkyl, C₁-C₁₂ alkoxyl, C₆-C₂₄ aryloxyl, or a halogen atom;
R¹⁹, R²⁰, R²¹, and R²² denote independently a hydrogen atom, a halogen atom, a C₁-C₂₅ alkyl group, a C₂-C₂₅ alkenyl group, a C₅-C₂₅ aryl group, an alkoxy group (-OR"), a sulfide group (-SR"), a sulfoxide group (-S(O)R"), a sulfonium group (-S⁺R"₂), a sulfone group (-SO₂R"), a sulfonamide group (-SO₂NR"₂), an amino group (-NR"₂), an ammonium group (-N⁺R"₃), a nitro group (-NO₂), a cyano group (-CN), a phosphinous group (-P(O)(OR")₂), a phosphinic group (-P(O)R"(OR")), a phosphonine group (-P(OR")₂), a phosphine group (-PR"₂), a phosphine oxide group (-P(O)R"₂), a phosphonium group (-P⁺R"₃), a carboxyl group (-COOH), an ester group (-COOR"), an amide group (-CONR"₂), an amide group (-NR"C(O)R"), a formyl group (-CHO), a ketone group (-COR"), a thioamide group (-CSNR"₂), a thioketone group (-CSR"), a thionoester group (-CSOR"), a thioester group (-COSR"), a dithioester group (-CS₂R"), in which the R" group denotes C₁-C₅ alkyl, C₁-C₅ perfluoroalkyl, C₆-C₂₄ aryl, C₇-C₂₄ aralkyl, C₅-C₂₄ perfluoroaryl, wherein the R¹⁶, R¹⁷, R¹⁸ and R¹⁹ substituents may be connected, thus forming a substituted or unsubstituted C₄-C₁₀ cyclic or C₄-C₁₂ polycyclic system.

5. The ruthenium complex according to claim 3 or claim 4 of the formula **1b-Ru** 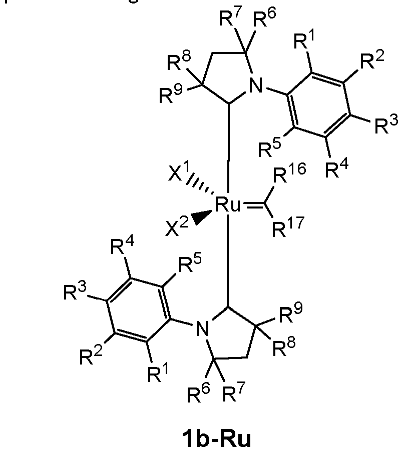
wherein X¹ and X² and the substituents R¹, R², R³, R⁴, R⁵, R⁶, R⁷, R⁸, R⁹ have the above defined meanings
R¹⁶ and R¹⁷ denote independently a hydrogen atom, a halogen atom, C₁-C₂₅ alkyl, optionally substituted; C₃-C₂₅ cycloalkyl, optionally substituted; C₁-C₁₂ perfluoroalkyl, optionally substituted; C₂-C₂₅ alkene, optionally substituted; C₂-C₂₅ alkenyl, optionally substituted; C₃-C₂₅ cycloalkenyl, optionally substituted; C₂-C₂₅ alkynyl, optionally substituted;C₃-C₂₅ cycloalkyl, optionally substituted; C₁-C₂₅ alkoxyl, optionally substituted; C₅-C₂₅ aryl, optionally substituted; C₅-C₂₅ aryloxyl, optionally substituted; C₆-C₂₅ arylalkyl, optionally substituted; C₅-C₂₅ heteroaryl, optionally substituted; C₅-C₂₅ heteroaryloxyl, optionally substituted; C₅-C₂₅ perfluoroaryl, optionally substituted; a 3-12-membered heterocycle comprising a sulfur, oxygen, nitrogen, selenium or a phosphorus atom, optionally substituted;
wherein the R¹⁶ and R¹⁷ substituents may be connected, forming a ring selected from a group comprising C₃-C₂₅ cycloalkyl, C₃-C₂₅ cycloalkenyl, C₃-C₂₅ cycloalkynyl, C₅-C₂₅ aryl, C₅-C₂₅ heteroaryl, C₅-C₂₅ perfluoroaryl, a 3-12-membered heterocycle containing a sulfur, oxygen, nitrogen, selenium or phosphorus atom, which may be independently substituted with one or more substituents selected from a group comprising a hydrogen atom, a halogen atom, C₁-C₂₅ alkyl, C₃-C₂₅ cycloalkyl, C₁-C₁₂ perfluoroalkyl, C₂-C₂₅ alkene, C₂-C₂₅ alkenyl, C₃-C₂₅ cycloalkenyl, C₂-C₂₅ alkynyl, C₃-C₂₅ cycloalkynyl, C₁-C₂₅ alkoxyl, C₅-C₂₅ aryl, C₅-C₂₅ aryloxyl, C₆-C₂₅ arylalkyl, C₅-C₂₅ heteroaryl, C₅-C₂₅ heteroaryloxyl, C₅-C₂₅ perfluoroaryl, a 3-12-membered heterocycle.

6. The ruthenium complex according to any one of claims 3-4, which is selected from the complexes represented by the formulae of **Ru1a, Ru2a, Ru3a, Ru4a, Ru5a, Ru6a, Ru7a, Ru8a, Ru9a, Ru10a, Ru11a, Ru11a, Ru12a, Ru13a, Ru14a, Ru15a, Ru16a, Ru17a, Ru18a, Ru19a, Ru20a, Ru21a, Ru22a:**

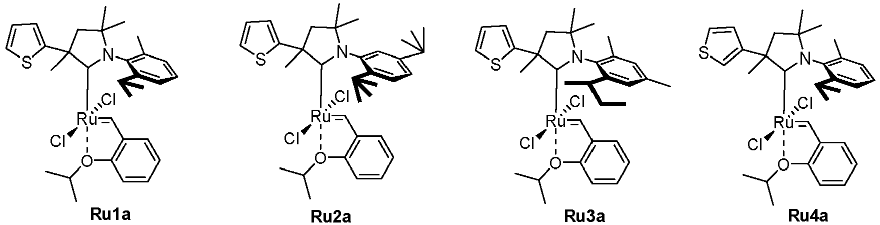

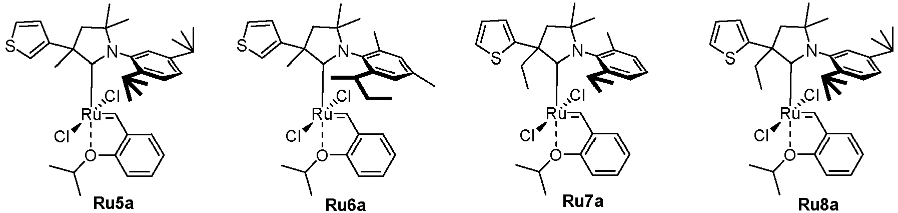

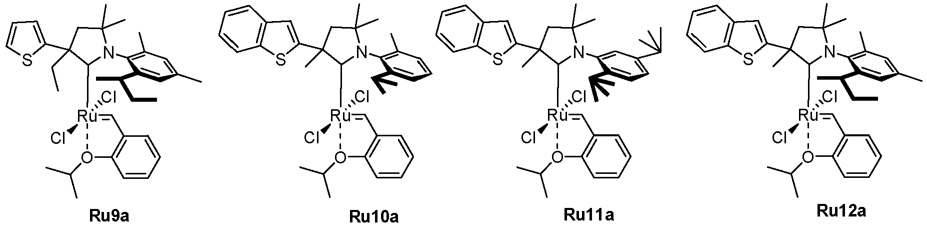

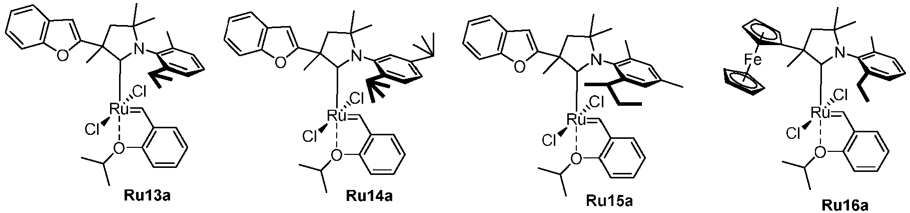

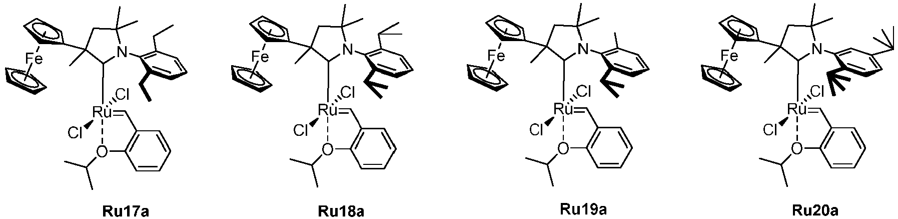

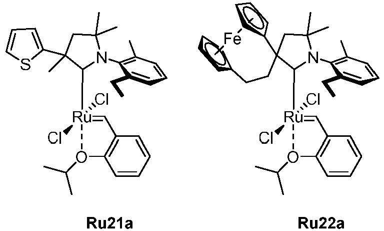

7. Method for the synthesis of the ruthenium complex of the formula **1a-Ru** 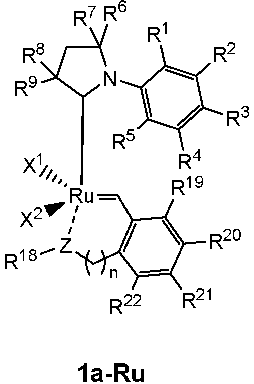 as defined in claim 4, **characterized in that** the alkylidene ruthenium complex of the formula 10 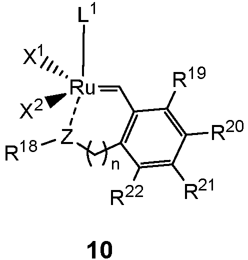 wherein:
L¹ denotes a neutral ligand selected from the group comprising pyridine or substituted pyridine, P(R')₃, P(OR')₃, O(R')₂, N(R')₃, wherein each R' independently denotes C₁-C₁₂ alkyl, C₃-C₁₂ cycloalkyl, C₅-C₂₀ aryl, C₇-C₂₄ aralkyl, C₅-C₂₄ perfluoroaryl, a 5-12-membered heteroaryl;
N, Z, X¹, X² and the substituents R¹⁸, R¹⁹, R²⁰, R²¹ and R²² have the above defined meanings
undergoes a reaction with the carbene of formula 8 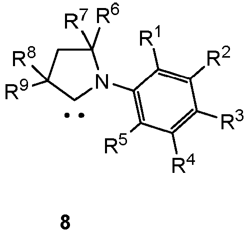
wherein the substituents R¹ to R⁹ have the above defined meanings.

8. Use of the compound of formula **1-Ru,** as defined in any one of claims 3-6, as a precatalyst and/or catalyst in olefin metathesis reactions, particularly in ring-closing metathesis (RCM), cross-metathesis (CM), homometathesis (cross-metathesis between two molecules of the same olefin), ethenolysis, isomerization, in a diastereoselective ring rearrangement metathesis (DRRM) reaction, alkene-alkyne (ene-yn) metathesis or ROMP or ADMET polymerization reaction.

9. Use according to claim 8, wherein the reaction is carried out in an organic solvent such as toluene, mesitylene, hexane, cyclohexane, ethyl acetate, methyl acetate, methyl carbonate, ethyl carbonate, tertbutyl methyl ether, cyclopentyl methyl ether, diethyl ether, THF, 2 Me-THF, 4-Me-THP, dioxane, DME, PAO. PEG, paraffin, esters of saturated fatty acids.

10. Use according to claim 8, wherein the reaction is carried out in a solvent-free system.

11. Use according to any one of the preceding claims, wherein the reaction is carried out at a temperature between 20 °C and 200 °C.

12. Use according to any one of the preceding claims, wherein the reaction is carried out over a period between 5 minutes and 48 hours.

13. Use according to any one of the preceding claims, wherein the compound **1-Ru** is applied in amount not exceeding 10 mol%, preferably in amount of not exceeding 0.1 mol%.

14. Use according to any one of the preceding claims, wherein the compound **1-Ru** is added to the reaction mixture in solid portions and/or continuously using a pump as a solution in an organic solvent.

15. Use according to any one of the preceding claims, wherein the gaseous reaction by-product, selected from ethylene, propylene, butylene, is actively removed from the reaction mixture using an inert gas barbotage or by means of a vacuum.

## Patentansprüche

1. Präkursor von zyklischen Alkylamin-Carbenen (CAAC) der Formel **CAAC-1** 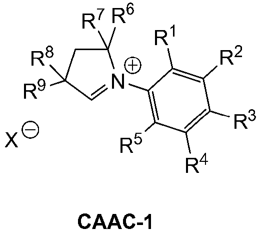
wobei X ein Anion bezeichnet, ausgewählt aus der Gruppe umfassend Halogenatome, BF₄⁻, PF₆⁻, ClO₄⁻, CF₃SO₂O⁻;
R¹, R², R³, R⁴ und R⁵ unabhängig ein Wasserstoffatom, eine C₁-C₁₂-Alkylgruppe, eine C₃-C₁₂-Cycloalkylgruppe, eine C₅-C₂₀-Arylgruppe oder eine C₅-C₂₀-Heteroarylgruppe, eine C₅-C₂₅-Aralkylgruppe bezeichnen, die unabhängig durch einen und/oder mehrere Substituenten substituiert sein können, ausgewählt aus der Gruppe umfassend ein Wasserstoffatom, ein Halogenatom, eine C₁-C₁₂-Alkylgruppe, eine C₁-C₁₂-Perfluoralkylgruppe, eine C₅-C₂₀-Arylgruppe, eine C₅-C₂₀-Perfluorarylgruppe, eine C₅-C₂₀-Heteroarylgruppe, eine C₁-C₁₂-Alkoxylgruppe, eine C₅-C₂₄-Aryloxylgruppe, eine C₅-C₂₀-Heteroaryloxylgruppe, eine Sulfidgruppe (-SR"), eine Aminogruppe (-NR"₂), in der die R"-Gruppe unabhängig ein Wasserstoffatom, C₁-C₅-Alkyl, C₆-C₂₄-Aryl, C₇-C₂₄-Aralkyl, bezeichnet, alternativ R¹, R², R³, R⁴ und R⁵ verbunden sind, um einen C₅-C₂₅-Ring zu bilden;
jeder Substituent R⁶, R⁷ und R⁸ ein Wasserstoffatom, ein Halogenatom, eine C₁-C₁₂-Alkylgruppe oder eine C₅-C₂₀-Arylgruppe bezeichnet, die unabhängig durch einen und/oder mehrere Substituenten substituiert sein kann, ausgewählt aus der wasserstoffhaltigen Gruppe, einer C₁-C₁₂-Alkylgruppe, einer C₁-C₁₂-Perfluoralkylgruppe, einer C₅-C₂₀-Arylgruppe, einer C₅-C₂₀-Perfluorarylgruppe, einer C₅-C₂₀-Heteroarylgruppe, einer C₁-C₁₂-Alkoxylgruppe, einer C₅-C₂₄-Aryloxylgruppe, einem C₅-C₂₀-Heteroaryloxyl oder einem Halogenatom, einer Sulfidgruppe (-SR"), eine Aminogruppe (-NR"₂), in denen die Gruppe R" unabhängig ein Wasserstoffatom, C₁-C₅-Alkyl, C₆-C₂₄-Aryl oder C₇-C₂₄-Aralkyl bezeichnet;
Substituent R⁹ eine substituierte oder unsubstituierte heterocyclische Gruppe oder eine metallorganische Komplexgruppe, ausgewählt aus Thiophen, Benzothiophen, Furan, Benzofuran oder Ferrocen bedeutet, die unabhängig durch einen und/oder mehrere Substituenten substituiert sein kann, ausgewählt aus der Gruppe umfassend ein Wasserstoffatom, ein Halogenatom, eine C₁-C₁₂-Alkylgruppe, eine C₁-C₁₂-Perfluoralkylgruppe, eine C₅-C₂₀-Arylgruppe, eine C₅-C₂₀-Perfluorarylgruppe, eine C₅-C₂₀-Heteroarylgruppe, eine C₁-C₁₂-Alkoxylgruppe, eine C₅-C₂₄-Aryloxylgruppe, eine C₅-C₂₀-Heteroaryloxylgruppe, eine Sulfidgruppe (-SR"), eine Aminogruppe (-NR"₂), wobei die R"-Gruppe unabhängig ein Wasserstoffatom, C₁-C₅-Alkyl, C₆-C₂₄-Aryl oder C₇-C₂₄-Aralkyl bezeichnet.

2. Präkursor der Formel **CAAC-1** nach Anspruch 1, der die durch die Formel **L1, L2, L3, L4, L5, L6, L7, L8, L9, L10, L11, L12, L13, L14, L15, L16, L17, L18, L19, L20, L21** oder **L22** dargestellte Struktur aufweist:

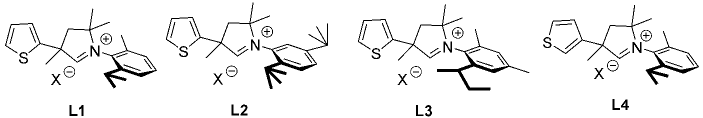

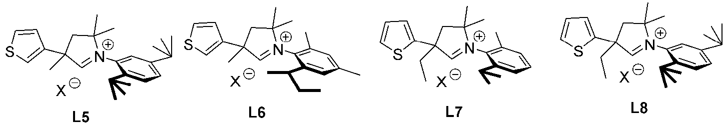

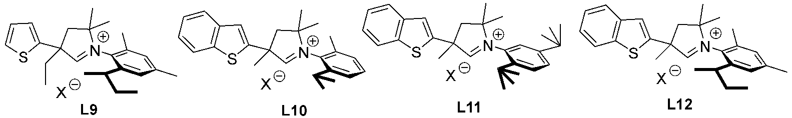

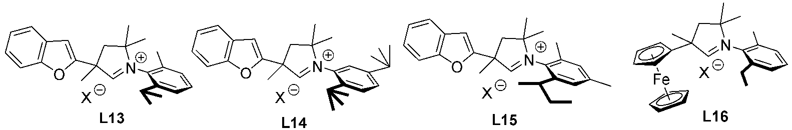

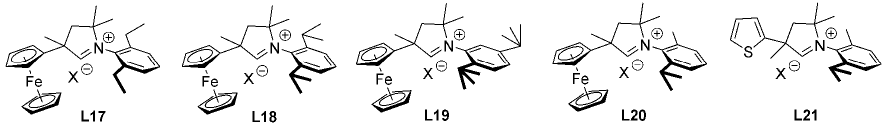

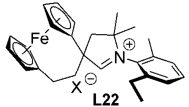

3. Rutheniumkomplex der Formel **1-Ru** 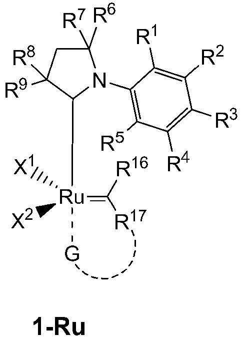 wobei:
X¹ und X² unabhängig einen anionischen Liganden, ausgewählt aus der Gruppe umfassend ein Halogenanion, eine -CN-, -SCN-, -OR^{a}-, -SR^{a}-, -O(C=O)R^{a}-, - O(SO₂)R^{a}- und -OSi(R^{a})₃-Gruppe bezeichnen, wobei R^{a} C₁-C₁₂-Alkyl, C₃-C₁₂-Cycloalkyl, C₂-C₁₂-Alkenyl oder C₅-C₂₀-Aryl, das optional mit mindestens einem C₁-C₁₂-Alkyl-, C₁-C₁₂-Perfluoralkyl-, C₁-C₁₂-Alkoxyl-, C₅-C₂₄-Aryloxyl-, C₅-C₂₀-heteroaryloxyl oder einem Halogenatom substituiert ist, bezeichnet;
R¹, R², R³, R⁴ und R⁵ unabhängig ein Wasserstoffatom, eine C₁-C₁₂-Alkylgruppe, eine C₃-C₁₂-Cycloalkylgruppe, eine C₅-C₂₀-Arylgruppe oder eine C₅-C₂₀-Heteroarylgruppe, eine C₅-C₂₅-Aralkylgruppe bezeichnen, die unabhängig mit einem und/oder mehreren Substituenten substituiert sein können, ausgewählt aus der Gruppe umfassend ein Wasserstoffatom, ein Halogenatom, eine C₁-C₁₂-Alkylgruppe, eine C₁-C₁₂-Perfluoralkylgruppe, eine C₅-C₂₀-Arylgruppe, eine C₅-C₂₀-Perfluorarylgruppe, eine C₅-C₂₀-Heteroarylgruppe, eine C₁-C₁₂-Alkoxylgruppe, eine C₅-C₂₄-Aryloxylgruppe, eine C₅-C₂₀-Heteroaryloxylgruppe, eine Sulfidgruppe (-SR"), eine Aminogruppe (-NR"₂), in denen die Gruppe R" unabhängig ein Wasserstoffatom, C₁-C₅-Alkyl, C₆-C₂₄-Aryl, C₇-C₂₄-Aralkyl bezeichnet,
alternativ R¹, R², R³, R⁴ und R⁵ miteinander verbunden sind, um einen C₅-C₂₅-Ring zu bilden;
jeder Substituent R⁶, R⁷ und R⁸ ein Wasserstoffatom, ein Halogenatom, eine C₁-C₁₂-Alkylgruppe oder eine C₅-C₂₀-Arylgruppe bezeichnen, die unabhängig mit einem und/oder mehreren Substituenten substituiert sein können, ausgewählt aus der Gruppe umfassend ein Wasserstoffatom, eine C₁-C₁₂-Alkylgruppe, eine C₁-C₁₂-Perfluoralkylgruppe, eine C₅-C₂₀-Arylgruppe, eine C₅-C₂₀-Perfluorarylgruppe, eine C₅-C₂₀-Heteroarylgruppe, eine C₁-C₁₂-Alkoxylgruppe, eine C₅-C₂₄-Aryloxylgruppe, eine C₅-C₂₀-Heteroaryloxylgruppe oder ein Halogenatom, eine Sulfidgruppe (-SR"), eine Aminogruppe (-NR"₂), in denen die Gruppe R" unabhängig ein Wasserstoffatom, C₁-C₅-Alkyl, C₆-C₂₄-Aryl oder C₇-C₂₄-Aralkyl bezeichnet;
Substituent R⁹ eine substituierte oder unsubstituierte heterocyclische Gruppe und/oder eine metallorganische Komplexgruppe, ausgewählt aus Thiophen, Benzothiophen, Furan, Benzofuran oder Ferrocen bezeichnet, die unabhängig mit einem und/oder mehreren Substituenten substituiert sein können, ausgewählt aus der Gruppe umfassend ein Wasserstoffatom, ein Halogenatom, eine C₁-C₁₂-Alkylgruppe, eine C₁-C₁₂-Perfluoralkylgruppe, eine C₅-C₂₀-Arylgruppe, eine C₅-C₂₀-Perfluorarylgruppe, eine C₅-C₂₀-Heteroarylgruppe, eine C₁-C₁₂-Alkoxylgruppe, eine C₅-C₂₄-Aryloxylgruppe, eine C₅-C₂₀-Heteroaryloxylgruppe, eine Sulfidgruppe (-SR"), eine Aminogruppe (-NR"₂), in denen die Gruppe R" unabhängig ein Wasserstoffatom, C₁-C₅-Alkyl, C₆-C₂₄-Aryl, C₇-C₂₄-Aralkyl bezeichnet,
R¹⁶ und R¹⁷ unabhängig ein Wasserstoffatom, ein Halogenatom, C₁-C₂₅-Alkyl, C₃-C₂₅-Cycloalkyl, C₁-C₁₂-Perfluoralkyl, C₂-C₂₅-Alken, C₂-C₂₅-Alkenyl, C₃-C₂₅-Cycloalkenyl, C₂-C₂₅-Alkinyl, C₃-C₂₅-Cycloalkinyl, C₁-C₂₅-Alkoxyl, C₅-C₂₅-Aryl, C₅-C₂₅-Aryloxyl, C₆-C₂₅-Arylalkyl, C₅-C₂₅-Heteroaryl, C₅-C₂₅-Heteroaryloxy, C₅-C₂₅-Perfluoraryl, einen 3-12-gliedrigen Heterocyclus, der ein Schwefel-, Sauerstoff-, Stickstoff-, Selen- oder Phosphoratom umfasst, bezeichnen;
wobei die Substituenten R¹⁶ und R¹⁷ miteinander verbunden sein können um einen Ring zu bilden, der ausgewählt ist aus der Gruppe umfassend C₃-C₂₅-Cycloalkyl, C₃-C₂₅-Cycloalkenyl, C₃-C₂₅-Cycloalkinyl, C₅-C₂₅-Aryl, C₅-C₂₅-Heteroaryl, C₅-C₂₅-Perfluoraryl, einen 3-12-gliedrigen Heterocyclus, der ein Schwefel-, Sauerstoff-, Stickstoff-, Selen- oder Phosphoratom umfasst, das unabhängig durch einen oder mehrere Substituenten substituiert sein kann, ausgewählt aus der Gruppe umfassend ein Wasserstoffatom, ein Halogenatom, C₁-C₂₅-Alkyl, C₃-C₂₅-Cycloalkyl, C₁-C₁₂-Perfluoralkyl, C₂-C₂₅-Alken, C₂-C₂₅-Alkenyl, C₃-C₂₅-Cycloalkenyl, C₂-C₂₅-Alkinyl, C₃-C₂₅-Cycloalkinyl, C₁-C₂₅-Alkoxyl, C₅-C₂₅-Aryl, C₅-C₂₅-Aryloxyl, C₆-C₂₅-Arylalkyl, C₅-C₂₅-Heteroaryl, C₅-C₂₅-Heteroaryloxy, C₅-C₂₅-Perfluoraryl, einen 3-12-gliedrigen Heterocyclus;
G ausgewählt ist aus zum Beispiel
- einem Liganden der Formel **CAAC-1** 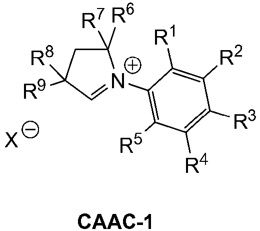
in der X und die Substituenten R¹ bis R⁹ die oben definierten Bedeutungen aufweisen
entweder
- Heteroatom 1
ausgewählt aus der Gruppe umfassend ein Sauerstoffatom, ein Schwefelatom, ein Selenatom, substituiert durch eine Gruppe ausgewählt aus zum Beispiel einem Wasserstoffatom, einem Fluoratom, einem Sauerstoffatom, C₁-C₂₅-Alkyl, C₁-C₂₅-Perfluoralkyl, C₃-C₂₅-Cycloalkyl, C₅-C₂₀-Alkoxyl, C₅-C₂₀-Aryl, C₅-C₂₀-Perfluoraryl, C₇-C₂₀-Aralkyl, C₅-C₂₄-Aryloxy, C₂-C₁₂-Alkenyl, C₆-C₂₀-Heteroaryl oder C₅-C₂₄-Heteroaryloxy, einem 3-12-gliedrigen Heterocyclus, optional substituiert mit einer Acylgruppe (-COR'), einer Cyanogruppe (-CN), einer Carboxylgruppe (-COOH), einer Estergruppe (-COOR'), einer Estergruppe (-CH₂COOR'), einer Estergruppe (-CHR'COOR'), einer Estergruppe (-C(R')₂COOR'), einer Amidgruppe (-CONR'₂), einem Amid vom Weinreb-Typ (CON(R')(OR')), einer Sulfongruppe (-SO₂R'), einer Formylgruppe (-CHO), einer Sulfonamidgruppe
(-SO₂NR'₂), einer Ketongruppe (-COR'), eine Thioamidgruppe (-CSNR'₂), einem Thioketon (-CSR'), einer Thionoestergruppe (-CSOR'), einer Thioestergruppe (-COSR'), einer Dithioestergruppe (-CS2R'), in der die Gruppe R' unabhängig C₁-C₂₅-Alkyl, C₁-C₂₅-Perfluoralkyl, C₃-C₂₅-Cycloalkyl, C₅-C₂₀-Alkoxyl, C₅-C₂₀-Aryl, C₅-C₂₀-Perfluoraryl, C₇-C₂₀-Aralkyl, C₅-C₂₄-Aryloxyl, C₂-C₁₂-Alkenyl, C₆-C₂₀-Heteroaryl, C₅-C₂₄-Heteroaryloxy bezeichnet, und dann die gestrichelte Linie eine direkte Bindung zwischen einem Heteroatom und dem Substituenten R¹⁷ oder eine Bindung zwischen dem Substituenten R¹⁷ und einem Heteroatom über eine Methylenbrücke -CH₂-, -CHR'-, oder -CR'₂-, bezeichnet, wobei der Substituent R¹⁴ C₅-C₁₅-Aryl ist, optional substituiert mit 1-4 Substituenten, unabhängig ausgewählt aus einer Gruppe umfassend ein Wasserstoffatom, ein Halogenatom, C₁-C₂₅-Alkyl, C₃-C₂₅-Cycloalkyl, C₂-C₂₅-Alkenyl, C₃-C₂₅-Cycloalkenyl, C₂-C₂₅-Alkinyl, C₃-C₂₅-Cycloalkinyl, C₁-C₂₅-Perfluoralkyl, C₅-C₂₀-Alkoxyl, C₅-C₂₀-Aryl, C₅-C₂₀-Perfluoraryl, C₇-C₂₀-Aralkyl, C₅-C₂₄-Aryloxyl, C₆-C₂₀-Heteroaryl oder C₅-C₂₄-Heteroaryloxyl, einen 3-12-gliedrigen Heterocyclus, eine Alkoxylgruppe (-OR"), eine Sulfidgruppe (-SR"), eine Sulfoxidgruppe (-S(O)R"), eine Sulfoniumgruppe (-S⁺R"₂), eine Sulfongruppe (-SO₂R"), eine Sulfonamidgruppe (-SO₂NR"₂), eine Aminogruppe (-NR"₂), eine Ammoniumgruppe (-N⁺R"₃), eine Nitrogruppe (-NO₂), eine Cyanogruppe (-CN), eine Phosphonatgruppe (-P(O)(OR")₂), eine Phosphinatgruppe (-P(O)R"(OR")), eine Phosphonitgruppe (-P(OR")₂), eine Phosphingruppe (-PR"₂), eine Phosphinoxidgruppe (-P(O)R"₂), eine Phosphoniumgruppe (-P⁺R"₃), eine Carboxylgruppe (-COOH), eine Estergruppe (-COOR"), eine Amidgruppe (-CONR"₂), eine Amidgruppe (-NR"C(O)R"), eine Formylgruppe (-CHO), eine Ketongruppe (-COR"), eine Thioamidgruppe (-CSNR"₂), eine Thioketongruppe (-CSR"), eine Thionoestergruppe (-CSOR"), eine Thioestergruppe (-COSR"), eine Dithioestergruppe (-CS₂R"), in der die R"-Gruppe C₁-C₅-Alkyl, C₁-C₅-Perfluoralkyl, C₆-C₂₄-Aryl, C₇-C₂₄-Aralkyl, C₅-C₂₄-Perfluoraryl, oder
- Heteroatom 2
ausgewählt aus einer Gruppe umfassend ein Stickstoffatom oder ein Phosphoratom, substituiert mit der Gruppe ausgewählt aus zum Beispiel einem Wasserstoffatom, Methyliden, optional substituiert mit dem Substituenten R', C₁-C₂₅-Alkyl, C₁-C₂₅-Perfluoralkyl, C₃-C₂₅-Cycloalkyl, C₅-C₂₀-Alkoxyl, C₅-C₂₀-Aryl, C₅-C₂₀-Perfluoraryl, C₇-C₂₀-Aralkyl, C₅-C₂₄-Aryloxy, C₂-C₁₂-Alkenyl, C₆-C₂₀-Heteroaryl oder C₅-C₂₄-Heteroaryloxy, einem 3-12-gliedrigen Heterocyclus, einer Acylgruppe (-COR'), einer Estergruppe (-COOR'), einer tert-Butylbutyloxycarbonylgruppe (t-Boc) oder einer 9-Fluorenylmethoxycarbonylgruppe (Fmoc), einer Carbamingruppe (-CONR'₂), eine Sulfongruppe (-SO₂R'), eine Formylgruppe (-CHO), in denen die Gruppe R' C₁-C₂₅-Alkyl, C₁-C₂₅-Perfluoralkyl, C₃-C₂₅-Cycloalkyl, C₅-C₂₀-Alkoxyl, C₅-C₂₀-Aryl, C₅-C₂₀-Perfluoraryl, C₇-C₂₀-Aralkyl, C₅-C₂₄-Aryloxy, C₂-C₁₂-Alkenyl, C₆-C₂₀-Heteroaryl oder C₅-C₂₄-Heteroaryloxy darstellt, optional substituiert mit einer Acylgruppe (-COR'), einer Cyanogruppe (-CN), einer Carboxylgruppe (-COOH), einer Estergruppe (-COOR'), einer Estergruppe (-CH₂COOR'), einer Estergruppe (-CHR'COOR'), einer Estergruppe (-C(R')₂COOR'), einer Amidgruppe (-CONR'₂), einer Sulfongruppe
(-SO₂R'), einer Formylgruppe (-CHO), einer Sulfonamidgruppe (-SO₂NR'₂), einer Ketongruppe (-COR'), einer Thioamidgruppe (-CSNR'₂), einer Thioketongruppe (-CSR'), einer Thionoestergruppe (-CSOR'), einer Thioestergruppe (-COSR'), einer Dithioestergruppe (-CS₂R'), in denen die Gruppe R' C₁-C₂₅-Alkyl, C₁-C₂₅-Perfluoralkyl, C₃-C₂₅-Cycloalkyl, C₅-C₂₀-Alkoxyl, C₅-C₂₀-Aryl, C₅-C₂₀-Perfluoraryl, C₇-C₂₀-Aralkyl, C₅-C₂₄-Aryloxyl, C₂-C₁₂-Alkenyl, C₆-C₂₀-Heteroaryl oder C₅-C₂₄-Heteroaryloxy bezeichnen, und dann die gestrichelte Linie eine direkte Bindung zwischen dem Heteroatom und dem Substituenten R¹⁴ bezeichnet oder sie eine Bindung zwischen dem Substituenten R¹⁷ und einem Heteroatom über eine Methylenbrücke (CH₂)-, -(CHR')-, oder -(CR'₂)- bezeichnet; wobei der Substituent R¹⁷ C₅-C₁₅-Aryl ist, optional substituiert mit 1-4 Substituenten, die unabhängig aus der Gruppe ausgewählt sind umfassend ein Wasserstoffatom, ein Halogenatom, C₁-C₂₅-Alkyl, C₃-C₂₅-Cycloalkyl, C₂-C₂₅-Alkenyl, C₃-C₂₅-Cycloalkenyl, C₂-C₂₅-Alkinyl, C₃-C₂₅-Cycloalkinyl, C₁-C₂₅-Perfluoralkyl, C₅-C₂₀-Alkoxyl, C₅-C₂₀-Aryl, C₅-C₂₀-Perfluoraryl, C₇-C₂₀-Aralkyl, C₅-C₂₄-Aryloxy, C₆-C₂₀-Heteroaryl oder C₅-C₂₄-Heteroaryloxy, einen 3-12-gliedrigen Heterocyclus, eine Alkoxylgruppe (-OR"), eine Sulfidgruppe (-SR"), eine Sulfoxidgruppe (-S(O)R"), eine Sulfoniumgruppe (-S⁺R"₂), eine Sulfongruppe (-SO₂R"), eine Sulfonamidgruppe (-SO₂NR"₂), eine Aminogruppe (-NR"₂), eine Ammoniumgruppe (-N⁺R"₃), eine Nitrogruppe (-NO₂), eine Cyanogruppe (-CN), eine Phosphonatgruppe (-P(O)(OR")₂), eine Phosphinatgruppe (-P(O)R"(OR")), eine Phosphonitgruppe (-P(OR")₂), eine Phosphingruppe (-PR"₂), eine Phosphinoxidgruppe (-P(O)R"₂), eine Phosphoniumgruppe (-P⁺R"₃), eine Carboxylgruppe (-COOH), eine Estergruppe (-COOR"), eine Amidgruppe (-CONR"₂), eine Amidgruppe (-NR"C(O)R"), eine Formylgruppe (-CHO), eine Ketongruppe (-COR"), eine Thioamidgruppe (-CSNR"₂), eine Thioketongruppe (-CSR"), eine Thionoestergruppe (-CSOR"), eine Thioestergruppe (COSR"), eine Dithioestergruppe (-CS₂R)", in denen die Gruppe R" C₁-C₅-Alkyl, C₁-C₅-Perfluoralkyl, C₆-C₂₄-Aryl, C₇-C₂₄-Aralkyl, C₅-C₂₄-Perfluoraryl bezeichnet, oder
- Heteroatom 3
ausgewählt aus einer Gruppe umfassend ein Halogenatom und dann bezeichnet die gestrichelte Linie eine direkte Bindung zwischen dem Heteroatom und dem R¹⁷-Substituenten, wobei der R¹⁷-Substituent C₅-C₁₅-Aryl oder C₅-C₂₅-Polyaryl ist, optional substituiert mit 1-4 Substituenten, die unabhängig ausgewählt sind aus einer Gruppe umfassend ein Wasserstoffatom, ein Halogenatom, C₁-C₂₅-Alkyl, C₃-C₂₅-Cycloalkyl, C₂-C₂₅-Alkenyl, C₃-C₂₅-Cycloalkenyl, C₂-C₂₅-Alkinyl, C₃-C₂₅-Cycloalkinyl, C₁-C₂₅-Perfluoralkyl, C₅-C₂₀-Alkoxyl, C₅-C₂₀-Aryl, C₅-C₂₀-Perfluoraryl, C₇-C₂₀-Aralkyl, C₅-C₂₄-Aryloxy, C₆-C₂₀-Heteroaryl oder C₅-C₂₄-Heteroaryloxy, einen 3-12-gliedrigen Heterocyclus, eine Alkoxygruppe (-OR"), eine Sulfidgruppe (-SR"), eine Sulfoxidgruppe (-S(O)R"), eine Sulfoniumgruppe (-S⁺R"₂), eine Sulfongruppe (-SO₂R"), eine Sulfonamidgruppe (-SO₂NR"₂), eine Aminogruppe (-NR"₂), eine Ammoniumgruppe (-N⁺R"₃), eine Nitrogruppe (-NO₂),
eine Cyanogruppe (-CN), eine Phosphonatgruppe (-P(O)(OR")₂), eine Phosphinatgruppe (-P(O)R"(OR")), eine Phosphonitgruppe (-P(OR")₂), eine Phosphingruppe (-PR"₂), eine Phosphinoxidgruppe (-P(O)R"₂), eine Phosphoniumgruppe (-P⁺R"₃), eine Carboxylgruppe (-COOH), eine Estergruppe (-COOR"), eine Amidgruppe (-CONR"₂), eine Amidgruppe (-NR"C(O)R"), eine Formylgruppe (-CHO), eine Ketongruppe (-COR"), eine Thioamidgruppe (-CSNR"₂), eine Thioketongruppe (-CSR"), eine Thionoestergruppe (-CSOR"), eine Thioestergruppe (-COSR"), eine Dithioestergruppe (-CS₂R"), in denen die Gruppe R" C₁-C₅-Alkyl, C₁-C₅-Perfluoralkyl, C₆-C₂₄-Aryl, C₇-C₂₄-Aralkyl oder C₅-C₂₄-Perfluoraryl bezeichnet.

4. Rutheniumkomplex nach Anspruch 3, dargestellt durch die Formel **1a-Ru** 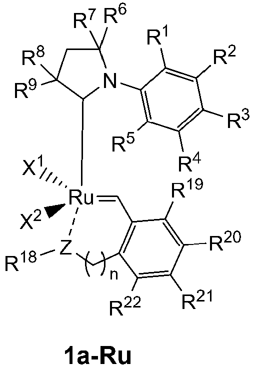
in dem X¹ und X² sowie die Substituenten R¹, R², R³, R⁴, R⁵, R⁶, R⁷, R⁸ und R⁹ die oben definierten Bedeutungen aufweisen
"n" 1 oder 0 bedeutet
Z ausgewählt ist aus einer Gruppe umfassend Halogenatome, ein O-Atom, ein S-Atom, ein Se-Atom oder eine NR"'-Gruppe, in der R‴ Methyliden, C₁-C₂₅-Alkyl, C₁-C₂₅-Perfluoralkyl, C₃-C₂₅-Cycloalkyl, C₅-C₂₀-Alkoxyl, C₅-C₂₀-Aryl, C₅-C₂₀-Perfluoraryl, C₇-C₂₀-Aralkyl, C₅-C₂₄-Aryloxy, C₂-C₁₂-Alkenyl, C₆-C₂₀-Heteroaryl oder C₅-C₂₄-Heteroaryloxy, einen 3-12-gliedrigen Heterocyclus, eine Acylgruppe (-COR'), eine Estergruppe (-COOR'), eine tert-Butyloxycarbonylgruppe (t-Boc) oder eine 9-Fluorenylmethoxycarbonylgruppe (Fmoc), eine Carbamingruppe (-CONR'₂), eine Sulfongruppe (-SO₂R'), eine Formylgruppe (-CHO) bezeichnet, in denen die R'-Gruppe C₁-C₂₅-Alkyl, C₁-C₂₅-Perfluoralkyl, C₃-C₂₅-Cycloalkyl, C₅-C₂₀-Alkoxyl, C₅-C₂₀-Aryl, C₅-C₂₀-Perfluoraryl, C₇-C₂₀-Aralkyl, C₅-C₂₄-Aryloxyl, C₂-C₁₂-Alkenyl, C₆-C₂₀-Heteroaryl oder C₅-C₂₄-Heteroaryloxyl,
oder ein Halogenatom bezeichnet, wobei wenn Z ein Halogenatom bezeichnet, R¹⁸ fehlt;
R¹⁸ unabhängig ein Wasserstoffatom, eine C₁-C₂₅-Alkylgruppe, eine C₁-C₂₅-Cycloalkylgruppe, eine C₅-C₂₀-Alkoxygruppe, eine C₅-C₂₀-Arylgruppe, eine C₅-C₂₄-Aryloxygruppe, eine -COOR‴-Gruppe, eine -CH₂COOR‴-Gruppe, eine -CONR"'₂-Gruppe, eine -CH₂CONR‴₂-Gruppe, eine -COR'"-Gruppe, eine -CH₂COR"'-Gruppe, eine -CON(OR"')(R"')-Gruppe, eine -CH₂CON(OR‴)(R‴)-Gruppe oder ein Halogenatom bedeutet, wobei R‴ C₁-C₁₂-Alkyl, C₃-C₁₂-Cycloalkyl, C₂-C₁₂-Alkenyl, C₆-C₂₀-Aryl bezeichnet, die optional mit mindestens einem C₁-C₁₂-Alkyl, C₁-C₁₂-Perfluoralkyl, C₁-C₁₂-Alkoxyl, C₆-C₂₄-Aryloxy oder einem Halogenatom substituiert sind;
R¹⁹, R²⁰, R²¹ und R²² unabhängig ein Wasserstoffatom, ein Halogenatom, eine C₁-C₂₅-Alkylgruppe, eine C₂-C₂₅-Alkenylgruppe, eine C₅-C₂₅-Arylgruppe, eine Alkoxygruppe (-OR"), eine Sulfidgruppe (-SR"), eine Sulfoxidgruppe (-S(O)R"), eine Sulfoniumgruppe (-S⁺R"₂), eine Sulfongruppe (-SO₂R"), eine Sulfonamidgruppe (-SO₂NR"₂), eine Aminogruppe (-NR"₂), eine Ammoniumgruppe (-N⁺R"₃), eine Nitrogruppe (-NO₂), eine Cyanogruppe (-CN), eine Phosphonatgruppe (-P(O)(OR")₂), eine Phosphinatgruppe (-P(O)R"(OR")), eine Phosphonitgruppe (-P(OR")₂), eine Phosphingruppe (-PR"₂), eine Phosphinoxidgruppe (-P(O)R"₂), eine Phosphoniumgruppe (-P⁺R"₃), eine Carboxylgruppe (-COOH), eine Estergruppe (-COOR"), eine Amidgruppe (-CONR"₂), eine Amidgruppe (-NR"C(O)R"), eine Formylgruppe (-CHO), eine Ketongruppe (-COR"), eine Thioamidgruppe (-CSNR"₂), eine Thioketongruppe (-CSR"), eine Thionoestergruppe (-CSOR"), eine Thioestergruppe (-COSR"), eine Dithioestergruppe (-CS₂R") bezeichnen, wobei die R"-Gruppe C₁-C₅-Alkyl, C₁-C₅-Perfluoralkyl, C₆-C₂₄-Aryl, C₇-C₂₄-Aralkyl, C₅-C₂₄-Perfluoraryl bezeichnet, wobei die Substituenten R¹⁶, R¹⁷, R¹⁸ und R¹⁹ verbunden sein können und demnach ein substituiertes oder unsubstituiertes C₄-C₁₀-cyclisches oder C₄-C₁₂-polycyclisches System bilden.

5. Rutheniumkomplex nach Anspruch 3 oder 4 der Formel **1b-Ru** 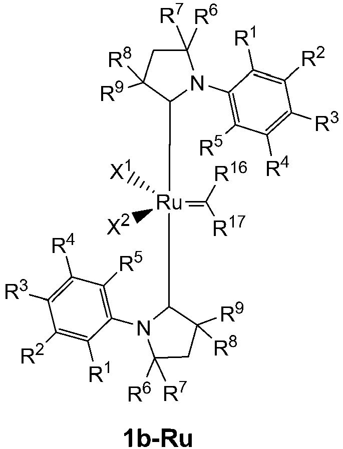
wobei X¹ und X² und die Substituenten R¹, R², R³, R⁴, R⁵, R⁶, R⁷, R⁸, R⁹ die oben definierten Bedeutungen aufweisen
R¹⁶ und R¹⁷ unabhängig ein Wasserstoffatom, ein Halogenatom, C₁-C₂₅-Alkyl, optional substituiert; C₃-C₂₅-Cycloalkyl, optional substituiert; C₁-C₁₂-Perfluoralkyl, optional substituiert; C₂-C₂₅-Alken, optional substituiert; C₂-C₂₅-Alkenyl, optional substituiert; C₃-C₂₅-Cycloalkenyl, optional substituiert; C₂-C₂₅-Alkinyl, optional substituiert; C₃-C₂₅-Cycloalkyl, optional substituiert; C₁-C₂₅-Alkoxyl, optional substituiert; C₅-C₂₅-Aryl, optional substituiert; C₅-C₂₅-Aryloxyl, optional substituiert; C₆-C₂₅-Arylalkyl, optional substituiert; C₅-C₂₅-Heteroaryl, optional substituiert; C₅-C₂₅-Heteroaryloxy, optional substituiert; C₅-C₂₅-Perfluoraryl, optional substituiert; einen 3-12-gliedrigen Heterocyclus, der ein Schwefel-, Sauerstoff-, Stickstoff-, Selen- oder Phosphoratom umfasst, optional substituiert; bezeichnen,
wobei die Substituenten R¹⁶ und R¹⁷ verbunden sein können und einen Ring bilden, ausgewählt aus einer Gruppe umfassend C₃-C₂₅-Cycloalkyl, C₃-C₂₅-Cycloalkenyl, C₃-C₂₅-Cycloalkinyl, C₅-C₂₅-Aryl, C₅-C₂₅-Heteroaryl, C₅-C₂₅-Perfluoraryl, einen 3-12-gliedrigen Heterocyclus, der ein Schwefel-, Sauerstoff-, Stickstoff-, Selen- oder Phosphoratom enthält, das unabhängig mit einem oder mehreren Substituenten substituiert sein kann, ausgewählt aus einer Gruppe umfassend ein Wasserstoffatom, ein Halogenatom, C₁-C₂₅-Alkyl, C₃-C₂₅-Cycloalkyl, C₁-C₁₂-Perfluoralkyl, C₂-C₂₅-Alken, C₂-C₂₅-Alkenyl, C₃-C₂₅-Cycloalkenyl, C₂-C₂₅-Alkinyl, C₃-C₂₅-Cycloalkinyl, C₁-C₂₅-Alkoxyl, C₅-C₂₅-Aryl, C₅-C₂₅-Aryloxyl, C₆-C₂₅-Arylalkyl, C₅-C₂₅-Heteroaryl, C₅-C₂₅-Heteroaryloxy, C₅-C₂₅-Perfluoraryl, einen 3-12-gliedrigen Heterocyclus.

6. Rutheniumkomplex nach einem der Ansprüche 3-4, der aus den durch die Formeln **Ru1a, Ru2a, Ru3a, Ru4a, Ru5a, Ru6a, Ru7a, Ru8a, Ru9a, Ru10a, Ru11a, Ru11a, Ru12a, Ru13a, Ru14a, Ru15a, Ru16a, Ru17a, Ru18a, Ru19a, Ru20a, Ru21a, Ru22a** dargestellten Komplexen ausgewählt ist:

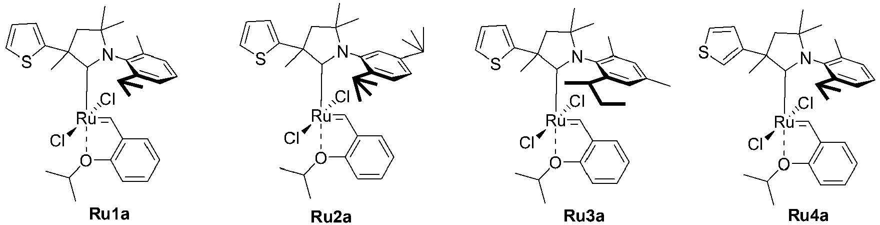

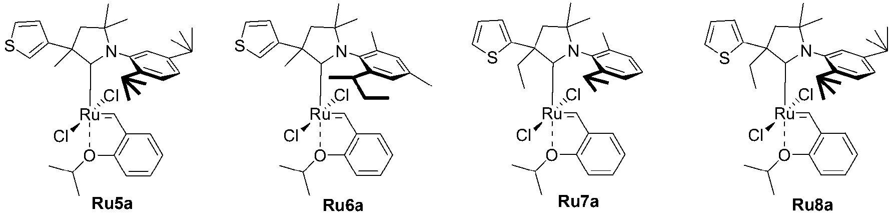

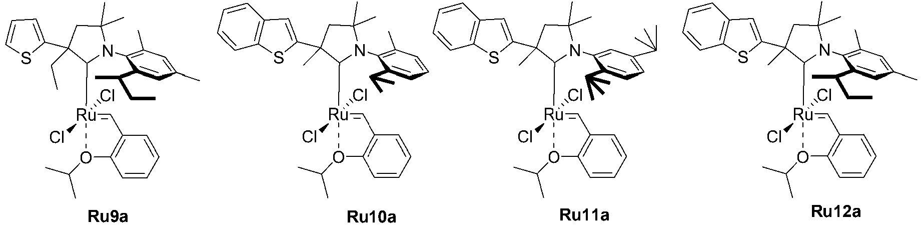

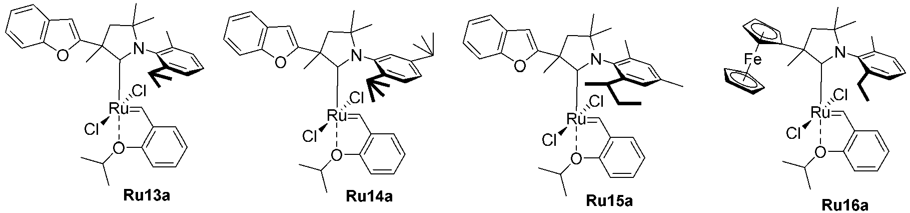

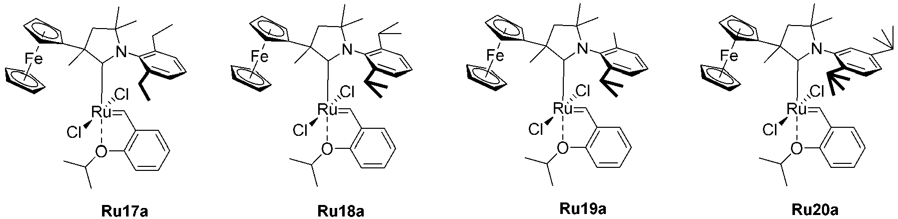

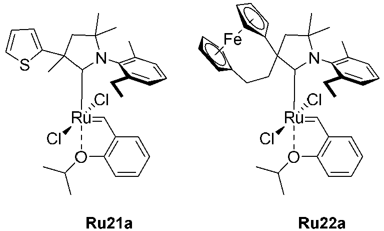

7. Verfahren zur Synthese des Rutheniumkomplexes der Formel **1a-Ru** 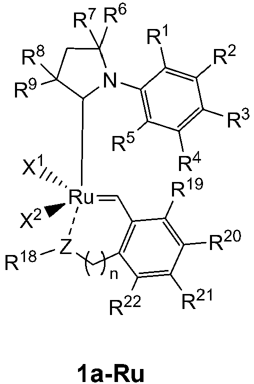
wie in Anspruch 4 definiert, **dadurch gekennzeichnet, dass** der Alkyliden-Ruthenium-Komplex der Formel 10 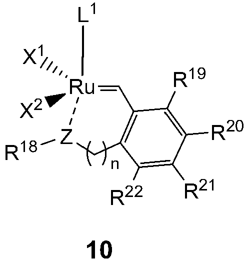
wobei:
L1 einen neutralen Liganden bezeichnet, der ausgewählt ist aus der Gruppe umfassend Pyridin oder substituiertes Pyridin, P(R')₃, P(OR')₃, O(R')₂, N(R')₃, wobei jedes R' unabhängig C₁-C₁₂-Alkyl, C₃-C₁₂-Cycloalkyl, C₅-C₂₀-Aryl, C₇-C₂₄-Aralkyl, C₅-C₂₄-Perfluoraryl, ein 5-12-gliedriges Heteroaryl bezeichnet;
N, Z, X¹, X² und die Substituenten R¹⁸, R¹⁹, R²⁰, R²¹ und R²² die oben definierten Bedeutungen aufweisen
mit dem Carben der Formel 8 reagiert 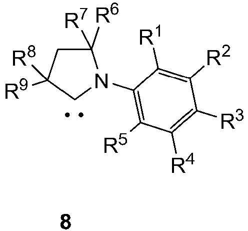
wobei die Substituenten R¹ bis R⁹ die oben definierten Bedeutungen aufweisen.

8. Verwendung der Verbindung der Formel **1-Ru,** wie in einem der Ansprüche 3-6 definiert, als Vorkatalysator und/oder Katalysator bei Olefin-Metathese-Reaktionen, insbesondere bei Ringverschluss-Metathese (RCM), Kreuzmetathese (CM), Homometathese (Kreuzmetathese zwischen zwei Molekülen desselben Olefins), Ethenolyse, Isomerisierung, in einer diastereoselektiven Ringumlagerungs-Metathese (DRRM), Alken-Alkin (Enin)-Metathese oder ROMP- oder ADMET-Polymerisationsreaktion.

9. Verwendung nach Anspruch 8, wobei die Reaktion in einem organischen Lösungsmittel wie Toluol, Mesitylen, Hexan, Cyclohexan, Ethylacetat, Methylacetat, Methylcarbonat, Ethylcarbonat, tert-Butylmethylether, Cyclopentylmethylether, Diethylether, THF, 2-Me-THF, 4-Me-THP, Dioxan, DME, PAO, PEG, Paraffin oder Estern gesättigter Fettsäuren durchgeführt wird.

10. Verwendung nach Anspruch 8, wobei die Reaktion in einem lösungsmittelfreien System durchgeführt wird.

11. Verwendung nach einem der vorstehenden Ansprüche, wobei die Reaktion bei einer Temperatur zwischen 20 °C und 200 °C durchgeführt wird.

12. Verwendung nach einem der vorstehenden Ansprüche, wobei die Reaktion über einen Zeitraum zwischen 5 Minuten und 48 Stunden durchgeführt wird.

13. Verwendung nach einem der vorstehenden Ansprüche, wobei die Verbindung **1-Ru** in einer Menge von nicht mehr als 10 Mol-%, bevorzugt in einer Menge von nicht mehr als 0,1 Mol-%, zugegeben wird.

14. Verwendung nach einem der vorstehenden Ansprüche, wobei die Verbindung **1-Ru** dem Reaktionsgemisch in festen Portionen und/oder kontinuierlich unter Verwenden einer Pumpe als Lösung in einem organischen Lösungsmittel zugegeben wird.

15. Verwendung nach einem der vorstehenden Ansprüche, wobei das gasförmige Reaktionsnebenprodukt, ausgewählt aus Ethylen, Propylen und Butylen, unter Verwenden einer Inertgas-Barbotage oder mittels Vakuum aktiv aus dem Reaktionsgemisch entfernt wird.

## Revendications

1. Précurseur de carbènes d'alkylamine cycliques (CAAC) de formule CAAC-1 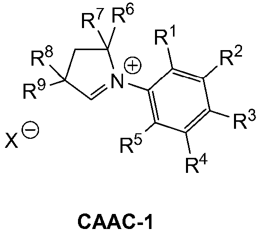
dans laquelle X représente un anion choisi parmi le groupe comprenant un atome d'halogène, BF₄⁻, PF₆⁻, ClO₄⁻, CF₃SO₂O⁻;
R¹, R², R³, R⁴ et R⁵ désignent chacun indépendamment un atome d'hydrogène, un groupe alkyle en C₁-C₁₂, un groupe cycloalkyle en C₃-C₁₂, un groupe aryle en C₅-C₂₀ ou un groupe hétéroaryle en C₅-C₂₀, un groupe aralkyle en C₅-C₂₅ aralkyle, qui peut être substitué indépendamment par un et/ou plusieurs substituants choisis dans le groupe comprenant un atome d'hydrogène, un atome d'halogène, un groupe alkyle en C₁-C₁₂, un groupe perfluoroalkyle en C₁-C₁₂, un groupe aryle en C₅-C₂₀ groupe aryle, un groupe C₅-C₂₀ perfluoroaryle, un groupe hétéroaryle en C₅-C₂₀, un groupe alcoxyle en C₁ -C₁₂, un groupe aryloxyle en C₅-C₂₄, un groupe hétéroaryloxy en C₅-C₂₀, un groupe sulfure (-SR"), un groupe amino (-NR"₂), dans lesquels le groupe R" désigne indépendamment un atome d'hydrogène, un groupe alkyle en C₁-C₅, un groupe aryle en C₆-C₂₄, un groupe aralkyle en C₇-C₂₄, alternativement R¹, R², R³, R⁴ et R⁵ sont reliés pour former un cycle en C₅-C₂₅ ;
chaque substituant R⁶, R⁷ et R⁸ représente un atome d'hydrogène, un atome d'halogène, un groupe alkyle en C₁-C₁₂ ou un groupe aryle en C₅-C₂₀, qui peut être substitué indépendamment par un ou plusieurs substituants choisis parmi le groupe contenant de l'hydrogène, un groupe alkyle en C₁- C₁₂, un groupe perfluoroalkyle en C₁-C₁₂, un groupe aryle en C₅-C₂₀, un groupe perfluoroaryle en C₅-C₂₀, un groupe hétéroaryle en C₅ -C₂₀, un groupe alcoxyle en C₁ -C₁₂, un groupe aryloxyle en C₅-C₂₄, un groupe hétéroaryloxy en C₅-C₂₀ ou un atome d'halogène, un groupe sulfure (-SR"), un groupe amino (-NR"₂), dans lequel le groupe R" désigne indépendamment un atome d'hydrogène, un alkyle en C₁-C₅, un aryle en C₆-C₂₄, un aralkyle en C₇ -C₂₄ ;
Le substituant R⁹ désigne un groupe hétérocyclique substitué ou non substitué ou un groupe complexe organométallique choisi parmi le thiophène, le benzothiophène, le furane, le benzofurane ou le ferrocène, qui peut être substitué indépendamment par un ou plusieurs substituants choisis parmi le groupe comprenant un atome d'hydrogène, un atome d'halogène, un groupe alkyle en C₁-C₁₂, un groupe perfluoroalkyle en C₁-C₁₂, un groupe aryle en C₅-C₂₀, un groupe perfluoroaryle en C₅-C₂₀, un groupe C₅-C₂₀ hétéroaryle, un groupe alcoxyle en C₁-C₁₂, un groupe aryloxyle en C₅-C₂₄, un groupe hétéroaryloxy en C₅-C₂₀, un groupe sulfure (-SR"), un groupe amino (-NR"₂), où le groupe R" désigne indépendamment un atome d'hydrogène, un alkyle en C₁-C₅, un aryle en C₆-C₂₄, un aralkyle en C₇ -C₂₄ .

2. Précurseur de la formule **CAAC-1** selon la revendication 1, ayant la structure représentée par la formule **L1, L2, L3, L4, L5, L6, L7, L8, L9, L10, L11, L12, L13, L14, L15, L16, L17, L18, L19, L20, L21 ou L22** :

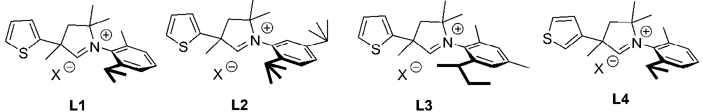

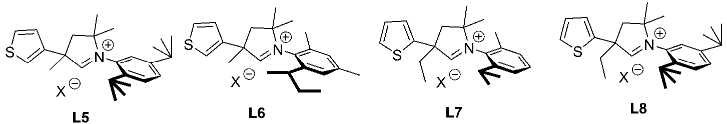

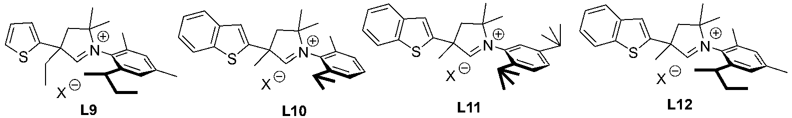

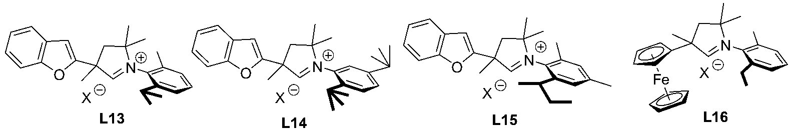

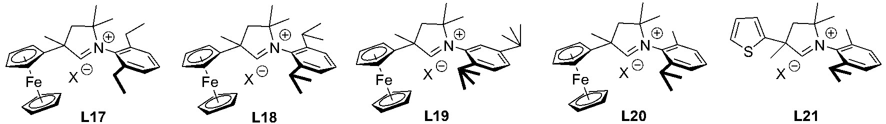

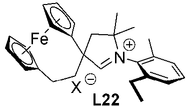

3. Un complexe de ruthénium de formule **1-Ru** 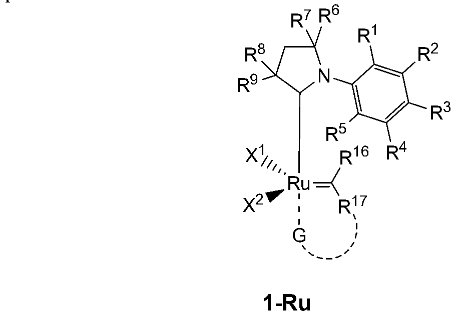 où :
X¹ et X² désignent, indépendamment l'un de l'autre, un ligand anionique choisi parmi le groupe comprenant un anion halogène, un groupe -CN, -SCN, -OR^{a} , -SR^{a} , -O(C=O)R^{a} , - O(SO₂)R^{a} et -OSi(R^{a})₃, où R^{a} désigne un alkyle en C₁-C₁₂, un cycloalkyle en C₃-C₁₂, un alcényle en C₂-C₁₂ ou un aryle en C₅-C₂₀, qui est éventuellement substitué par au moins un alkyle en C₁-C₁₂, un perfluoroalkyle en C₁-C₁₂, un alcoxyle en C₁-C₁₂, un aryloxyle en C₅-C₂₄, un hétéroaryloxy en C₅-C₂₀ ou un atome d'halogène ;
R¹, R², R³, R⁴ et R⁵ représentent chacun indépendamment un atome d'hydrogène, un groupe alkyle en C₁-C₁₂, un groupe cycloalkyle en C₃-C₁₂, un groupe aryle en C₅-C₂₀ ou un groupe hétéroaryle en C₅-C₂₀, un groupe aralkyle en C₅-C₂₅, qui peut être indépendamment substitué par un ou plusieurs substituants choisis parmi le groupe comprenant un atome d'hydrogène, un atome d'halogène, un groupe alkyle en C₁-C₁₂, un groupe perfluoroalkyle en C₁-C₁₂, un groupe aryle en C₅-C₂₀, un groupe perfluoroaryle en C₅-C₂₀, un groupe hétéroaryle en C₅ -C₂₀, un groupe alcoxyle en C₁-C₁₂, un groupe aryloxyle en C₅-C₂₄, un groupe hétéroaryloxy en C₅-C₂₀, un groupe sulfure (-SR"), un groupe amino (-NR"₂), dans lequel le groupe R" désigne indépendamment un atome d'hydrogène, un alkyle en C₁-C₅, un aryle en C₆-C₂₄, un aralkyle en C₇-C₂₄,
alternativement, R¹, R², R³, R⁴et R⁵ sont liés les uns aux autres pour former un cycle en C₅-C₂₅;
chaque substituant R⁶, R⁷et R⁸ représente un atome d'hydrogène, un atome d'halogène, un groupe alkyle en C₁-C₁₂ou un groupe aryle en C₅à C₂₀ aryle, qui peut être substitué indépendamment par un ou plusieurs substituants choisis dans le groupe comprenant un atome d'hydrogène, un groupe alkyle en C₁-C₁₂, un groupe perfluoroalkyle en C₁-C₁₂, un groupe aryle en C₅-C₂₀, un groupe perfluoroaryle en C₅-C₂₀, un groupe hétéroaryle en C₅ C₂₀, un groupe alcoxyle en C₁-C₁₂, un groupe aryloxyle en C₅-C₂₄, un groupe hétéroaryloxy en C₅-C₂₀ ou un atome d'halogène, un groupe sulfure (-SR"), un groupe amino (-NR"₂), dans lequel le groupe R" désigne indépendamment un atome d'hydrogène, un alkyle en C₁-C₅, un aryle en C₆-C₂₄, un aralkyle en C₇-C₂₄ ;
Le substituant R⁹ désigne un groupe hétérocyclique et/ou un groupe complexe organométallique, substitué ou non substitué, choisi parmi le thiophène, le benzothiophène, le furane, le benzofurane ou le ferrocène, qui peut être substitué indépendamment par un ou plusieurs substituants choisis parmi le groupe comprenant un atome d'hydrogène, un atome d'halogène, un groupe alkyle en C₁-C₁₂, un groupe perfluoroalkyle en C₁-C₁₂, un groupe aryle en C₅-C₂₀, un groupe perfluoroaryle en C₅-C₂₀, un groupe hétéroaryle en C₅-C₂₀, un groupe alcoxyle en C₁ -C₁₂, un groupe aryloxyle en C₅-C₂₄, un groupe hétéroaryloxy en C₅-C₂₀, un groupe sulfure (-SR"), un groupe amino (-NR"₂), dans lequel le groupe R" désigne indépendamment un atome d'hydrogène, un alkyle en C₁-C₅, un aryle en C₆-C₂₄, un aralkyle C₇-C₂₄,
R¹⁶ et R¹⁷ représentent chacun, indépendamment, un atome d'hydrogène, un atome d'halogène, un alkyle en C₁-C₂₅ , un cycloalkyle en C₃-C₂₅, un perfluoroalkyle en C₁-C₁₂, un alcène en C₂-C₂₅, un alcényle en C₂-C₂₅, un cycloalcényle en C₃-C₂₅, alcynyle en C₂-C₂₅, un cycloalkynyle C₃-C₂₅, un alcoxyle en C₁-C₂₅, un aryle en C₅-C₂₅, un aryloxyle en C₅-C₂₅, un arylalkyle en C₆ -C₂₅, un hétéroaryle en C₅-C₂₅, un hétéroaryloxy en C₅-C₂₅, un perfluoroaryle en C₅-C₂₅, un hétérocycle de 3 à 12 chaînons comprenant un atome de soufre, d'oxygène, d'azote, de sélénium ou de phosphore ;
les substituants R¹⁶ et R¹⁷ pouvant être liés entre eux pour former un cycle choisi dans le groupe comprenant les _{groupes}cycloalkyle en C₃à C₂₅, cycloalcényle en C₃à C₂₅, C₃ -C₂₅ cycloalkynyle, Cs -C₂₅ aryle, C₅ -C₂₅ hétéroaryle, C₅ -C₂₅ perfluoroaryle, un hétérocycle de 3 à 12 chaînons comprenant un atome de soufre, d'oxygène, d'azote, de sélénium ou de phosphore, qui peut être substitué indépendamment par un ou plusieurs substituants choisis dans le groupe comprenant un atome d'hydrogène, un atome d'halogène, un groupe alkyle en C₁ -C₂₅, un groupe C₃ -C₂₅ cycloalkyle, C₁ -C₁₂ perfluoroalkyle, C₂ -C₂₅ alcène, C₂ -C₂₅ alcényle, C₃ -C₂₅ cycloalcényle, C₂ -C₂₅ alcynyle, C₃ -C₂₅ cycloalkynyle, C₁ -C₂₅ alcoxyle, C₅ -C₂₅ aryle, C₅ -C₂₅ aryloxyle, C₆ -C₂₅ arylalkyle, C₅ -C₂₅ hétéroaryle, C₅ -C₂₅ hétéroaryloxy, C₅ -C₂₅ perfluoroaryle, un hétérocycle de 3 à 12 chaînons ;
G est choisi parmi, tel que,
- un ligand de formule **CAAC-1** 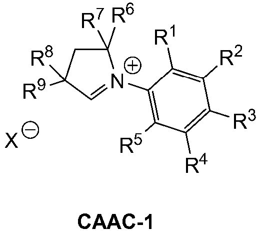 dans laquelle X et les substituants R¹ à R⁹ ont les significations définies ci-dessus
soit
- un hétéroatome 1
choisi parmi le groupe comprenant un atome d'oxygène, un atome de soufre, un atome de sélénium, substitué par un groupe choisi parmi un atome d'hydrogène, un atome de fluor, un atome d'oxygène, un alkyle en C₁-C₂₅, un perfluoroalkyle en C₁-C₂₅, un cycloalkyle en C₃-C₂₅, un alcoxyle en C₅-C₂₀, un aryle en C₅-C₂₀, un perfluoroaryle en C₅-C₂₀, un aralkyle en C₇-C₂₀, un aryloxy en C₅-C₂₄, un alcényle en C₂ -C₁₂, un hétéroaryle en C₆-C₂₀ ou un hétéroaryloxyle en C₅-C₂₄, un hétérocycle de 3 à 12 chaînons, éventuellement substitué par un groupe acyle (-COR'), un groupe cyano (-CN), un groupe carboxyle (-COOH), un groupe ester (-COOR'), un groupe ester (-CH₂ COOR'), un groupe ester (-CHR'COOR'), un groupe ester (-C(R')₂ COOR'), un groupe amide (-CONR'₂), une amide de type Weinreb (-CON(R')(OR')), un groupe sulfone (-SO₂ R'), un groupe formyle (-CHO), un groupe sulfonamide (-SO₂NR'₂), un groupe cétone (-COR'), un groupe thioamide (-CSNR'₂), un groupe thiocétone (-CSR'), un groupe thionoester (-CSOR'), un groupe thioester (-COSR"), un groupe dithioester (-CS₂R'), dans lequel le groupe R' désigne indépendamment un alkyle en C₁-C₂₅, un perfluoroalkyle en C₁-C₂₅, un cycloalkyle en C₃-C₂₅, un alcoxyle en C₅-C₂₀, un aryle en C₅-C₂₀, un perfluoroaryle en C₅-C₂₀, un aralkyle en C₇-C₂₀, un aryloxy en C₅-C₂₄, un alcényle en C₂ -C₁₂, un hétéroaryle en C₆-C₂₀, un hétéroaryloxy en C₅-C₂₄, et la ligne pointillée désigne alors une liaison directe entre un hétéroatome et le substituant R¹⁷ ou une liaison entre le substituant R¹⁷ et un hétéroatome via un pont méthylène -CH₂-, -CHR'-, ou -CR'₂- où le substituant R¹⁴ est un aryle en C₅-C₁₅, éventuellement substitué par 1 à 4 substituants, choisis indépendamment parmi un groupe comprenant un atome d'hydrogène, un atome d'halogène, un alkyle en C₁-C₂₅, un cycloalkyle en C₃-C₂₅, un alcényle en C₂-C₂₅, un cycloalcényle en C₃-C₂₅, un alcynyle en C₂-C₂₅, un cycloalkynyle en C₃-C₂₅, un perfluoroalkyle en C₁-C₂₅, un alcoxyle en C₅-C₂₀, un aryle en C₅-C₂₀, un perfluoroaryle en C₅-C₂₀, un aralkyle en C₇-C₂₀, un aryloxy en C₅-C₂₄, un hétéroaryle en C₆-C₂₀ ou un hétéroaryloxyle en C₅-C₂₄, un hétérocycle de 3 à 12 chaînons, un groupe alcoxyle (-OR"), un groupe sulfure (-SR"), un groupe sulfoxyde (-S(O)R"), un groupe sulfonium (-S⁺R"₂), un groupe sulfone (-SO₂R"), un groupe sulfonamide (-SO₂NR"₂), un groupe amino (-NR"₂), un groupe ammonium (-N⁺R"₃), un groupe nitro (-NO₂), un groupe cyano (-CN), un groupe phosphine (-P(O)(OR")₂), un groupe phosphinique (-P(O)R"(OR")), un groupe phosphonine (-P(OR")₂), un groupe phosphine (-PR"₂), un groupe oxyde de phosphine (-P(O)R"₂), un groupe phosphonium (-P⁺R"₃), un groupe carboxyle (-COOH), un groupe ester (-COOR"), un groupe amide (-CONR"₂), un groupe amide (-NR"C(O)R"), un groupe formyle (-CHO), un groupe cétone (-COR"), un groupe thioamide (-CSNR"₂), un groupe thiocétone (-CSR"), un groupe thionoester (-CSOR"), un groupe thioester (-COSR"), un groupe dithioester (-CS₂ R"), dans lequel le groupe R" désigne un alkyle en C₁-C₅, un perfluoroalkyle en C₁-C₅, un aryle en C₆-C₂₄, un aralkyle en C₇-C₂₄, un perfluoroaryle en C₅-C₂₄, ou
- hétéroatome 2
choisi parmi un groupe comprenant un atome d'azote ou un atome de phosphore, substitué par un groupe choisi parmi un atome d'hydrogène, un groupe méthylidène éventuellement substitué par le substituant R', un alkyle en C₁-C₂₅, un perfluoroalkyle en C₁-C₂₅, un cycloalkyle C₃-C₂₅, un alcoxyle en C₅-C₂₀, un aryle en C₅-C₂₀, un perfluoroaryle en C₅-C₂₀, un aralkyle en C₇-C₂₀, un aryloxy en C₅-C₂₄, un alcényle en C₂-C₁₂, un hétéroaryle en C₆-C₂₀ ou un hétéroaryloxyle en C₅-C₂₄, un hétérocycle de 3 à 12 chaînons, un groupe acyle (-COR'), un groupe ester (-COOR'), un groupe tert-butylbutyloxycarbonyle (t-Boc) ou un groupe 9-fluorénylméthoxycarbonyle (Fmoc), un groupe carbamine (-CONR'₂), un groupe sulfone (-SO₂R'), un groupe formyle (-CHO), dans lequel le groupe R' désigne un alkyle en C₁-C₂₅, un perfluoroalkyle en C₁-C₂₅, un cycloalkyle en C₃-C₂₅, un alcoxyle en C₅-C₂₀, un aryle en C₅-C₂₀, un perfluoroaryle en C₅-C₂₀, un aralkyle en C₇-C₂₀, un aryloxy en C₅-C₂₄, un alcényle en C₂-C₁₂, un hétéroaryle en C₆-C₂₀ ou un hétéroaryloxy en C₅-C₂₄, éventuellement substitué par un groupe acyle (-COR'), un groupe cyano (-CN), un groupe carboxyle (-COOH), un groupe ester (-COOR'), un groupe ester (-CH₂ COOR'), un groupe ester (-CHR'COOR'), un groupe ester (-C(R')₂ COOR'), un groupe amide (-CONR'₂), un groupe sulfone (-SO₂ R'), un groupe formyle (-CHO), un groupe sulfonamide (-SO₂ NR'₂), un groupe cétone (-COR'), un groupe thioamide (-CSNR'₂), un groupe thiocétone (-CSR'), un groupe thionoester (-CSOR'), un groupe thioester (-COSR'), un groupe dithioester (-CS₂R'), dans lequel le groupe R' désigne un alkyle en C₁-C₂₅, un perfluoroalkyle en C₁-C₂₅, un cycloalkyle en C₃-C₂₅, un alcoxyle en C₅ -C₂₀, un aryle en C₅-C₂₀, un perfluoroaryle en C₅-C₂₀, un aralkyle en C₇-C₂₀, un aryloxy en C₅-C₂₄, un alcényle en C₂-C₁₂, un hétéroaryle en C₆-C₂₀ ou un hétéroaryloxy en C₅-C₂₄, et la ligne pointillée désigne alors une liaison directe entre l'hétéroatome et le substituant R¹⁴ ou une liaison entre le substituant R¹⁷ et un hétéroatome via un pont méthylène (CH₂)-, -(CHR')-, ou -(CR'₂) ; où le substituant R¹⁷ est un groupe C₅ -C₁₅ aryle, éventuellement substitué par 1 à 4 substituants choisis indépendamment dans le groupe comprenant un atome d'hydrogène, un atome d'halogène, un groupe un alkyle en C₁-C₂₅, un cycloalkyle en C₃-C₂₅, un alcényle C₂-C₂₅, un cycloalcényle en C₃-C₂₅, un alcynyle en C₂-C₂₅, un cycloalkynyle en C₃-C₂₅, un perfluoroalkyle en C₁-C₂₅, un alcoxyle en C₅-C₂₀, un aryle en C₅-C₂₀, un perfluoroaryle en C₅-C₂₀, un aralkyle en C₇-C₂₀, un aryloxy en C₅-C₂₄, un hétéroaryle en C₆-C₂₀ ou un hétéroaryloxy en C₅-C₂₄, un hétérocycle de 3 à 12 chaînons, un groupe alcoxy (-OR"), un groupe sulfure (-SR"), un groupe sulfoxyde (-S(O)R"), un groupe sulfonium (-S⁺ R"₂), un groupe sulfone (-SO₂ R"), un groupe sulfonamide (-SO₂ NR"₂), un groupe amino (-NR"₂), un groupe ammonium (-N⁺R"₃), un groupe nitro (-NO₂), un groupe cyano (-CN), un groupe phosphine (-P(O)(OR")₂), un groupe phosphinique (-P(O)R"(OR")), un groupe phosphonine (-P(OR")₂), un groupe phosphine (-PR"₂), un groupe oxyde de phosphine (-P(O)R"₂), un groupe phosphonium (-P⁺ R"₃), un groupe carboxyle (-COOH), un groupe ester (-COOR"), un groupe amide (-CONR"₂), un groupe amide (-NR"C(O)R"), un groupe formyle (-CHO), un groupe cétone (-COR"), un groupe thioamide (-CSNR"₂), un groupe thiocétone (-CSR"), un groupe thionoester (-CSOR"), un groupe thioester (-COSR"), un groupe dithioester (-CS₂ R"), dans lequel le groupe R" désigne un alkyle en C₁-C₅, un perfluoroalkyle en C₁-C₅, un aryle en C₆-C₂₄, un aralkyle en C₇-C₂₄, un perfluoroaryle en C₅ - C₂₄, ou
- hétéroatome 3
choisi parmi un groupe comprenant un atome d'halogène, et la ligne pointillée désigne une liaison directe entre l'hétéroatome et le substituant R¹⁷, le substituant R¹⁷ étant un aryle en C₅-C₁₅ ou un polyaryle en C₅-C₂₅, éventuellement substitué par 1 à 4 substituants choisis indépendamment parmi un groupe comprenant un atome d'hydrogène, un atome d'halogène, un alkyle en C₁-C₂₅, un cycloalkyle en C₃-C₂₅, un alcényle en C₂-C₂₅, un cycloalcényle en C₃-C₂₅, un alcynyle en C₂-C₂₅, un cycloalcynyle en C₃-C₂₅, un perfluoroalkyle en C₁-C₂₅, un alcoxyle en C₅-C₂₀, un aryle en C₅-C₂₀, un perfluoroaryle en C₅-C₂₀, un aralkyle en C₇ -C₂₀, un aryloxy en C₅-C₂₄, un hétéroaryle en C₆-C₂₀ ou un hétéroaryloxy en C₅ -C₂₄, un hétérocycle de 3 à 12 chaînons, un groupe alcoxy (-OR"), un groupe sulfure (-SR"), un groupe sulfoxyde (-S(O)R"), un groupe sulfonium (-S⁺R"₂), un groupe sulfone (-SO₂ R"), un groupe sulfonamide (-SO₂NR"₂), un groupe amino (-NR"₂), un groupe ammonium (-N⁺ R"₃), un groupe nitro (-NO₂), un groupe cyano (-CN), un groupe phosphonique (-P(O)(OR")₂), un groupe phosphine (-P(O)R"(OR")), un groupe phosphonine (-P(OR")₂), un groupe phosphine (-PR"₂), un groupe oxyde de phosphine (-P(O)R"₂), un groupe phosphonium (-P⁺R"₃), un groupe carboxyle (-COOH), un groupe ester (-COOR"), un groupe amide (-CONR"₂), un groupe amide (-NR"C(O)R"), un groupe formyle (-CHO), un groupe cétone (-COR"), un groupe thioamide (-CSNR"₂), un groupe thiocétone (-CSR"), un groupe thionoester (-CSOR"), un groupe thioester (-COSR"), un groupe dithioester (-CS₂R"), dans lequel le groupe R" représente un alkyle en C₁-C₅, un perfluoroalkyle en C₁-C₅, un aryle en C₆-C₂₄, un aralkyle en C₇-C₂₄, un perfluoroaryle en C₅-C₂₄.

4. Le complexe de ruthénium selon la revendication 3, représenté par la formule **1a-Ru** 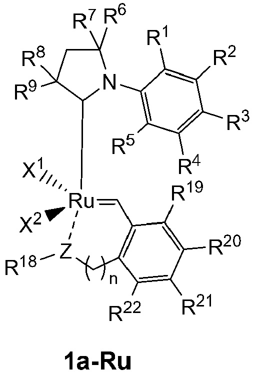 dans laquelle X¹ et X² ainsi que les substituants R¹, R², R³, R⁴, R⁵, R⁶, R⁷, R⁸, R⁹ ont les significations définies ci-dessus
« n » vaut 1 ou 0
Z est choisi parmi un groupe comprenant des atomes d'halogène, un atome O, un atome S, un atome Se ou un groupe NR"', dans lequel R"' représente un groupe méthylidène, un alkyle en C₁-C₂₅, un perfluoroalkyle en C₁-C₂₅, un cycloalkyle en C₃-C₂₅, un alcoxyle en C₅-C₂₀, un aryle en C₅-C₂₀, un perfluoroaryle en C₅-C₂₀, un aralkyle en C₇ -C₂₀, un aryloxy en C₅-C₂₄, un alcényle en C₂-C₁₂, un hétéroaryle en C₆-C₂₀ ou un hétéroaryloxyle en C₅-C₂₄, un hétérocycle de 3 à 12 chaînons, un groupe acyle (-COR'), un groupe ester (-COOR'), un groupe tert-butyloxycarbonyle (t-Boc) ou un groupe 9-fluorénylméthoxycarbonyle (Fmoc), un groupe carbamine (-CONR'₂), un groupe sulfone (-SO₂ R'), un groupe formyle (-CHO), dans lequel le groupe R' désigne un alkyle en C₁-C₂₅, un perfluoroalkyle en C₁-C₂₅, un cycloalkyle C₃-C₂₅, un alcoxyle en C₅-C₂₀, un aryle en C₅-C₂₀, un groupe perfluoroaryle en C₅-C₂₀, un aralkyle en C₇-C₂₀, un aryloxy en C₅-C₂₄, un alcényle en C₂-C₁₂, un hétéroaryle C₆-C₂₀ ou un hétéroaryloxy en C₅ -C₂₄,
ou un atome d'halogène, où si Z représente un atome d'halogène, R18 est absent ;
R¹⁸ représente indépendamment un atome d'hydrogène, un alkyle en C₁ -C₂₅, un cycloalkyle en C₁-C₂₅, un alcoxyle en C₅-C₂₀, un aryle en C₅-C₂₀, un aryloxy en C₅-C₂₄, un groupe -COOR"', un groupe -CH₂COOR‴, un groupe -CONR‴₂, un groupe -CH₂ CONR‴₂, un groupe -COR"', un groupe -CH₂COR‴, un groupe -CON(OR"')(R"'), un groupe -CH₂CON(OR‴)(R‴) ou un atome d'halogène, où R'" désigne un groupe alkyle en C₁ -C₁₂, cycloalkyle en C₃ -C₁₂, alcényle en C₂ - C₁₂, aryle en C₆-C₂₀ aryle, qui sont éventuellement substitués par au moins un alkyle en C₁-C₁₂, un perfluoroalkyle en C₁-C₁₂, un alcoxyle en C₁-C₁₂,un aryloxyle en C₅-C₂₄, ou un atome d'halogène ;
R¹⁹, R²⁰, R²¹ et R²² représentent chacun indépendamment un atome d'hydrogène, un atome d'halogène, un groupe alkyle en C₁-C₂₅, un groupe alcényle en C₂-C₂₅, un groupe aryle en C₅-C₂₅, un groupe alcoxy (-OR"), un groupe sulfure (-SR"), un groupe sulfoxyde (-S(O)R"), un groupe sulfonium (-S⁺R"₂), un groupe sulfone (-SO₂R"), un groupe sulfonamide (-SO₂ NR"₂), un groupe amino (-NR"₂), un groupe ammonium (-N⁺R"₃), un groupe nitro (-NO₂), un groupe cyano (-CN), un groupe phosphine (-P(O)(OR")₂), un groupe phosphinique (-P(O)R"(OR")), un groupe phosphonine (-P(OR")₂), un groupe phosphine (-PR"₂), un groupe oxyde de phosphine (-P(O)R"₂), un groupe phosphonium (-P⁺ R"₃), un groupe carboxyle (-COOH), un groupe ester (-COOR"), un groupe amide (-CONR"₂), un groupe amide (-NR"C(O)R"), un groupe formyle (-CHO), un groupe cétone (-COR"), un groupe thioamide (-CSNR"₂), un groupe thiocétone (-CSR"), un groupe thionoester (-CSOR"), un groupe thioester (-COSR"), un groupe dithioester (-CS₂ R"), dans lequel le groupe R" désigne un alkyle en C₁ -C₅, un perfluoroalkyle en C₁-C₅, un aryle en C₆ C₂₄, un aralkyle en C₇-C₂₄, un perfluoroaryle en C₅-C₂₄, où les substituants R¹⁶, R¹⁷, R¹⁸ et R¹⁹pouvant être reliés entre eux, formant ainsi un système cyclique en C₄-C₁₀ou polycyclique en C₄-C₁₂, substitué ou non.

5. Complexe de ruthénium selon la revendication 3 ou la revendication 4, de formule **1b-Ru** 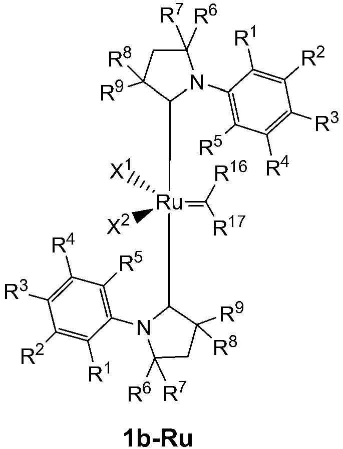
dans laquelle X¹ et X² ainsi que les substituants R¹, R², R³, R⁴, R⁵, R⁶, R⁷, R⁸, R⁹ ont les significations définies ci-dessus
R¹⁶ et R¹⁷ représentent chacun indépendamment un atome d'hydrogène, un atome d'halogène, un alkyle en C₁-C₂₅, éventuellement substitué ; un cycloalkyle en C₃-C₂₅, éventuellement substitué ; un perfluoroalkyle en C₁-C₁₂, éventuellement substitué ; un alcène en C₂-C₂₅, éventuellement substitué ; un alcényle en C₂-C₂₅, éventuellement substitué ; un cycloalcényle en C₃-C₂₅, éventuellement substitué ; un alcynyle en C₂-C₂₅, éventuellement substitué ; un cycloalkyle en C₃-C₂₅, éventuellement substitué ; un alcoxyle en C₁-C₂₅, éventuellement substitué ; un aryle C₅-C₂₅, éventuellement substitué ; un aryloxyle en C₅-C₂₅, éventuellement substitué ; un arylalkyle en C₆-C₂₅, éventuellement substitué ; un hétéroaryle en C₅-C₂₅, éventuellement substitué ; un hétéroaryloxy en C₅-C₂₅, éventuellement substitué ; un perfluoroaryle en C₅-C₂₅, éventuellement substitué ; un hétérocycle de 3 à 12 chaînons comprenant un atome de soufre, d'oxygène, d'azote, de sélénium ou de phosphore, éventuellement substitué ;
dans laquelle les substituants R¹⁶et R¹⁷peuvent être reliés pour former un cycle choisi parmi le groupe comprenant les groupes cycloalkyle en C₃-C₂₅, cycloalcényle en C₃-C₂₅, cycloalkynyle en C₃-C₂₅, aryle en C₅-C₂₅, hétéroaryle en C₅-C₂₅, perfluoroaryle en C₅-C₂₅, un hétérocycle de 3 à 12 chaînons contenant un atome de soufre, d'oxygène, d'azote, de sélénium ou de phosphore, qui peut être substitué indépendamment par un ou plusieurs substituants choisis parmi un groupe comprenant un atome d'hydrogène, un atome d'halogène, un alkyle en C₁ -C₂₅, un cycloalkyle en C₃-C₂₅, un perfluoroalkyle en C₁-C₁₂, un alcène en C₂-C₂₅, un alcényle en C₂-C₂₅, un cycloalcényle en C₃-C₂₅, un alcynyle en C₂-C₂₅, un cycloalcynyle en C₃-C₂₅, un alcoxyle en C₁ -C₂₅, un aryle en C₅-C₂₅, un aryloxyle en C₅-C₂₅, un arylalkyle en C₆-C₂₅, un hétéroaryle en C₅-C₂₅, un hétéroaryloxy en C₅-C₂₅, un perfluoroaryle en C₅ -C₂₅, un hétérocycle de 3 à 12 chaînons.

6. Complexe de ruthénium selon l'une quelconque des revendications 3 à 4, qui est choisi parmi les complexes représentés par les formules **Ru1a, Ru2a, Ru3a, Ru4a, Ru5a, Ru6a, Ru7a, Ru8a, Ru9a, Ru10a, Ru11a, Ru11a, Ru12a, Ru13a, Ru14a, Ru15a, Ru16a, Ru17a, Ru18a, Ru19a, Ru20a, Ru21a, Ru22a :**

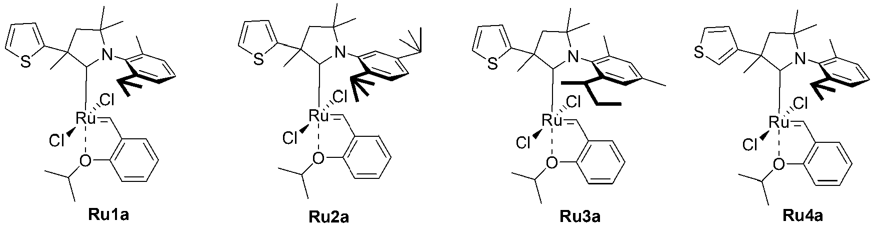

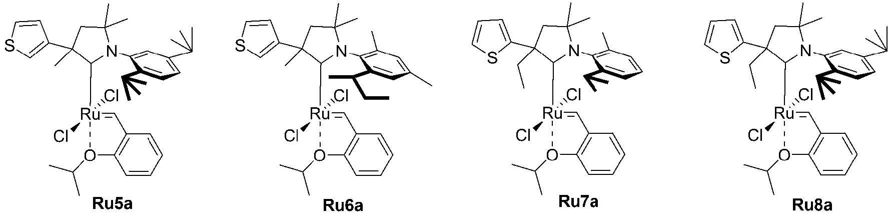

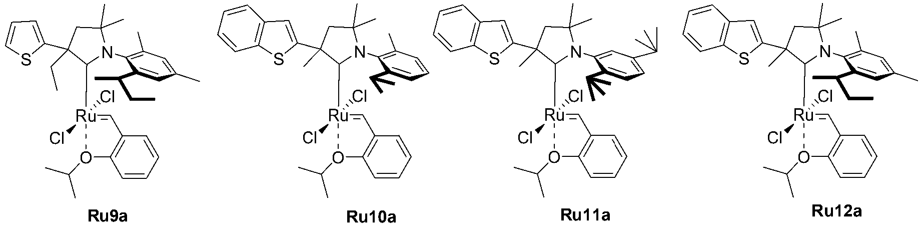

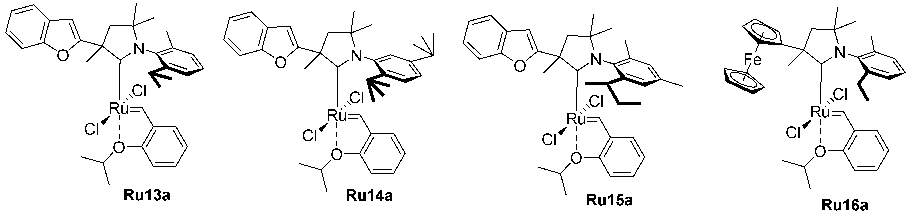

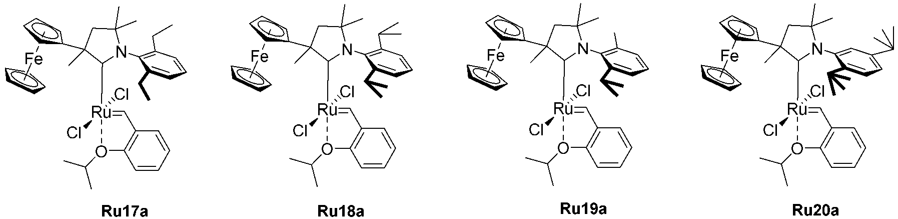

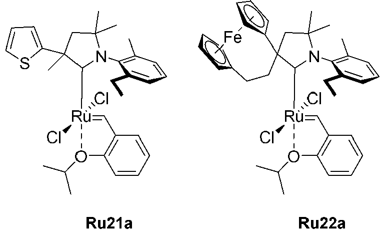

7. Méthode de synthèse du complexe de ruthénium de formule **1a-Ru** 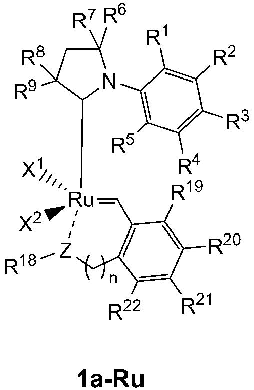
telle que définie dans la revendication 4, **caractérisé en ce que** l'on utilise le complexe d'alkylidène-ruthénium de formule 10 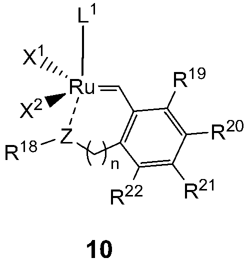
où :
L¹ désigne un ligand neutre choisi dans le groupe comprenant une pyridine ou une pyridine substituée, P(R')₃, P(OR')₃, O(R')₂, N(R')₃, où chaque R' désigne indépendamment un groupe alkyle en C₁-C₁₂, un cycloalkyle en C₃-C₁₂, un aryle en C₅-C₂₀, un aralkyle en C₇-C₂₄, un perfluoroaryle en C₅-C₂₄, un hétéroaryle de 5 à 12 chaînons ;
N, Z, X¹, X² et les substituants R¹⁸, R¹⁹, R²⁰, R²¹et R²² ont les significations définies ci-dessus subit une réaction avec le carbène de formule 8 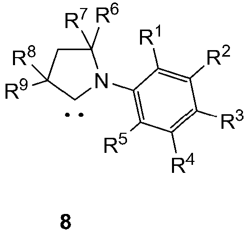
où les substituants R¹ à R9 ont les significations définies ci-dessus.

8. Utilisation du composé de formule **1-Ru,** tel que défini dans l'une quelconque des revendications 3 à 6, en tant que précatalyseur et/ou catalyseur dans des réactions de métathèse d'oléfines, en particulier dans la métathèse avec fermeture de cycle (RCM), la métathèse croisée (CM), l'homométathèse (métathèse croisée entre deux molécules de la même oléfine), l'éthénolyse, l'isomérisation, dans une réaction de métathèse par réarrangement diastéréosélectif (DRRM), la métathèse alcène-alcyne (ène-yne) ou une réaction de polymérisation ROMP ou ADMET.

9. Utilisation selon la revendication 8, dans laquelle la réaction est effectuée dans un solvant organique tel que le toluène, le mésitylène, l'hexane, le cyclohexane, l'acétate d'éthyle, l'acétate de méthyle, le carbonate de méthyle, le carbonate d'éthyle, l'éther méthylique de tert-butyle, l'éther méthylique de cyclopentyle, l'éther diéthylique, le THF, le 2-Me-THF, le 4-Me-THP, le dioxane, le DME, le PAO. PEG, de la paraffine, des esters d'acides gras saturés.

10. Utilisation selon la revendication 8, dans laquelle la réaction est effectuée dans un système sans solvant.

11. Utilisation selon l'une quelconque des revendications précédentes, dans laquelle la réaction est effectuée à une température comprise entre 20°C et 200°C.

12. Utilisation selon l'une quelconque des revendications précédentes, dans laquelle la réaction est effectuée pendant une durée comprise entre 5 minutes et 48 heures.

13. Utilisation selon l'une quelconque des revendications précédentes, dans laquelle le composé **1-Ru** est appliqué en une quantité ne dépassant pas 10 % en mole, de préférence en une quantité ne dépassant pas 0,1 % en mole.

14. Utilisation selon l'une quelconque des revendications précédentes, dans laquelle le composé **1-Ru** est ajouté au mélange réactionnel sous forme de portions solides et/ou en continu à l'aide d'une pompe, sous forme de solution dans un solvant organique.

15. Utilisation selon l'une quelconque des revendications précédentes, dans laquelle le sous-produit gazeux de la réaction, choisi parmi l'éthylène, le propylène et le butylène, est activement éliminé du mélange réactionnel à l'aide d'un barbotage de gaz inerte ou par le biais d'un vide.
